(19) Europäisches Patentamt
European Patent Office
Office européen des brevets

(11) EP 3 510 041 B1

(12) **EUROPEAN PATENT SPECIFICATION**

(45) Date of publication and mention of the grant of the patent:
**31.05.2023 Bulletin 2023/22**

(21) Application number: **17777421.3**

(22) Date of filing: **30.08.2017**

(51) International Patent Classification (IPC):
***C07K 1/18*** *(2006.01)* ***C07K 1/34*** *(2006.01)*

(52) Cooperative Patent Classification (CPC):
**C07K 1/34; C07K 1/18**

(86) International application number:
**PCT/US2017/049376**

(87) International publication number:
**WO 2018/048698 (15.03.2018 Gazette 2018/11)**

# (54) PROCESSES FOR SEPARATING AGGREGATED PROTEINS FROM MONOMERIC PROTEINS IN A BIOLOGICAL SOLUTION

VERFAHREN ZUR TRENNUNG VON AGGREGIERTEN PROTEINEN VON MONOMEREN PROTEINEN IN EINER BIOLOGISCHEN LÖSUNG

PROCÉDÉS DE SÉPARATION DE PROTÉINES AGRÉGÉES À PARTIR DE PROTÉINES MONOMÈRES DANS UNE SOLUTION BIOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2016 US 201662385385 P**

(43) Date of publication of application:
**17.07.2019 Bulletin 2019/29**

(73) Proprietor: **3M Innovative Properties Company**
**St. Paul, MN 55133-3427 (US)**

(72) Inventors:
- **COLAK ATAN, Semra**
  **Saint Paul, Minnesota 55133-3427 (US)**
- **VAIL, Andrew W.**
  **Saint Paul, Minnesota 55133-3427 (US)**
- **RASMUSSEN, Jerald K.**
  **Saint Paul, Minnesota 55133-3427 (US)**
- **GRIESGRABER, George W.**
  **Saint Paul, Minnesota 55133-3427 (US)**
- **BOTHOF, Catherine A.**
  **Saint Paul, Minnesota 55133-3427 (US)**

(74) Representative: **Mathys & Squire**
**Theatinerstraße 7**
**80333 München (DE)**

(56) References cited:
**WO-A1-2010/098867 WO-A1-2015/050767**
**WO-A1-2015/088677**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 510 041 B1

## Description

## Cross Reference To Related Application

This application claims the benefit of U.S. Provisional Patent Application No. 62/385385, filed September 9, 2016.

## BACKGROUND

Isolation and purification of target biomaterials, particularly monomeric proteins, such as monomeric antibodies (particularly monomeric monoclonal antibodies) are important for therapeutic uses for a variety of diseases including rheumatoid arthritis, Crohn's disease, hypercholesterolemia, and a variety of cancers. Monoclonal antibodies, for example, are preferred over conventional therapies because they can be directed to specific targets without significant adverse effect on healthy tissue. Removal of aggregated proteins from the monomeric proteins is particularly important for such therapeutic uses.

Polymeric materials have been widely used for the separation and purification of various target biomaterials. Such separation and purification methods can be based on any of a number of binding factors or mechanisms including the presence of an ionic group, the size of the target biomaterial, a hydrophobic interaction, an affinity interaction, a hydrogen bonding interaction, the formation of a covalent bond, and so forth.

Membrane-based technologies, especially in disposable format, are becoming increasingly important in biopharmaceutical and vaccine manufacturing processes. Membranes have been used in passive, size-based separations, and, more recently, in active filtration (for example, for the removal of minor contaminants in later stages of purification processes).

Functionalized membranes (including functional polymer-bearing membranes) have typically suffered from relatively low biomaterial binding capacities, however, and this has generally limited their use in large-scale purifications. Porous beaded chromatography resins (bearing ion exchange or other interactive functional groups), rather than functionalized membranes, therefore have been standardly used in "capture-and-elute" or "bind-and-elute" type protein purification processes.

Thus, there is a need for processes that are relatively simple, cost-effective, and/or efficient (for example, involving relatively easily accessible starting materials and/or relatively few process steps) for isolation and purification of monomeric proteins, particularly separation of aggregated proteins from monomeric proteins, particularly monomeric monoclonal antibodies.

Documents WO 2015/088677 A1 and WO 2010/098867 A1 disclose a process for separating aggregated proteins from monomeric proteins in a biological solution. Both documents are silent on an extended spacer group which contains at least 6 catenated atoms that directly links the at least one acidic group or salt thereof to the hydrocarbon backbone. Document WO 2015/050767 A1 D3 discloses a modified a porous substrates with an extended spacer group which contains at least 6 catenated atoms that directly links the at least one acidic group or salt thereof to the hydrocarbon backbone for purification of target biomaterials such as target proteins. D3, however, is silent on utilizing such substrates in a process for specifically separating aggregated proteins from monomeric proteins in a biological solution.

The present disclosure provides a process for separating aggregated proteins from monomeric proteins in a biological solution. The process includes: providing at least one filter element having a contacting surface (e.g., an upstream or upper surface); and allowing an initial biological solution to contact the contacting surface (e.g., upstream or upper surface) of the filter element under conditions effective to separate the aggregated proteins from the monomeric proteins such that a final biological solution includes purified monomeric proteins.

In one embodiment of such process, the filter element includes filter media including: a porous substrate; and disposed on the porous substrate, a polymer that includes a hydrocarbon backbone and a plurality of pendant groups attached to the hydrocarbon backbone, wherein each of a first plurality of pendant groups includes:

(1) at least one acidic group or salt thereof; and
(2) a spacer group that directly links the at least one acidic group or salt thereof to the hydrocarbon backbone by a chain of at least 6 catenated atoms.

In another embodiment of such process, the filter element includes filter media that includes: a porous substrate; and disposed on the porous substrate, a polymer that includes interpolymerized units of at least one monomer including:

(1) at least one ethylenically unsaturated group;
(2) at least one acidic group or salt thereof; and
(3) a spacer group that directly links the at least one ethylenically unsaturated group and the at least one acidic

group or salt thereof by a chain of at least 6 catenated atoms.

**Definitions**

As used in this patent application:

"aggregated protein" or "protein aggregate" refers to an association of at least two molecules (i.e., monomers) of a product of interest, e.g., a therapeutic protein (e.g., antibody). The association of at least two molecules of a product of interest may arise by any means including, but not limited to, covalent, non-covalent, disulfide, or non-reducible crosslinking;
"boronato" means a group of formula $-B(OH)_2$;
"carbonylimino" means a divalent group or moiety of formula -(CO)NR-, where R is hydrogen, alkyl (for example, selected from alkyl groups having from one to four carbon atoms), or aryl (preferably, R is hydrogen);
"carboxy" means a group of formula -COOH;
"catenated atom" means an in-chain atom (rather than an atom of a chain substituent);
"catenated heteroatom" means an atom other than carbon (for example, oxygen, nitrogen, or sulfur) that replaces one or more carbon atoms in a carbon chain (for example, so as to form a carbon-heteroatom-carbon chain or a carbon-heteroatom-heteroatom-carbon chain);
"ethylenically unsaturated" means a group of formula $-CY=CH_2$ where Y is hydrogen, alkyl, cycloalkyl, or aryl;
"graft density" was determined by measuring the mass gain after conversion of the substrate to the finished filter element, divided by the theoretical molecular weight of the monomer mixture used, and expressed as mmol/g, that is, millimoles (or milliequivalents) of ligand group grafted per gram of original substrate;
"heteroatom" means an atom other than carbon or hydrogen;
"hydrogen bond acceptor" means a heteroatom selected from oxygen, nitrogen, and sulfur that has a lone electron pair;
"hydrogen bond donor" means a moiety consisting of a hydrogen atom covalently bonded to a heteroatom selected from oxygen, nitrogen, and sulfur;
"hydrogen bonding moiety" means a moiety that includes at least one hydrogen bond donor and at least one hydrogen bond acceptor;
"hydroxy" means a monovalent group of formula -OH;
"iminocarbonylimino" means a divalent group or moiety of formula -N(R)-C(O)-N(R)-, wherein each R is independently hydrogen, alkyl (for example, selected from alkyl groups having from one to four carbon atoms), or aryl (preferably, at least one R is hydrogen; more preferably, both are hydrogen);
"iminothiocarbonylimino" means a divalent group or moiety of formula -N(R)-C(S)-N(R)-, wherein each R is independently hydrogen, alkyl (for example, selected from alkyl groups having from one to four carbon atoms), or aryl (preferably, at least one R is hydrogen; more preferably, both are hydrogen);
"isocyanate" means a group of formula -N=C=O;
"oxycarbonylimino" means a divalent group or moiety of formula -O-C(O)-N(R)-, wherein R is hydrogen, alkyl (for example, selected from alkyl groups having from one to four carbon atoms), or aryl (preferably, R is hydrogen);
"oxythiocarbonylimino" means a divalent group or moiety of formula -O-C(S)-N(R)-, wherein R is hydrogen, alkyl (for example, selected from alkyl groups having from one to four carbon atoms), or aryl (preferably, R is hydrogen);
"phosphate" means a group of formula $-OPO_3H_2$;
"phosphono" means a group of formula $-PO_3H_2$ wherein this group is not attached to an oxygen atom;
"purified" in the context of monomeric proteins (e.g., antibodies) in a biological solution means that the amount of monomeric proteins (e.g., antibodies) relative to the amount of aggregated proteins (e.g., antibodies) in the final biological solution compared to the initial biological solution is increased;
"sulfato" means a group of formula $-OSO_3H$;
"sulfono" means a group of formula $-SO_3H$ wherein this group is not attached to an oxygen atom;
"thiocarbonylimino" means a divalent group or moiety of formula -(CS)NR-, where R is hydrogen, alkyl (for example, selected from alkyl groups having from one to four carbon atoms), or aryl (preferably, R is hydrogen).

Also, herein, the terms "comprises" and variations thereof do not have a limiting meaning where these terms appear in the description and claims. Such terms will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting

essentially of" indicates that the listed elements are required or mandatory, but that other elements are optional and may or may not be present depending upon whether or not they materially affect the activity or action of the listed elements.

The words "preferred" and "preferably" refer to embodiments of the disclosure that may afford certain benefits under certain circumstances. Other embodiments may also be preferred, however, under the same or other circumstances. Furthermore, the recitation of one or more preferred embodiments does not imply that other embodiments are not useful, and is not intended to exclude other embodiments from the scope of the disclosure.

In this application, terms such as "a," "an," and "the" are not intended to refer to only a singular entity, but include the general class of which a specific example may be used for illustration. The terms "a," "an," and "the" are used interchangeably with the term "at least one." The phrases "at least one of" and "comprises at least one of" followed by a list refers to any one of the items in the list and any combination of two or more items in the list.

The phrases "at least one of" and "comprises at least one of" followed by a list refers to any one of the items in the list and any combination of two or more items in the list.

As used herein, the term "or" is generally employed in its usual sense including "and/or" unless the content clearly dictates otherwise.

The term "and/or" means one or all of the listed elements or a combination of any two or more of the listed elements (e.g., preventing and/or treating an affliction means preventing, treating, or both treating and preventing further afflictions).

Herein, various sets of numerical ranges (for example, of the number of carbon atoms in a particular moiety, of the amount of a particular component, or the like) are described, and, within each set, any lower limit of a range can be paired with any upper limit of a range. Such numerical ranges also are meant to include all numbers subsumed within the range (for example, 1 to 5 includes 1, 1.5, 2, 2.75, 3, 3.80, 4, 5, and so forth).

Also herein, all numbers are assumed to be modified by the term "about" and preferably by the term "exactly." As used herein in connection with a measured quantity, the term "about" refers to that variation in the measured quantity as would be expected by the skilled artisan making the measurement and exercising a level of care commensurate with the objective of the measurement and the precision of the measuring equipment used. Herein, "up to" a number (e.g., up to 50) includes the number (e.g., 50).

Reference throughout this specification to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout this specification are not necessarily referring to the same embodiment of the disclosure. Furthermore, the particular features, configurations, compositions, or characteristics may be combined in any suitable manner in one or more embodiments.

When a group is present more than once in a formula described herein, each group is "independently" selected, whether specifically stated or not. For example, when more than one $R^2$ group is present in a formula, each $R^2$ group is independently selected. Furthermore, subgroups contained within these groups are also independently selected.

The above Summary section is not intended to describe every embodiment or every implementation of the disclosure. The detailed description that follows more particularly describes illustrative embodiments. Throughout the detailed description, guidance is provided through lists of examples, which examples can be used in various combinations. In each instance, a recited list serves only as a representative group and should not be interpreted as being an exclusive list.

## DETAILED DESCRIPTION

The present disclosure provides a process for separating aggregated proteins from monomeric proteins in a biological solution. The process includes: providing at least one filter element having a contacting surface (e.g., an upstream or upper surface); and allowing the biological solution to contact the contacting surface (e.g., upstream or upper surface) of the filter element under conditions effective to separate the aggregated proteins from the monomeric proteins.

In certain embodiments of such process, the filter element includes filter media including: a porous substrate; and disposed on the porous substrate, a polymer that includes a hydrocarbon backbone and a plurality of pendant groups attached to the hydrocarbon backbone, wherein each of a first plurality of pendant groups includes:

(1) at least one acidic group or salt thereof; and
(2) a spacer group that directly links the at least one acidic group or salt thereof to the hydrocarbon backbone by a chain of at least 6 catenated atoms.

The polymer disposed on the porous substrate can be formed prior to being disposed thereon. For example, a polymer can be formed and then coated on the substrate surface. In an alternative embodiment, the polymer can be

formed from monomers in the presence of the substrate and grafted thereto. For example, monomers that include the acidic group or salt thereof can be subjected to electron beam radiation in the presence of the substrate to graft and polymerize such monomers. In another embodiment, compounds that include the acidic group or salt thereof can be reacted with monomer units previously grafted to the substrate surface.

In certain embodiments of such process, the polymer disposed on the porous substrate includes interpolymerized units of at least one monomer including:

(1) at least one ethylenically unsaturated group (in some embodiments, a terminal ethylenically unsaturated group);
(2) at least one acidic group or salt thereof; and
(3) a spacer group that directly links the at least one ethylenically unsaturated group and the at least one acidic group or salt thereof by a chain of at least 6 catenated atoms.

In certain embodiments, the porous substrate is a porous membrane, and in certain embodiments, a porous polymeric membrane.

In certain embodiments, the acidic groups or salts thereof in the plurality of pendant groups of the polymer or in the monomer are selected from carboxy, phosphono, phosphate, sulfono, sulfato, boronato, and combinations thereof.

In certain embodiments, the spacer group in the plurality of pendant groups of the polymer or in the monomer includes at least one hydrogen bonding moiety.

Surprisingly, the acidic groups or salts thereof of such polymers can be especially effective in interacting with aggregated proteins, relative to monomeric proteins. Porous substrates bearing the polymer (in certain embodiments, bearing grafted polymer) can exhibit unexpectedly higher aggregated protein binding capacities than corresponding porous substrates bearing polymer with a shorter linking chain. Aggregated protein binding capacities can be surprisingly further enhanced by including at least one hydrogen bonding moiety in the linking chain.

In certain embodiments, the relatively high binding capacity functionalized substrates for binding to aggregated proteins, relative to monomeric proteins, can be prepared by using certain monomers having a multi-atom spacer group between the monomer's ethylenically unsaturated group and acidic group or salt thereof. The monomers include compounds in which at least one acidic group or salt thereof is separated or spaced from at least one ethylenically unsaturated group by a linking chain of at least six catenated atoms. When free radically polymerized, such monomers provide polymers bearing acidic groups or salts thereof that are separated or spaced from the resulting polymer chain or polymer backbone by the linking chain of at least six (6) catenated atoms.

A wide variety of biological solutions containing a protein may be utilized in the processes of the present disclosure. The solution, which contains a protein, may include, for example, fermentation broth or cell culture or murine ascites fluid. The protein can be any protein, or fragment thereof, known in the art. The protein may be derived from natural sources or from recombinant sources. The protein may have a native sequence or a non-natural sequence. In some embodiments, the protein is an enzyme. In some embodiments, the protein is a hormone. In some embodiments, the protein is an antibody. In a particular embodiment, the protein is a monoclonal antibody, or a fragment thereof. Fragments of antibodies include F(ab), F(ab'), $F(ab')_2$, Fv, and single-chain antibodies. In some cases, the protein may be a human monoclonal antibody. In other cases, the protein is an immunoglobulin G (IgG) antibody. In still other cases, the protein is a fusion protein such as an Fc-fusion protein.

The process of separation of aggregated proteins from monomeric proteins in a biological solution results from binding aggregated proteins to the filter media while permitting passage of monomeric proteins. In early stages of separation with low challenge loads, monomeric proteins may be bound to the filter media; however, as the challenge load increases, preferential binding of the aggregated proteins displaces monomeric proteins. Thus, although the process may involve binding of monomeric proteins to the filter media at low challenge loads, the process ultimately results in separating the aggregated proteins from the monomeric proteins such that a final biological solution (i.e., the product obtained by contacting the initial biological solution with the filter element) includes purified monomeric proteins.

In certain embodiments of the process, allowing the biological solution to contact the contacting surface (e.g., upstream or upper surface) of the filter element occurs by passing the biological solution across the filter media or by allowing the biological solution to flow through the filter media, or both. In certain embodiments, allowing the biological solution to contact the contacting surface (e.g., upstream or upper surface) involves allowing the biological solution to flow through filter media.

In certain embodiments, the process involves purifying the monomeric proteins without the need for an eluting step. That is, this process does not bind the monomeric proteins and wash through the aggregated proteins; rather, it is binding the aggregated proteins and allowing the monomeric proteins to pass through. This is advantageous at least because it avoids dilution of the monomeric proteins, which prevents the need to later concentrate the eluted monomeric proteins, thereby providing processing efficiency and decreasing processing time. This is also advantageous because it allows for the capture and disposal of aggregated proteins bound to the filter media, which is desirable when the filter media is enclosed within a disposable filter article. Furthermore, buffer exchanges and pH adjustments may be unnecessary.

In certain embodiments, the process involves recovering, in a final solution, at least 40%, or at least 45%, or at least 50%, or at least 55%, or at least 60%, or at least 65%, or at least 70%, or at least 75%, or at least 80%, or at least 85%, or at least 90%, or at least 95%, of the monomeric proteins present in the initial biological solution (i.e., the biological solution before being subjected to a process as described herein).

In certain embodiments, the process involves removing at least 5%, or at least 10%, or at least 15%, or at least 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, of the aggregated proteins from the initial biological solution.

The process involves allowing the biological solution to contact the contacting surface (e.g., upstream or upper surface) of the filter element under conditions effective to separate the aggregated proteins from the monomeric proteins to provide the desired results.

In certain embodiments, such conditions include a pH of the biological solution of below 9, or below 8.5, or below 8, or below 7.5, or below 7, or below 6.5. In certain embodiments, such conditions include a pH of the biological solution of at least 4, or at least 4.5, or at least 5.

In certain embodiments, such conditions include a conductivity of the biological solution of at least 1 millisiemens per centimeter (mS/cm), or at least 2 mS/cm, or at least 3 mS/cm, or at least 4 mS/cm, or at least 5 mS/cm, or at least 6 mS/cm, or at least 7 mS/cm, or at least 8 mS/cm, or at least 9 mS/cm, or at least 10 mS/cm. In certain embodiments, such conditions include a conductivity of the biological solution of no greater than 110 mS/cm, or no greater than 100 mS/cm, or no greater than 50 mS/cm, or no greater than 40 mS/cm, or no greater than 30 mS/cm.

In certain embodiments, such conductivity of the biological solution is provided by a buffer that includes an inorganic salt, organic salt, or a combination thereof. In certain embodiments, such buffer salt includes a chloride (e.g., NaCl), a phosphate, a citrate, a sulfate, an acetate, or a combination thereof.

In certain embodiments, such conditions include a challenge load of at least 1 gram per liter (g/L) of filter media, or at least 10 g/L of filter media, or at least 25 g/L of filter media, or at least 50 g/L of filter media, or at least 75 g/L of filter media, or at least 100 g/L, or at least 500 g/L, or at least 1000 g/L, or at least 2000 g/L of filter media. In this context, "challenge load" means the amount of proteins (aggregated and monomeric) per volume of filter media in grams per liter. The upper limit of the challenge load is determined by a number of things, including the selectivity for aggregated proteins over monomeric proteins, the actual amount of aggregated proteins in the initial biological solution, the total protein concentration in the initial biological solution, etc. Desirably, the challenge load is in the kilogram to multi-kilogram range and as high a challenge load as possible.

In certain embodiments, assessment of optimal conditions for the process is conveniently conducted using an array of sample containers, wherein each container includes filter elements of the present disclosure. A particularly useful array of containers is, for example, a 96-well filter plate in which each well includes filter elements of the disclosure. Each well of an individual plate may include the same filter element, that is, the filter elements may be functionalized with the same polymer composition. Alternatively, two or more different filter elements, i.e., functionalized with different polymer compositions, may be disposed within the wells of a single plate. Each well may include a single layer of a filter element, or alternatively may contain more than one layer of filter elements. In the latter case, the multiple layers of filter elements may be functionalized with the same polymer, or with different polymers. Protein solutions may then be prepared utilizing a variety of buffer salts, buffer pH values, buffer conductivities, and/or protein concentrations. These initial solutions may then be applied into the wells of the plate so as to contact the contacting surface (e.g., upper surface) of the filter elements. Following a predetermined contact time, the protein solution may be passed through the filter elements, such as by centrifugation or by vacuum filtration, and may be collected in the wells of a collection plate. The filtrate (final solution) thus collected may be analyzed for concentration of monomeric protein and aggregated protein, such as by HPLC analysis, and compared with that of the initial solution. In this manner, optimal conditions (in terms of buffer, pH, conductivity, etc.) for removal of aggregated protein with minimal loss of monomeric protein can be assessed within the context of a single, high throughput experiment. Such techniques are well known to one of skill in the art.

## Monomers

Monomers suitable for use in preparing the polymers used in filter media in the process of the disclosure include those that comprise (or consist of): (a) at least one ethylenically unsaturated group; (b) at least one acidic group or salt thereof; and (c) a spacer group that directly links the at least one ethylenically unsaturated group and the at least one acidic group or salt thereof by a chain of at least 6 catenated atoms.

The monomers can be in a neutral state but can also be negatively charged under some pH conditions.

The ethylenically unsaturated group (as defined above) of the monomer(s) can be represented by the formula $-CY=CH_2$, wherein Y is hydrogen, alkyl, cycloalkyl, or aryl. In certain embodiments, ethylenically unsaturated groups include ethenyl, 1-alkylethenyl, and combinations thereof (that is, Y is preferably hydrogen or alkyl; more preferably, Y

is hydrogen or C to $C_4$ alkyl; and even more preferably, Y is hydrogen or methyl).

The monomer(s) can include a single ethylenically unsaturated group or multiple ethylenically unsaturated groups (for example, two or three or up to as many as 6), which can be the same or different in nature (preferably, the same). The monomer(s) preferably have only one ethylenically unsaturated group.

Suitable acidic groups of the monomers include those that exhibit at least a degree of acidity (which can range from relatively weak to relatively strong), as well as salts thereof. Such acidic groups or salts thereof include those commonly utilized as ion exchange or metal chelate type ligands.

In certain embodiments, the at least one acidic group or salt thereof is a heteroatom-containing group. Useful acidic groups include heterohydrocarbyl groups and other heteroatom-containing groups. For example, useful acidic groups can include one or more heteroatoms selected from oxygen, sulfur, phosphorus, boron, and the like, and combinations thereof. Useful salts of acidic groups include those having counter ions selected from alkali metal (for example, sodium or potassium), alkaline earth metal (for example, magnesium or calcium), ammonium, and tetraalkylammonium ions, and the like, and combinations thereof.

The monomer(s) can include a single acidic group or multiple acidic groups (for example, two or three or up to as many as 6), or salts thereof, which can be the same or different in nature (preferably, the same). In certain embodiments, the acidic group(s) are selected from carboxy, phosphono, phosphate, sulfono, sulfato, boronato, and combinations thereof. In certain embodiments, the acidic group(s) include carboxy, phosphono, sulfono, and combinations thereof. In certain embodiments, the acidic group is a carboxy group.

The spacer group of the monomer(s) can be directly linked to at least one ethylenically unsaturated group and at least one acidic group or salt thereof by a chain of at least 6 catenated atoms. Thus, the chain can include 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, or more catenated atoms (for example, including up to as many as 40 or 50). In certain embodiments, the chain includes at least 7 catenated atoms (more preferably, at least 8, and even more preferably, at least 9, at least 10, at least 11, or at least 12 catenated atoms) and/or includes no more than 30 catenated atoms (more preferably, no more than 25, even more preferably, no more than 20, and even more preferably, no more than 16 catenated atoms).

Although not wishing to be bound by theory, the length of the chain may contribute to adoption of helical or partially helical conformations by the polymer backbone (formed through monomer polymerization). When the chain is relatively short (for example, less than 6 catenated atoms), ionic repulsion between acidic groups may force the polymer backbone into a random coil type conformation. As chain length increases, adoption of helical conformations may become possible and may be maximized at chain lengths of 8 to 14 catenated atoms. A helical conformation of substrate-grafted polymer may facilitate presentation of the acidic group(s), or salt(s) thereof, for interaction with aggregated proteins, particularly aggregated monoclonal antibodies.

In certain embodiments, spacer groups include at least one hydrogen bonding moiety, which is defined above as a moiety including at least one hydrogen bond donor and at least one hydrogen bond acceptor (both of which are heteroatom-containing). Thus, preferred spacer groups include heteroatom-containing hydrocarbon groups (more preferably, catenated heteroatom-containing hydrocarbon groups). More preferred spacer groups include at least two hydrogen bonding moieties or include at least one hydrogen bonding moiety and at least one hydrogen bond acceptor that is distinct from (not part of) the hydrogen bonding moiety.

In certain embodiments, hydrogen bonding moieties include those that include at least two hydrogen bond donors (for example, donors such as imino, thio, or hydroxy), at least two hydrogen bond acceptors (for example, acceptors in the form of carbonyl, carbonyloxy, or ether oxygen), or both. For example, an iminocarbonylimino moiety (having two N-H donors and at least two acceptors in the form of two lone electron pairs on carbonyl) can sometimes be preferred over a single iminocarbonyl moiety. In certain embodiments, the spacer groups include those that include at least one iminocarbonylimino moiety (more preferably, in combination with at least one acceptor such as carbonyloxy), at least two iminocarbonyl moieties, or a combination thereof.

The hydrogen bond donor and hydrogen bond acceptor of the hydrogen bonding moiety can be adjacent (directly bonded) to each other or can be non-adjacent (preferably, adjacent or separated by a chain of no more than 4 catenated atoms; more preferably, adjacent). The heteroatoms of the hydrogen bond donor and/or hydrogen bond acceptor can be located in the chain of catenated atoms of the spacer group or, alternatively, can be located in chain substituents.

Although hydrogen bond donors can also function as hydrogen bond acceptors (through a lone electron pair of the donor's heteroatom), the hydrogen bonding moiety preferably includes distinct donor and acceptor moieties. This can facilitate intramolecular (intermonomer) hydrogen bond formation. Although not wishing to be bound by theory, such intramolecular hydrogen bonds between adjacent monomer or near neighbor repeat units in the polymer molecule may contribute to at least a degree of spacer group stiffening, which may facilitate presentation of the acidic group(s) or salt(s) thereof for interaction with aggregated proteins, particularly aggregated monoclonal antibodies.

Surprisingly, the acidic groups or salts thereof of such polymers can be especially effective in interacting with aggregated proteins, relative to monomeric proteins. Aggregated protein binding capacities can be surprisingly further enhanced by including at least one hydrogen bonding moiety in the linking chain of the monomer.

In certain embodiments, hydrogen bonding moieties include carbonylimino, thiocarbonylimino, iminocarbonylimino, iminothiocarbonylimino, oxycarbonylimino, oxythiocarbonylimino, and the like, and combinations thereof. In certain embodiments, hydrogen bonding moieties include carbonylimino, iminocarbonylimino, oxycarbonylimino, and combinations thereof (more preferably, carbonylimino, iminocarbonylimino, and combinations thereof). In certain embodiments, spacer groups include those that are divalent, trivalent, or tetravalent (more preferably, divalent or trivalent; and even more preferably, divalent).

A class of useful monomers includes those represented by the following general Formula I:

$$CH_2{=}CR^1{-}C({=}O){-}X{-}R^2{-}[Z{-}R^2]_n{-}L \qquad (I)$$

wherein:

$R^1$ is selected from hydrogen, alkyl, cycloalkyl, aryl, and combinations thereof;
each $R^2$ is independently selected from hydrocarbylene, heterohydrocarbylene, and combinations thereof;
X is -O- or $-NR^3-$, where $R^3$ is selected from hydrogen, hydrocarbyl, heterohydrocarbyl, and combinations thereof;
Z is heterohydrocarbylene including at least one hydrogen bond donor, at least one hydrogen bond acceptor, or a combination thereof;
n is an integer of 0 or 1; and
L is a functional group including at least one acidic group or salt thereof.

In certain embodiments of Formula I, $R^1$ is hydrogen or alkyl (more preferably, hydrogen or $C_1$ to $C_4$ alkyl; and even more preferably, hydrogen or methyl).

In certain embodiments, each $R^2$ is independently hydrocarbylene or heterohydrocarbylene. In certain embodiments, each $R^2$ is independently aralkylene (i.e., an alkylene substituted with an aryl), heteroaralkylene, hydroxy-substituted alkylene, or a hydroxy-substituted aralkylene. In certain embodiments, each $R^2$ is independently hydrocarbylene. In certain embodiments, each $R^2$ is independently alkylene).

In certain embodiments, X is -O- or $-NR^3-$, where $R^3$ is hydrogen.

In certain embodiments, Z is heterohydrocarbylene including at least one moiety selected from carbonyl, carbonylimino, carbonyloxy, ether oxygen, thiocarbonylimino, iminocarbonylimino, iminothiocarbonylimino, oxycarbonylimino, oxythiocarbonylimino, and combinations thereof (more preferably, selected from carbonyl, carbonylimino, carbonyloxy, ether oxygen, iminocarbonylimino, oxycarbonylimino, and combinations thereof; even more preferably, selected from carbonylimino, carbonyloxy, ether oxygen, iminocarbonylimino, and combinations thereof; and even more preferably, selected from carbonylimino, iminocarbonylimino, and combinations thereof).

In certain embodiments, n is an integer of 1.

In certain embodiments, L is a functional group including at least one acidic group or salt thereof selected from carboxy, phosphono, phosphate, sulfono, sulfato, boronato, and combinations thereof (more preferably, selected from carboxy, phosphono, sulfono, and combinations thereof).

Such monomers can be prepared by known synthetic methods or by analogy to known synthetic methods. For example, amino group-containing carboxylic, sulfonic, or phosphonic acids can be reacted with ethylenically unsaturated compounds that include at least one group that is reactive with an amino group. Similarly, acidic group-containing compounds that also contain a hydroxy group can be reacted with ethylenically unsaturated compounds that include at least one group that is reactive with a hydroxy group, optionally in the presence of a catalyst.

Preferred monomers are (meth)acryloyl-functional. As used herein, the term "(meth)acryloyl-functional" refers to acryloyl-functional and/or methacryloyl-functional; similarly, the term "(meth)acrylate" refers to an acrylate and/or a methacrylate. In such monomers, the carbonyl group is part of the spacer group.

Representative examples of useful monomers include those derived from the reaction of an alkenyl azlactone of general Formula II:

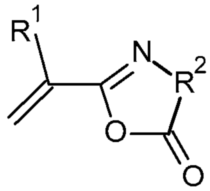

(II)

or an ethylenically unsaturated isocyanate of general Formula III:

$CH_2=C(R^1)-C(=O)-X-R^2-N=C=O$ (III)

with an acidic group-containing compound (or salt thereof) of general Formula IV:

$H-X-R^2-L$ (IV)

to produce monomer of general Formula I (wherein $R^1$, X, $R^2$, and L in Formulas II, III, and/or IV are as defined above for Formula I).

In certain embodiments, filter media used in processes of the present disclosure can be made by subjecting a mixture of monomers of general Formula I, for example, to radiation (e.g., electron beam radiation) in the presence of a suitable substrate. A first monomer from the mixture of monomers is attached to the surface of the substrate creating a radical that reacts with a second monomer to form another radical that reacts with a third monomer, etc. In this way, a polymer can be grafted to the substrate surface.

In an alternative, although not necessarily preferred, method for the preparation of filter media as described herein, a porous substrate can be grafted, in a first step, with a monomer of general Formula II or general Formula III. The grafted substrate can then, in a second step, be reacted with the acidic group-containing compound of general Formula IV. The first step can be carried out using any of the common processes known for grafting of monomers to substrates (e.g., by using UV, gamma, and/or e-beam irradiation), with the precaution that solvents that are not reactive with the monomers of Formulas II and III are used. In the second step, a solvent that does not react with the azlactone group or isocyanate group of the grafted polymer, but that does dissolve the acidic group-containing compound of Formula IV is chosen. Such precautions are taken to minimize competing hydrolysis or solvolysis of the very reactive azlactone and isocyanate groups. Optionally, the acidic group-containing compound may be neutralized (converted to the salt form) prior to or subsequent to reaction.

Representative examples of useful alkenyl azlactones of Formula II include 4,4-dimethyl-2-vinyl-4H-oxazol-5-one (vinyldimethylazlactone, VDM), 2-isopropenyl-4H-oxazol-5-one, 4,4-dimethyl-2-isopropenyl-4H-oxazol-5-one, 2-vinyl-4,5-dihydro-[1,3]oxazin-6-one, 4,4-dimethyl-2-vinyl-4,5-dihydro-[1,3]oxazin-6-one, 4,5-dimethyl-2-vinyl-4,5-dihydro-[1,3]oxazin-6-one, and the like, and combinations thereof.

Representative examples of ethylenically unsaturated isocyanates of general Formula III include 2-isocyanatoethyl (meth)acrylate (IEM or IEA), 3-isocyanatopropyl (meth)acrylate, 4-isocyanatocyclohexyl (meth)acrylate, and the like, and combinations thereof.

Representative examples of useful compounds of general Formula IV include amino group-containing carboxylic, sulfonic, boronic, and phosphonic acids and combinations thereof, or salts thereof. Useful amino carboxylic acids include $\alpha$-amino acids (L-, D-, or DL-$\alpha$-amino acids) such as glycine, alanine, valine, proline, serine, phenylalanine, histidine, tryptophan, asparagine, glutamine, N-benzylglycine, N-phenylglycine, sarcosine, and the like; $\beta$-aminoacids such as $\beta$-alanine, homoleucine, homoglutamine, homophenylalanine, and the like; other $\alpha,\omega$-aminoacids such as $\gamma$-aminobutyric acid, 6-aminohexanoic acid, 11-aminoundecanoic acid, peptides (such as diglycine, triglycine, tetraglycine, as well as other peptides containing a mixture of different aminoacids), and the like; and combinations thereof. Useful amino sulfonic acids include aminomethanesulfonic acid, 2-aminoethanesulfonic acid (taurine), 3-amino-1-propanesulfonic acid, 6-amino-1-hexanesulfonic acid, and the like, and combinations thereof. Useful aminoboronic acids include m-aminophenylboronic acid, p-aminophenylboronic acid, and the like, and combinations thereof. Useful aminophosphonic acids include 1-aminomethylphosphonic acid, 2-aminoethylphosphonic acid, 3-aminopropylphosphonic acid, and the like, and combinations thereof.

Useful compounds containing more than one acidic group include aspartic acid, glutamic acid, $\alpha$-aminoadipic acid, iminodiacetic acid, $N_\alpha,N_\alpha$-bis(carboxymethyl)lysine, cysteic acid, N-phosphonomethylglycine, and the like, and combinations thereof.

Representative examples of other useful acidic group-containing compounds include compounds including a hydroxy group and an acidic group. Specific examples include glycolic acid, lactic acid, 6-hydroxyhexanoic acid, citric acid, 2-hydroxyethylsulfonic acid, 2-hydroxyethylphosphonic acid, and the like, and combinations thereof.

Many of the above-described acidic group-containing compounds are commercially available. Still other useful acidic group-containing compounds can be prepared by common synthetic procedures. For example, various diamines or aminoalcohols can be reacted with one equivalent of a cyclic anhydride to produce an intermediate acidic group-containing compound including a carboxyl group and an amino or hydroxy group.

Useful monomers can also be prepared by the reaction of acidic group-containing compounds with ethylenically unsaturated acyl halides (for example, (meth)acryloyl chloride). In addition, useful monomers can be prepared by reaction of hydroxy- or amine-containing (meth)acrylate or (meth)acrylamide monomers with a cyclic anhydride to produce carboxyl group-containing monomers.

In certain embodiments, useful monomers may be prepared from the reaction of alkenyl azlactones with aminocarboxylic acids, monomers prepared from the reaction of alkenyl azlactones with aminosulfonic acids, monomers prepared from the reaction of ethylenically unsaturated isocyanates with aminocarboxylic acids, monomers prepared from the reaction of ethylenically unsaturated isocyanates with aminosulfonic acids, or combinations thereof.

In certain embodiments, useful monomers are the following (which are shown as acids, but salts of such acids may also be used):

VDM-4-aminomethyl-cyclohexanecarboxylic acid:

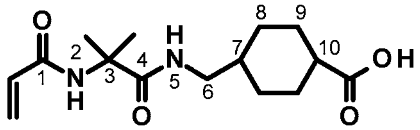

,

VDM-2-hydroxy-4-aminobutanoic acid:

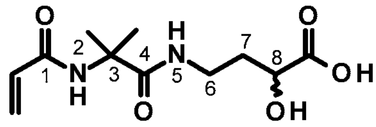

,

VDM-2-amino-3-hydroxypropanoic acid (VDM-serine):

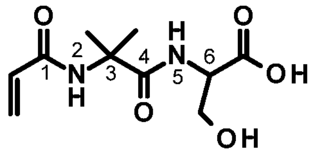

,

VDM-2-amino-3-(4-hydroxyphenyl)propanoic acid (VDM-tyrosine):

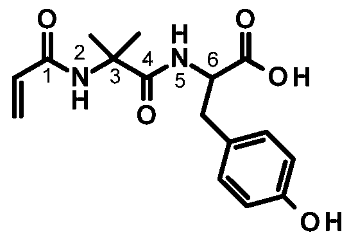

,

VDM-(2S)-2-amino-3-(1H-indol-3-yl)propanoic acid (VDM-tryptophan):

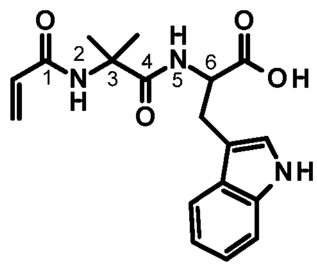

,

VDM-7-aminoheptanoic acid:

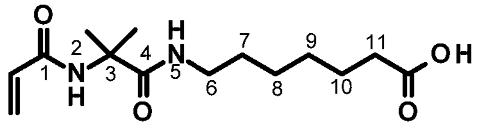

,

VDM-2-amino-3-(1H-imidazol-4-yl)propanoic acid (VDM-histidine):

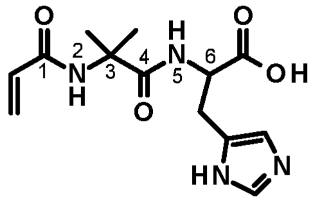

,

IEM-3-aminopropanoic acid:

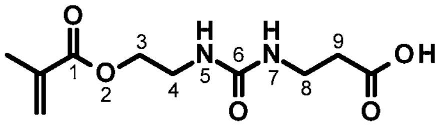

,

IEM-taurine:

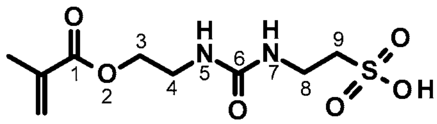

,

VDM-taurine:

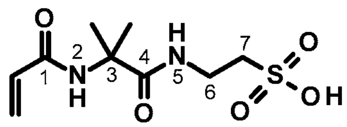

,

VDM-2-(hydroxyethyl)phosphonic acid:

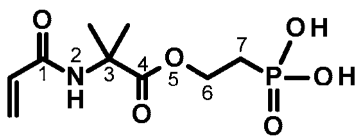

,

VDM-3-aminopropanoic acid:

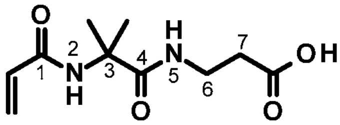

,

VDM-4-aminobutyric acid:

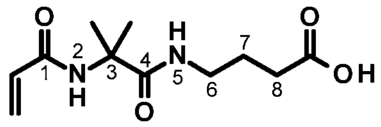

,

VDM-5-aminovaleric acid:

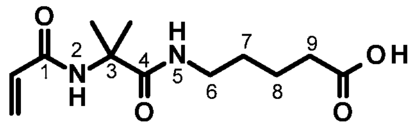

,

VDM-6-aminocaproic acid:

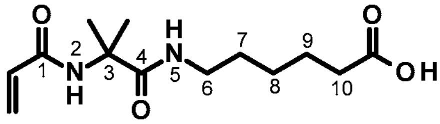

,

VDM-Phenylalanine:

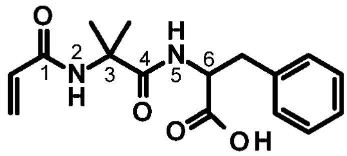

,

and
IEM-Phenylalanine:

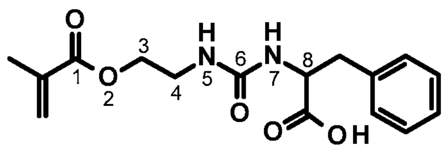

.

The above-described monomers generally (and preferably) can be homopolymerized.

A skilled artisan will recognize, however, that the monomers can be copolymerized with other monomers (hereinafter, termed "modifying comonomers"; for example, modifying comonomers having shorter spacer groups or modifying comonomers including other types of ligands or even modifying comonomers that are ligand-free) in order to adjust binding capacity and/or to achieve special properties, provided that the type and degree of binding capacity desired for a particular application can be achieved.

For example, the monomer(s) optionally can be copolymerized with one or more hydrophilic modifying comonomer(s) including at least one alkenyl group (preferably, a (meth)acryloyl group) and a hydrophilic group (including, for example, a poly(oxyalkylene) group, hydroxyl group, amino group, or amido group) in order to impart a degree of hydrophilicity to the porous substrate. Suitable hydrophilic modifying comonomers include acrylamide, dimethylacrylamide, hydroxyethyl(meth)acrylate, hydroxypropyl(meth)acrylate, polyethyleneglycolmono(meth)acrylate, 2-hydroxyethylacrylamide, N-vinylpyrrolidone, and the like, and combinations thereof.

Optionally, the monomer(s) can be copolymerized with one or more modifying (meth)acryloyl comonomer(s) containing at least two free radically polymerizable groups. Such modifying multifunctional (meth)acryloyl comonomer(s) (including multifunctional (meth)acrylate(s) and (meth)acrylamide(s)) can be incorporated in a blend of polymerizable

monomer(s) generally in only relatively small amounts (for example, from 0.1 to 5 percent by weight, based upon the total weight of monomer(s) and comonomer(s)) to impart a degree of branching and/or relatively light crosslinking to a resulting copolymer. Higher amounts can be used for certain applications, but it should be understood that the use of higher amounts may reduce binding capacity for aggregated proteins.

Useful modifying multifunctional (meth)acryloyl comonomers include di(meth)acrylates, tri(meth)acrylates, tetra(meth)acrylates, multifunctional (meth)acrylamides, and the like, and combinations thereof. Such multifunctional (meth)acryloyl comonomers include ethyleneglycol di(meth)acrylate, 1,6-hexanediol di(meth)acrylate, polyethylene glycol) di(meth)acrylates, polybutadiene di(meth)acrylate, polyurethane di(meth)acrylates, propoxylated glycerin tri(meth)acrylate, methylenebisacrylamide, ethylenebisacrylamide, hexamethylenebisacrylamide, diacryloylpiperazine, and the like, and combinations thereof.

**Filter Media**

Polymerization of the monomer(s) can be carried out by using known techniques. For example, the polymerization can be initiated with either a thermal initiator or a photoinitiator (preferably, a photoinitiator). A wide variety of conventional free radical initiators can be used to generate the initial radical. Examples of suitable thermal initiators include peroxides such as benzoyl peroxide, dibenzoyl peroxide, dilauryl peroxide, cyclohexane peroxide, methyl ethyl ketone peroxide, hydroperoxides (for example, tert-butyl hydroperoxide and cumene hydroperoxide), dicyclohexyl peroxydicarbonate, t-butyl perbenzoate; and the like; and combinations thereof. Examples of commercially available thermal initiators include initiators available from DuPont Specialty Chemical (Wilmington, DE) under the VAZO trade designation including VAZO 67 (2,2'-azo-bis(2-methylbutyronitrile)), VAZO 64 (2,2'-azo-bis(isobutyronitrile)), and VAZO 52 (2,2'-azo-bis(2,2-dimethylvaleronitrile)), as well as that available under the trade designation LUCIDOL 70 (benzoylperoxide) from Elf Atochem North America (Philadelphia, PA).

Useful photoinitiators include benzoin ethers such as benzoin methyl ether and benzoin isopropyl ether; substituted acetophenones such as 2,2-dimethoxyacetophenone available as IRGACURE 651 photoinitiator (Ciba Specialty Chemicals), 2,2 dimethoxy-2-phenyl-1-phenylethanone available as ESACURE KB-1 photoinitiator (Sartomer Co.; West Chester, PA), 1-[4-(2-hydroxyethoxy)phenyl]-2-hydroxy-2-methyl-1-propan-1-one available as IRGACURE 2959 (Ciba Specialty Chemicals), and dimethoxyhydroxyacetophenone; substituted $\alpha$-ketols such as 2- methyl-2-hydroxy propiophenone; aromatic sulfonyl chlorides such as 2-naphthalene-sulfonyl chloride; photoactive oximes such as 1-phenyl-1,2-propanedione-2-(O-ethoxy-carbonyl)oxime; and the like; and combinations thereof. Particularly preferred among these are the substituted acetophenones (especially 1-[4-(2-hydroxyethoxy)phenyl]-2-hydroxy-2-methyl-1-propan-1-one, IRGACURE 2959, due to its water solubility). A useful polymerizable photoinitiator is a 1:1 adduct of 2-vinyl-4,4-dimethylazlactone and IRGACURE 2959, which can be prepared essentially as described in Example 1 of U.S. Patent No. 5,506,279 (Babu et al.).

Other useful photoinitiators include hydrogen-abstracting (Type II) photoinitiators such as benzophenone, 4-(3-sulfopropyloxy)benzophenone sodium salt, Michler's ketone, benzil, anthraquinone, 5,12-naphthacenequinone, aceanthracenequinone, benz(A)anthracene-7,12-dione, 1,4-chrysenequinone, 6,13-pentacenequinone, 5,7,12,14-pentacenetetrone, 9-fluorenone, anthrone, xanthone, thioxanthone, 2-(3-sulfopropyloxy)thioxanthen-9-one, acridone, dibenzosuberone, acetophenone, chromone, and the like, and combinations thereof.

The initiator can be used in an amount effective to initiate free radical polymerization of the monomer(s). Such amount will vary depending upon, for example, the type of initiator and polymerization conditions utilized. The initiator generally can be used in amounts ranging from 0.01 part by weight to 5 parts by weight, based upon 100 parts total monomer.

The polymerization solvent can be essentially any solvent that can substantially dissolve (or, in the case of emulsion or suspension polymerizations, disperse or suspend) the monomer(s) (and comonomer(s), if used). In many embodiments, the solvent can be water or a water/water-miscible organic solvent mixture. The ratio of water to organic solvent can vary widely, depending upon monomer solubility. With some monomers, the ratio typically can be greater than 1:1 (volume/volume) water to organic solvent (preferably, greater than 5:1; more preferably, greater than 7:1). With other monomers, a higher proportion of organic solvent (even up to 100 percent) can be preferred (with some alcohols especially).

Any such water-miscible organic solvent preferably has no groups that would retard polymerization. In some embodiments, the water-miscible solvents can be protic group-containing organic liquids such as the lower alcohols having 1 to 4 carbon atoms, lower glycols having 2 to 6 carbon atoms, and lower glycol ethers having 3 to 6 carbon atoms and 1 to 2 ether linkages. In some embodiments, higher glycols such as polyethylene glycol) can be used. Specific examples include methanol, ethanol, isopropanol, n-butanol, t-butyl alcohol, ethylene glycol, methoxyethanol, ethoxyethanol, propoxyethanol, butoxyethanol, methyl carbitol, ethyl carbitol, and the like, and combinations thereof.

In other embodiments, non-protic water-miscible organic solvents can be used. Such solvents include aliphatic esters (for example, methoxyethyl acetate, ethoxyethyl acetate, propoxyethyl acetate, butoxyethyl acetate, and triethyl

phosphate), ketones (for example, acetone, methyl ethyl ketone, and methyl propyl ketone), and sulfoxides (for example, dimethyl sulfoxide).

The monomer concentration in the polymerization solvent can vary, depending upon a number of factors including, but not limited to, the nature of the monomer or monomers, the extent of polymerization desired, the reactivity of the monomer(s), and the solvent used. Typically, the monomer concentration can range from 0.1 weight percent (wt%) to 60 wt% (preferably, from 1 wt% to 40 wt%; more preferably, from 5 wt% to 30 wt%), based upon the total weight of monomer and solvent.

An aqueous monomer mixture optionally can be formulated with relatively higher levels of multifunctional (crosslinking) monomers or modifying comonomers (for example, from 5 wt% to 90 wt%, based upon the total weight of monomer(s) and comonomer(s)) and polymerized as a suspension or dispersion in a nonpolar, immiscible organic solvent, optionally in the presence of added porogen(s), to produce crosslinked, porous particles including the instant monomer(s). Such methods are well known and are described, for example, in U.S. Patent Nos. 7, 098,253 (Rasmussen et al.), 7, 674,835 (Rasmussen et al.), 7,647,836 (Rasmussen et al.), and 7, 683,100 (Rasmussen et al.).

If desired, the polymerization can be carried out in the presence of a porous substrate, so as to form an article including a porous substrate bearing the resulting polymer. For example, an imbibing or coating solution including the monomer(s), any comonomer(s), initiator(s), and solvent(s) can be imbibed by or coated (or otherwise deposited) on a porous substrate.

The porous substrate can be in essentially any form such as particles, fibers, films, webs, membranes, sponges, or sheets. Suitable porous substrates can be organic, inorganic, or a combination thereof (preferably, organic; more preferably, polymeric). Suitable porous substrates include porous particles, porous membranes, porous nonwoven webs, porous woven webs, porous sponges, porous fibers, and the like, and combinations thereof. Preferred porous substrates include porous membranes (more preferably, porous polymeric membranes; even more preferably, porous polyamide membranes) and combinations thereof.

For example, the porous substrate can be formed from any suitable thermoplastic polymeric material. Suitable polymeric materials include polyolefins, poly(isoprenes), poly(butadienes), fluorinated polymers, chlorinated polymers, polyamides, polyimides, polyethers, poly(ether sulfones), poly(sulfones), poly(vinyl acetates), polyesters such as poly(lactic acid), copolymers of vinyl acetate such as poly(ethylene)-co-poly(vinyl alcohol), poly(phosphazenes), poly(vinyl esters), poly(vinyl ethers), poly(vinyl alcohols), poly(carbonates), and the like, and combinations thereof.

In some embodiments, the thermoplastic polymer can be surface treated, such as by plasma discharge or by use of a primer, to provide suitable functionality to the surface of the porous substrate. Surface treatment can provide functional groups such as hydroxy groups that can improve wetting by the monomer solution. One such useful plasma treatment is described in U.S. Patent No. 7,125,603 (David et al.).

Suitable polyolefins include poly(ethylene), poly(propylene), poly(1-butene), copolymers of ethylene and propylene, alpha olefin copolymers (such as copolymers of ethylene or propylene with 1-butene, 1-hexene, 1-octene, and 1-decene), poly(ethylene-*co*-1-butene), poly(ethylene-co-1-butene-co-1-hexene), and the like, and combinations thereof.

Suitable fluorinated polymers include poly(vinyl fluoride), poly(vinylidene fluoride), copolymers of vinylidene fluoride (such as poly(vinylidene fluoride-*co*-hexafluoropropylene)), copolymers of chlorotrifluoroethylene (such as poly(ethylene-*co*-chlorotrifluoroethylene)), and the like, and combinations thereof.

Suitable polyamides include poly(iminoadipolyliminohexamethylene), poly(iminoadipolyliminodecamethylene), polycaprolactam, and the like, and combinations thereof. Suitable polyimides include poly(pyromellitimide), and the like, and combinations thereof.

Suitable poly(ether sulfones) include poly(diphenylether sulfone), poly(diphenylsulfone-*co*-diphenylene oxide sulfone), and the like, and combinations thereof.

Suitable copolymers of vinyl acetate include poly(ethylene-*co*-vinyl acetate), such copolymers in which at least some of the acetate groups have been hydrolyzed to afford various poly(vinyl alcohols), and the like, and combinations thereof.

A preferred porous substrate is a microporous membrane such as a thermally induced phase separation (TIPS) membrane. TIPS membranes are often prepared by forming a solution of a thermoplastic material and a second material above the melting point of the thermoplastic material. Upon cooling, the thermoplastic material crystallizes and phase separates from the second material. The crystallized material is often stretched. The second material is optionally removed either before or after stretching. Microporous membranes are further described in U.S. Patent Nos. 4,539,256 (Shipman); 4,726,989 (Mrozinski); 4,867,881 (Kinzer); 5,120,594 (Mrozinski); 5,260,360 (Mrozinski); and 5,962,544 (Waller, Jr.). Some exemplary TIPS membranes include poly(vinylidene fluoride) (PVDF), polyolefins such as poly(ethylene) or poly(propylene), vinyl-containing polymers or copolymers such as ethylene-vinyl alcohol copolymers and butadiene-containing polymers or copolymers, and acrylate-containing polymers or copolymers. For some applications, a TIPS membrane including PVDF can be particularly desirable. TIPS membranes including PVDF are further described in U.S. Patent No. 7,338,692 (Smith et al.).

In many embodiments, the porous substrate can have an average pore size that is typically greater than 0.2

micrometer in order to minimize size exclusion separations, minimize diffusion constraints, and maximize surface area and separation based on binding of aggregated proteins, particularly aggregated antibodies, and more particularly aggregated monoclonal antibodies. Generally, the pore size can be in the range of 0.1 to 10 micrometers (preferably, 0.5 to 3 micrometers; and more preferably, 0.8 to 2 micrometers when used for binding aggregated proteins).

In an exemplary embodiment, the porous substrate can include a nylon microporous film or sheet (for example, a microporous membrane), such as those described in U.S. Patent Nos. 6,056,529 (Meyering et al.), 6,267,916 (Meyering et al.), 6,413,070 (Meyering et al.), 6,776,940 (Meyering et al.), 3,876,738 (Marinaccio et al.), 3,928,517 (Knight et al.), 4,707,265 (Barnes, Jr. et al.), and 5,458,782 (Hou et al.).

In other embodiments, the porous substrate can be a nonwoven web, which can include nonwoven webs manufactured by any of the commonly known processes for producing nonwoven webs. As used herein, the term "nonwoven web" refers to a fabric that has a structure of individual fibers or filaments that are randomly and/or unidirectionally interlaid in a mat-like fashion.

For example, the fibrous nonwoven web can be made by wet laid, carded, air laid, spunlaced, spunbonding, or melt-blowing techniques, or combinations thereof. Spunbonded fibers are typically small diameter fibers that are formed by extruding molten thermoplastic polymer as filaments from a plurality of fine, usually circular capillaries of a spinneret, with the diameter of the extruded fibers being rapidly reduced. Meltblown fibers are typically formed by extruding molten thermoplastic material through a plurality of fine, usually circular, die capillaries as molten threads or filaments into a high velocity, usually heated gas (for example, air) stream, which attenuates the filaments of molten thermoplastic material to reduce their diameter. Thereafter, the meltblown fibers are carried by the high velocity gas stream and are deposited on a collecting surface to form a web of randomly-dispersed, meltblown fibers. Any of the nonwoven webs can be made from a single type of fiber or from two or more fibers that differ in the type of thermoplastic polymer and/or thickness.

Further details of manufacturing methods of useful nonwoven webs have been described by Wente in "Superfine Thermoplastic Fibers," 48 Indus. Eng. Chem. 1342 (1956) and by Wente et al. in "Manufacture Of Superfine Organic Fibers," Naval Research Laboratories Report No. 4364 (1954).

Following polymerization, washing, and drying, typical total weight gains by the porous substrate generally can be in the range of 0.5 percent (%) to 30% (preferably, in the range of 2% to 10%). Polymerization of the monomer(s) in the presence of a porous substrate can produce a polymer-bearing porous substrate. The polymer can be in the form of a coating or, in preferred embodiments, the polymer can be grafted (covalently bonded) to the surface of the porous substrate. If desired, the polymerization can be carried out separately and the resulting polymer then coated (optionally in the presence of suitable crosslinker) or grafted or otherwise applied to the porous substrate, but this is generally less preferred.

In an exemplary method, the monomer(s) can be free radically polymerized and grafted to the surface of a porous substrate in the presence of a Type II photoinitiator, as described in Patent Application No. US 2015/0136698 (3M Innovative Properties Co.). Alternatively, the monomer(s) can be free radically polymerized and grafted to a porous substrate including a crosslinked copolymer layer, the copolymer including photoinitiator-containing monomer units, as described in International Publication No. WO 2014/052215 (Rasmussen et al.). In addition, the monomer(s) can be free radically polymerized and grafted to a porous substrate including a crosslinked polymer primer layer, as described in U.S. Patent Application Publication No. 2012/0252091 A1 (Rasmussen et al.). In yet another exemplary method, the monomer(s) can be free radically polymerized and grafted to a porous particle, as described in U.S. Patent Application Publication No. 2011/0100916 A1 (Shannon et al.).

In certain embodiments, acidic groups or salts thereof of the polymer are disposed on the porous substrate at a density of at least 0.02 millimole per gram (mmole/gram) of filter media, or at least 0.03 mmole/gram of filter media, or at least 0.04 mmole/gram of filter media, or at least 0.05 mmole/gram of filter media. In certain embodiments, acidic groups or salts thereof of the polymer are disposed on the porous substrate at a density of up to 0.6 mmole/gram of filter media, or up to 0.5 mmole/gram of filter media, or up to 0.4 mmole/gram of filter media, up to 0.35 mmole/gram, or up to 0.3 mmole/gram, of filter media.

The coated or grafted polymer (which is a functional polymer due to the presence of the acidic groups, or salts thereof, of the monomer(s)) can alter the original nature of the porous substrate. The resulting polymer-bearing porous substrates (functionalized porous substrates) can retain many of the advantages of the original porous substrate (for example, mechanical and thermal stability, porosity, and so forth) but can also exhibit enhanced binding capacity for aggregated proteins. Porous substrates bearing the functional polymer can be particularly useful as filter media for the selective binding and/or removal of aggregated proteins, relative to monomeric proteins, from biological samples. Articles including the polymer-bearing porous substrates can further include conventional components such as housings, holders, adapters, and the like, and combinations thereof.

If desired, efficiency of binding and capture of aggregated proteins can be improved by using a plurality of stacked or layered, functionalized porous substrates (for example, functionalized porous membranes) as a filter element. Thus, a filter element can include one or more layers of functionalized porous substrate. The individual layers of the

filter element can be the same or different. The layers can vary in porosity, degree of grafting, and so forth. The filter element can further include an upstream prefilter layer and/or a downstream support layer. The individual layers can be planar or pleated, as desired.

Examples of suitable prefilter and support layer materials include any suitable porous membranes of polypropylene, polyester, polyamide, resin-bonded or binder-free fibers (for example, glass fibers), and other synthetics (woven and nonwoven fleece structures); sintered materials such as polyolefins, metals, and ceramics; yarns; special filter papers (for example, mixtures of fibers, cellulose, polyolefins, and binders); polymer membranes; and the like; and combinations thereof.

Useful articles for aggregated protein capture or filtration applications include a filter cartridge including one or more of the above-described filter elements, a filter assembly including one or more of the above-described filter elements and a filter housing, and the like. Other useful articles can be in the form of an array of sample containers (e.g., a 96-well plate) with each container including a filter element having a contacting surface (e.g., an upstream or upper surface).

## EMBODIMENTS

Embodiment 1 is a process for separating aggregated proteins from monomeric proteins in a biological solution, the process comprising:

providing at least one filter element having a contacting surface (e.g., an upstream or upper surface), wherein the filter element comprises filter media comprising:

a porous substrate; and
disposed on the porous substrate, a polymer comprising a hydrocarbon backbone and a plurality of pendant groups attached to the hydrocarbon backbone, wherein each of a first plurality of pendant groups comprises:

(1) at least one acidic group or salt thereof; and
(2) a spacer group that directly links the at least one acidic group or salt thereof to the hydrocarbon backbone by a chain of at least 6 catenated atoms; and

allowing an initial biological solution to contact the contacting surface of the filter element under conditions effective to separate the aggregated proteins from the monomeric proteins such that a final biological solution includes purified monomeric proteins.

Embodiment 2 is a process for separating aggregated proteins from monomeric proteins in a biological solution, the process comprising:

providing at least one filter element having a contacting surface (e.g., an upstream or upper surface), wherein the filter element comprises filter media comprising:

a porous substrate; and
disposed on the porous substrate, a polymer comprising interpolymerized units of at least one monomer comprising (in some embodiments, consisting of):

(1) at least one ethylenically unsaturated group (in some embodiments, a terminal ethylenically unsaturated group);
(2) at least one acidic group or salt thereof; and
(3) a spacer group that directly links the at least one ethylenically unsaturated group and the at least acidic group or salt thereof by a chain of at least 6 catenated atoms; and

allowing an initial biological solution to contact the contacting surface of the filter element under conditions effective to separate the aggregated proteins from the monomeric proteins such that a final biological solution includes purified monomeric proteins.

Embodiment 3 is the process of embodiment 1 or 2 further comprising recovering the monomeric proteins without eluting.

Embodiment 4 is the process of any one of embodiments 1 through 3 wherein the conditions are effective to recover, in a final solution, at least 70%, or at least 75%, or at least 80%, or at least 85%, or at least 90%, or at least 95%, of the monomeric proteins present in the initial biological solution.

Embodiment 5 is the process of any one of embodiments 1 through 4 wherein the conditions are effective to remove at least 10%, or at least 15%, or at least 20%, or at least 25%, or at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, of the aggregated proteins from the initial biological solution.

Embodiment 6 is the process of any one of embodiments 1 through 5 wherein the proteins comprise antibodies, enzymes, or hormones.

Embodiment 7 is the process of embodiment 6 wherein the proteins comprise antibodies.

Embodiment 8 is the process of embodiment 7 wherein the antibodies comprise monoclonal antibodies, or fragments thereof.

Embodiment 9 is the process of any one of embodiments 1 through 8 wherein the conditions comprise a pH of the biological solution of below 9, or below 8.5, or below 8, or below 7.5, or below 7, or below 6.5.

Embodiment 10 is the process of any one of embodiments 1 through 9 wherein the conditions comprise a pH of the biological solution of at least 4, or at least 4.5, or at least 5.

Embodiment 11 is the process of any one of embodiments 1 through 10 wherein the conditions comprise a conductivity of the biological solution of at least 1 mS/cm, or at least 2 mS/cm, or at least 3 mS/cm, or at least 4 mS/cm, or at least 5 mS/cm, or at least 6 mS/cm, or at least 7 mS/cm, or at least 8 mS/cm, or at least 9 mS/cm, or at least 10 mS/cm.

Embodiment 12 is the process of embodiment 11 wherein the conductivity of the biological solution is provided by a buffer comprising an inorganic salt, organic salt, or a combination thereof.

Embodiment 13 is the process of embodiment 12 wherein the buffer salt comprises a chloride (e.g., NaCl), a phosphate, a citrate, a sulfate, an acetate, or a combination thereof.

Embodiment 14 is the process of any one of embodiments 1 through 13 wherein the conditions comprise a conductivity of the biological solution of no greater than 110 mS/cm, or no greater than 100 mS/cm, or no greater than 50 mS/cm, or no greater than 40 mS/cm, or no greater than 30 mS/cm.

Embodiment 15 is the process of any one of embodiments 1 through 14 wherein the conditions comprise a challenge load of at least 1 g/L of filter media, or at least 10 g/L of filter media, or at least 25 g/L of filter media, or at least 50 g/L of filter media, or at least 75 g/L of filter media, or at least 100 g/L, or at least 500 g/L, or at least 1000 g/L, or at least 2000 g/L, of filter media.

Embodiment 16 is the process of any one of embodiments 1 through 15 wherein the porous substrate is polymeric.

Embodiment 17 is the process of any one of embodiments 1 through 16 wherein the porous substrate is a porous membrane.

Embodiment 18 is the process of any one of embodiments 1 through 17 wherein the at least one acidic group or salt thereof of the polymer disposed on the porous substrate is present at a density of at least 0.02 mmole/gram of filter media, or at least 0.03 mmole/gram of filter media, or at least 0.04 mmole/gram of filter media, or at least 0.05 mmole/gram of filter media.

Embodiment 19 is the process of any one of embodiments 1 through 18 wherein the at least one acidic group or salt thereof of the polymer disposed on the porous substrate is present at a density of up to 0.6 mmole/gram of filter media, or up to 0.5 mmole/gram of filter media, or up to 0.4 mmole/gram of filter media, up to 0.35 mmole/gram, or up to 0.3 mmole/gram, of filter media.

Embodiment 20 is the process of any one of embodiments 2 through 19, as dependent on embodiment 2, wherein the at least one monovalent ethylenically unsaturated group is selected from an ethenyl group, a 1-alkylethenyl group, and a combination thereof.

Embodiment 21 is the process of embodiment 20 wherein the at least one monomer comprises a (meth)acryloyl monomer.

Embodiment 22 is the process of any one of embodiments 1 through 21 wherein the at least one acidic group or salt thereof is selected from a carboxy group, a phosphono group, a phosphate group, a sulfono group, a sulfato group, a boronato group, and a combination thereof.

Embodiment 23 is the process of embodiment 22 wherein the at least one acidic group or salt thereof is selected from a carboxy group, a phosphono group, a sulfono group, and a combination thereof.

Embodiment 24 is the process of embodiment 23 wherein the at least one acidic group or salt thereof is a carboxy group.

Embodiment 25 is the process of any one of embodiments 1 through 24 wherein the spacer group is a catenated heteroatom-containing hydrocarbon group.

Embodiment 26 is the process of any one of embodiments 1 through 25 wherein the spacer group comprises at least one hydrogen bonding moiety.

Embodiment 27 is the process of embodiment 26 wherein the hydrogen bonding moiety is selected from a carbonylimino moiety, a thiocarbonylimino moiety, an iminocarbonylimino group, an iminothiocarbonylimino moiety, an oxycarbonylimino moiety, an oxythiocarbonylimino moiety, and a combination thereof.

Embodiment 28 is the process of embodiment 27 wherein the hydrogen bonding moiety is selected from a carbonylimino moiety, an iminocarbonylimino group, an oxycarbonylimino moiety, and a combination thereof.

Embodiment 29 is the process of any one of embodiments 1 through 28 wherein the chain of the spacer group has at least 7 catenated atoms.

Embodiment 30 is the process of embodiment 29 wherein the chain of the spacer group has at least 8 catenated atoms.

Embodiment 31 is the process of any one of embodiments 1 through 30 wherein the chain of the spacer group has no more than 50 catenated atoms.

Embodiment 32 is the process of embodiment 30 or 31 wherein the chain of the spacer group has from 9 to 16 catenated atoms.

Embodiment 33 is the process of any one of embodiments 2 through 32, as dependent on embodiment 2, wherein the at least one monomer is one of a class represented by the following general formula:

$$CH_2=CR^1-C(=O)-X-R^2-[Z-R^2]_n-L \qquad (I)$$

wherein:

$R^1$ is selected from hydrogen, an alkyl group, a cycloalkyl group, an aryl group, and a combination thereof;
each $R^2$ is independently selected from a hydrocarbylene group, a heterohydrocarbylene group, and a combination thereof;
X is -O- or -$NR^3$-, where $R^3$ is selected from hydrogen, a hydrocarbyl group, a heterohydrocarbyl group, and a combination thereof;
Z is a heterohydrocarbylene group comprising at least one hydrogen bond donor, at least one hydrogen bond acceptor, or a combination thereof;
n is an integer of 0 or 1; and
L is a functional group comprising at least one acidic group or salt thereof.

Embodiment 34 is the process of embodiment 33 wherein $R^1$ is selected from hydrogen, methyl, or a combination thereof.

Embodiment 35 is the process of embodiment 34 wherein the at least one monomer is selected from:

VDM-4-aminomethyl-cyclohexanecarboxylic acid:

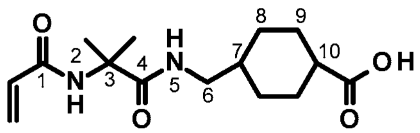,

VDM-2-hydroxy-4-aminobutanoic acid:

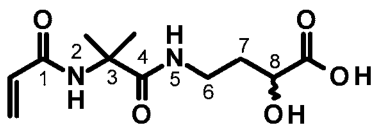,

VDM-2-amino-3-hydroxypropanoic acid (VDM-serine):

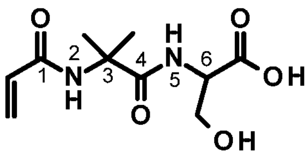,

VDM-2-amino-3-(4-hydroxyphenyl)propanoic acid (VDM-tyrosine):

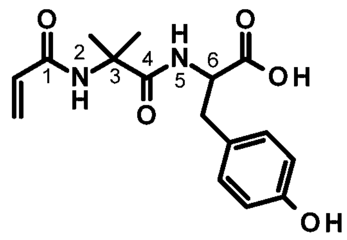

,

VDM-(2S)-2-amino-3-(1H-indol-3-yl)propanoic acid (VDM-tryptophan):

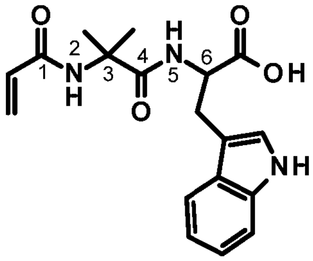

,

VDM-7-aminoheptanoic acid:

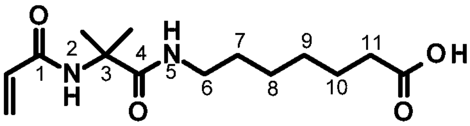

,

VDM-2-amino-3-(1H-imidazol-4-yl)propanoic acid (VDM-histidine):

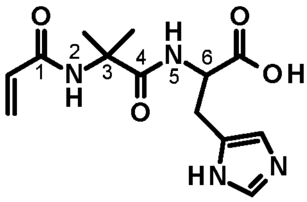

,

IEM-3-aminopropanoic acid:

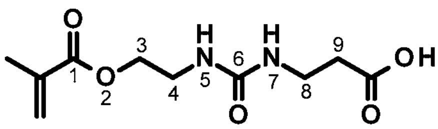

,

IEM-taurine:

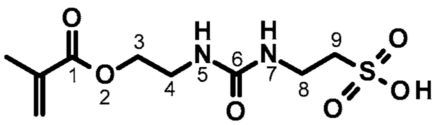

,

VDM-taurine:

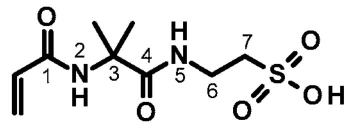

,

VDM-2-(hydroxyethyl)phosphonic acid:

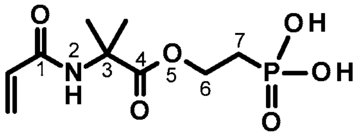

,

VDM-3-aminopropanoic acid:

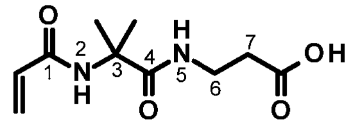

,

VDM-4-aminobutyric acid:

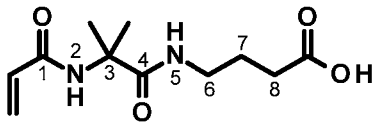

,

VDM-5-aminovaleric acid:

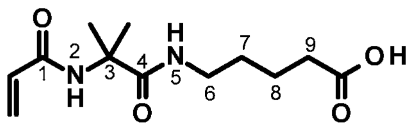

,

VDM-6-aminocaproic acid:

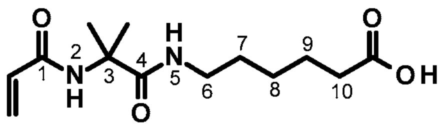

,

VDM-Phenylalanine:

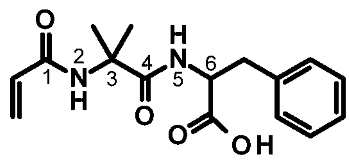

,

and
IEM-Phenylalanine:

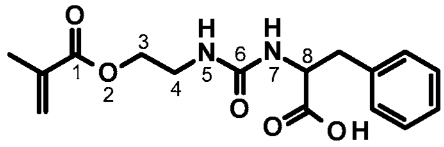

Embodiment 36 is the process of any one of embodiments 1 through 35 wherein the polymer is grafted to the porous substrate.
Embodiment 37 is the process of any one of embodiments 1 through 35 wherein the polymer is coated on the porous substrate.
Embodiment 38 is the process of any one of embodiments 2 through 37, as dependent on embodiment 2, wherein disposed on the porous substrate is a polymer comprising interpolymerized units of a modifying comonomer selected from a hydrophilic comonomer comprising at least one alkenyl group and at least one hydrophilic group.
Embodiment 39 is the process of embodiment 38 wherein the hydrophilic modifying comonomer is selected from acrylamide, dimethylacrylamide, hydroxyethyl(meth)acrylate, hydroxypropyl(meth)acrylate, polyethyleneglycolmono(meth)acrylate, 2-hydroxyethylacrylamide, N-vinylpyrrolidone, and a combination thereof.
Embodiment 40 is the process of any one of embodiments 2 through 39, as dependent on embodiment 2, wherein disposed on the porous substrate is a polymer comprising interpolymerized units of a modifying multifunctional (meth)acryloyl comonomer comprising at least two free radically polymerizable groups.
Embodiment 41 is the process of embodiment 40 wherein the modifying multifunctional (meth)acryloyl comonomer is selected from a di(meth)acrylate, a tri(meth)acrylate, a tetra(meth)acrylate, a multifunctional (meth)acrylamide, and a combination thereof.
Embodiment 42 is the process of embodiment 41 wherein the modifying multifunctional (meth)acryloyl comonomer is selected from ethyleneglycol di(meth)acrylate, 1,6-hexanediol di(meth)acrylate, polyethylene glycol) di(meth)acrylates, polybutadiene di(meth)acrylate, polyurethane di(meth)acrylates, propoxylated glycerin tri(meth)acrylate, methylenebisacrylamide, ethylenebisacrylamide, hexamethylenebisacrylamide, diacryloylpiperazine, and a combination thereof.

**EXAMPLES**

Objects and advantages of this disclosure are further illustrated by the following examples, but the particular materials and amounts thereof recited in these examples, as well as other conditions and details, should not be construed to unduly limit this disclosure. These examples are merely for illustrative purposes only and are not meant to be limiting on the scope of the appended claims.

Materials:

TABLE 1. Materials

| Description (abbreviation) | Source |
|---|---|
| 2-[Bis(2-hydroxyethyl)amino]-2-(hydroxymethyl)propane-1,3-diol (Bis-Tris) | Sigma-Aldrich, St. Louis, MO |
| 2-(N-morpholino)ethanesolfonic acid (MES) | Alfa Aesar, Ward Hill, MA |
| Sodium phosphate (monobasic and dibasic) | JT Baker, Phillipsburg, NJ |
| Tris(hydroxymethyl)-aminomethane (Tris) | Alfa Aesar, Ward Hill, MA |
| Sodium citrate | Alfa Aesar, Ward Hill, MA |
| Citric acid | Alfa Aesar, Ward Hill, MA |
| Vinyldimethylazlactone (VDM) | SNPE, Inc., Princeton, NJ |

(continued)

| Description (abbreviation) | Source |
|---|---|
| 2-Isocyanatoethyl methacrylate (IEM) | Showa Denko KK, Kanagawa, Japan |
| 4-(3-Sulfopropyloxy)benzophenone, sodium salt (S-BP) | Described in Japanese Patent No. 47040913 (Teijin Ltd.) |

IgG Antibody Solution Preparation:

Monoclonal antibody IgG (33.2 mg/mL, 3-4% aggregate content, pI >8, pH 5.3, 3.0 mS/cm) was diluted either 10-fold or 40-fold with a 20 mM buffer solution of citrate (pH 5 and 6), MES (pH 6), Bis-Tris (pH 6), sodium phosphate (pH 7), or Tris (pH 8). The resulting buffer solutions were adjusted with sodium chloride to have conductivities ranging between 1.5-105 mS/cm. The conductivity and pH measurements of the IgG-containing buffer solutions were determined using an Accumet Excel XL50 conductivity meter (Fisher Scientific, Hampton, NH) and a VWR sympHony™ benchtop pH meter (VWR, Radnor, PA), respectively. In Table 2, the buffer, pH and conductivity for each of the IgG-containing buffer solutions (IgG Solution Numbers 1-27) are recorded.

TABLE 2

| IgG-containing solutions used for examples | | | |
|---|---|---|---|
| IgG Solution Number | Buffer (20 mM) | pH | Conductivity (mS/cm) |
| 1 | Citrate | 5 | 3.4 |
| 2 | Citrate | 5 | 10.5 |
| 3 | Citrate | 5 | 22.1 |
| 4 | Citrate | 5 | 35.0 |
| 5 | Citrate | 5 | 99.7 |
| 6 | Citrate | 6 | 2.8 |
| 7 | Citrate | 6 | 9.9 |
| 8 | Citrate | 6 | 20.5 |
| 9 | Citrate | 6 | 36.8 |
| 10 | Citrate | 6 | 98.3 |
| 11 | MES | 6 | 1.7 |
| 12 | MES | 6 | 9.0 |
| 13 | MES | 6 | 20.1 |
| 14 | MES | 6 | 35.9 |
| 15 | Bis-Tris | 6 | 2.5 |
| 16 | Bis-Tris | 6 | 11.9 |
| 17 | Bis-Tris | 6 | 22.5 |
| 18 | Phosphate | 7 | 3.0 |
| 19 | Phosphate | 7 | 10.2 |
| 20 | Phosphate | 7 | 20.4 |
| 21 | Phosphate | 7 | 36.2 |
| 22 | Phosphate | 7 | 101.4 |
| 23 | Tris | 8 | 2.1 |
| 24 | Tris | 8 | 9.6 |

(continued)

| IgG-containing solutions used for examples | | | |
|---|---|---|---|
| IgG Solution Number | Buffer (20 mM) | pH | Conductivity (mS/cm) |
| 25 | Tris | 8 | 21.1 |
| 26 | Tris | 8 | 35.5 |
| 27 | Tris | 8 | 97.5 |

Monomer Preparation:

Monomer Example A.

4-[[[2-Methyl-2-(prop-2-enoylamino)propanoyl]amino]methyl]cyclohexanecarboxylic acid, sodium salt

(VDM-4-aminomethyl-cyclohexanecarboxylic acid, sodium salt)

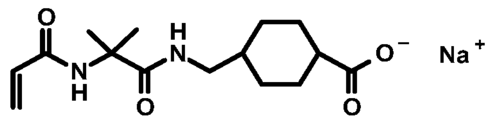

*Trans*-4-Aminomethyl-cyclohexanecarboxylic acid (2.00 g, 0.013 mol) was added to a 100 mL round bottom flask. An aqueous solution of sodium hydroxide (1.0 N, 13 mL) was added to the flask and the resulting mixture was stirred until the solids dissolved. The flask was then placed in an ice-water bath and stirred for 15 minutes. 2-Vinyl-4,4-dimethylazlactone (VDM) (1.77 g, 1.7 mL, 0.013 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 4-[[[2-methyl-2-(prop-2-enoylamino)propanoyl]amino]methyl]cyclohexanecarboxylic acid, sodium salt. $^1$H-NMR ($D_2O$, 500 MHz) δ 0.89 (q, 2H), 1.27 (q, 2H), 1.42 (s, 6H), 1.43 (m, 1H), 1.69 (d, 2H), 1.82 (d, 2H), 2.03 (m, 1H), 2.98 (d, 2H), 5.72 (m, 1H), 6.14 (m, 1H), 6.23 (m, 1H).

Monomer Example B.

2-Hydroxy-4-[[2-methyl-2-(prop-2-enoylamino)propanoyl]amino]butanoic acid, sodium salt

(VDM-2-hydroxy-4-aminobutanoic acid, sodium salt)

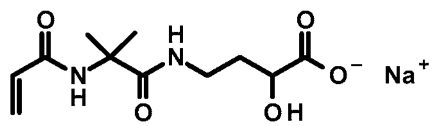

2-Hydroxy-4-aminobutanoic acid (5.00 g, 0.042 mol) was added to a 100 mL round bottom flask. An aqueous solution of sodium hydroxide solution (1.0 N, 42 mL) was added to the flask and the resulting mixture was stirred until the solids dissolved. The flask was then placed in an ice-water bath and stirred for 15 minutes. VDM (5.84 g, 5.6 mL, 0.042 mol) was added by syringe and the reaction was stirred for 30 minutes with the flask continuously maintained in the ice-water bath. The cooling bath was then removed and the reaction was allowed to warm to room temperature over a period of 30 minutes. $^1$H-NMR of an aliquot confirmed the formation of 2-hydroxy-4-[[2-methyl-2-(prop-2-enoylamino)propanoyl]amino]butanoic acid, sodium salt. The pH of the reaction was adjusted to about 7 by the addition of a few drops of a concentrated hydrochloric acid solution. $^1$H-NMR ($D_2O$, 500 MHz) δ 1.43 (s, 6H), 1.71 (m, 1H), 1.89 (m, 1H), 3.22 (m, 1H), 3.29 (m, 1H), 3.99 (m, 2H), 5.73 (m, 1H), 6.15 (m, 1H), 6.23 (m, 1H).

Monomer Example C.

3-Hydroxy-2-[[2-methyl-2-(prop-2-enoylamino)propanoyl]amino]propanoic acid, sodium salt

(VDM-serine, sodium salt)

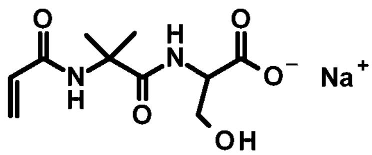

L-Serine (5.00 g, 0.048 mol) was added to a 100 mL round bottom flask. An aqueous solution of sodium hydroxide (1.0 N, 48 mL) was added to the flask and the resulting mixture was stirred until the solids dissolved. The flask was then placed in an ice-water bath and stirred for 15 minutes. VDM (6.62 g, 6.4 mL, 0.048 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 3-hydroxy-2-[[2-methyl-2-(prop-2-enoylamino)propanoyl]amino]propanoic acid, sodium salt. $^1$H-NMR ($D_2O$, 500 MHz) $\delta$ 1.48 (s, 6H), 3.79 (m, 2H), 4.22 (m, 1H), 5.74 (m, 1H), 6.16 (m, 1H), 6.25 (m, 1H).

Monomer Example D.

3-(4-Hydroxyphenyl)-2-[[2-methyl-2-(prop-2-enoylamino)propanoyl]amino]propanoic acid, sodium salt

(VDM-tyrosine, sodium salt)

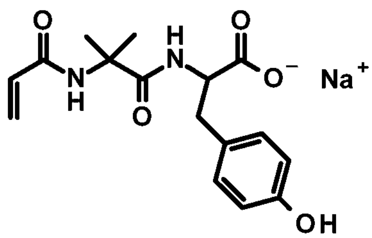

DL-Tyrosine (5.00 g, 0.028 mol) was added to a 250 mL round bottom flask. An aqueous solution of sodium hydroxide (0.5 N, 150 mL) was added to the flask and the resulting mixture was stirred until the solids dissolved. The flask was then placed in an ice-water bath and stirred for 15 minutes. VDM (3.84 g, 3.7 mL, 0.028 mol) was added by syringe and the reaction was stirred for 2 hours with the flask continuously maintained in the ice-water bath. The cooling bath was then removed and the reaction was allowed to warm to room temperature over a period of 30minutes. $^1$H-NMR of an aliquot confirmed the formation of 3-(4-hydroxyphenyl)-2-[[2-methyl-2-(prop-2-enoylamino)propanoyl]amino]propanoic acid, sodium salt. $^1$H-NMR ($D_2O$, 500 MHz) $\delta$ 1.39 (s, 6H), 2.70 (m, 1H), 2.84 (m, 1H), 3.42 (m, 1H), 5.67 (m, 1H), 6.11 (m, 1H), 6.22 (m, 1H), 6.58 (m, 2H), 6.95 (m, 2H).

Monomer Example E.

3-(1H-Indol-3-yl)-2-[[2-methyl-2-(prop-2-enoylamino)propanoyl]amino]propanoic acid, sodium salt

(VDM-tryptophan, sodium salt)

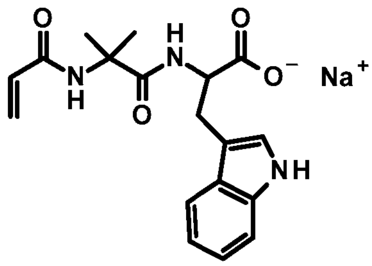

DL-Tryptophan (5.00 g, 0.025 mol) was added to a 250 mL round bottom flask. An aqueous solution of sodium hydroxide solution (0.5 N, 100 mL) was added to the flask and the resulting mixture was stirred until the solids dissolved. The flask was then placed in an ice-water bath and stirred for 15 minutes. VDM (3.40 g, 3.5 mL, 0.025 mol) was added by syringe and the reaction was stirred for 3 hours with the flask continuously maintained in the ice-water bath. The cooling bath was then removed and the reaction was allowed to warm to room temperature over a period of 30 minutes. $^1$H-NMR of an aliquot confirmed the formation of 3-(1H-indol-3-yl)-2-[[2-methyl-2-(prop-2-enoylamino)propanoyl]amino]propanoic acid, sodium salt. $^1$H-NMR ($D_2O$, 500 MHz) $\delta$ 1.39 (s, 6H), 3.01 (m, 1H), 3.13 (m, 1H), 3.54 (m, 1H), 5.67 (m, 1H), 6.11 (m, 1H), 6.21 (m, 1H), 7.12 (m, 1H), 7.19 (m, 2H), 7.46 (m, 1H), 7.70 (m, 1H).

Monomer Example F.

7-[[2-Methyl-2-(prop-2-enoylamino)propanoyl]amino]heptanoic acid, sodium salt

(VDM-7-aminoheptanoic acid, sodium salt)

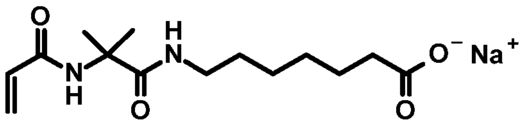

7-Aminoheptanoic acid (5.00 g, 0.035 mol) was added to a 100 mL round bottom flask. An aqueous solution of sodium hydroxide (1.0 N, 35 mL) was added to the flask and the resulting mixture was stirred until the solids dissolved. The flask was then placed in an ice-water bath and stirred for 15 minutes. VDM (4.79 g, 4.6 mL, 0.035 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 7-[[2-methyl-2-(prop-2-enoylamino)propanoyl]amino]heptanoic acid, sodium salt. $^1$H-NMR ($D_2O$, 500 MHz) $\delta$ 1.24 (s, 6H), 1.41 (m, 8H), 1.49 (m, 2H), 2.11 (m, 2H), 3.11 (m, 2H), 5.72 (m, 1H), 6.15 (m, 1H), 6.22 (m, 1H).

Monomer Example G.

3-(1H-Imidazol-5-yl)-2-[[2-methyl-2-(prop-2-enoylamino)propanoyl]amino]propanoic acid, sodium salt

(VDM-histidine, sodium salt)

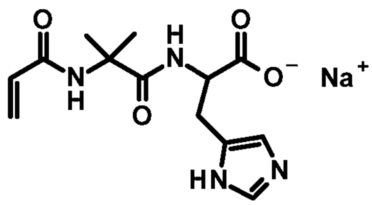

L-Histidine (2.00 g, 0.013 mol) was added to a 100 mL round bottom flask. An aqueous solution of sodium hydroxide 1.0 N, 13 mL) was added to the flask and the resulting mixture was stirred until the solids dissolved. The flask was then placed in an ice-water bath and stirred for 15 minutes. VDM (1.79 g, 1.72 mL, 0.013 mol) was added by syringe and the reaction was stirred for 30 minutes with the flask continuously maintained in the ice-water bath. The cooling bath was then removed and the reaction was allowed to warm to room temperature over a period of 30 minutes. $^1$H-NMR of an aliquot confirmed the formation of 3-(1H-imidazol-5-yl)-2-[[2-methyl-2-(prop-2-enoylamino)propanoyl]amino]propanoic acid, sodium salt. The pH of the reaction was adjusted to about 7 by the addition of a few drops of a concentrated hydrochloric acid solution. $^1$H-NMR ($D_2O$, 500 MHz) 1.30 (d, 6H), 2.87 (dd, 1H), 3.04 (dd, 1H), 4.24 (q, 1H), 5.61 (d, 1H), 6.01 (d, 1H), 6.12 (dd, 1H), 6.90 (s, 1H), 7.92 (s, 1H).

Monomer Example H.

3-[2-(2-Methylprop-2-enoyloxy)ethylcarbamoylamino]propanoic acid, sodium salt

(IEM-3-aminopropanoic acid, sodium salt)

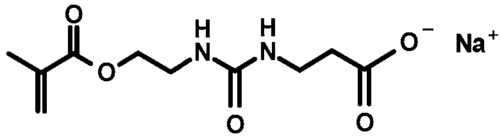

3-Aminopropanoic acid (1.78 g, 0.02 mol) was added to a 100 mL round bottom flask. An aqueous solution of sodium hydroxide solution (1.0 N, 20 mL) was added to the flask and the resulting mixture was stirred until the solids dissolved. The flask was then placed in an ice-water bath and stirred for 15 minutes. 2-Isocyanatoethyl methacrylate (IEM) (3.1 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. A colorless precipitate was filtered from the reaction mixture. The pH of the filtrate was adjusted to about 7 by the addition of a few drops of a concentrated hydrochloric acid solution. $^1$H-NMR of an aliquot of the filtrate confirmed the formation of 3-[2-(2-methylprop-2-enoyloxy)ethylcarbamoylamino]propanoic acid, sodium salt. $^1$H-NMR ($D_2O$, 500 MHz) $\delta$ 1.78 (s, 3H), 2.22 (t, 2H), 3.16 (t, 2H), 3.30 (t, 2H), 4.07 (t, 2H), 5.58 (s, 1H), 5.99 (s, 1H).

Monomer Example I.

2-[2-(2-Methylprop-2-enoyloxy)ethylcarbamoylamino]ethanesulfonic acid, sodium salt

(IEM-taurine, sodium salt)

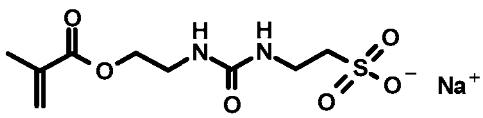

Taurine (2.50 g, 0.02 mol) was added to a 100 mL round bottom flask. An aqueous solution of sodium hydroxide solution (1.0 N, 20 mL) was added to the flask and the resulting mixture was stirred until the solids dissolved. The flask was then placed in an ice-water bath and stirred for 15 minutes. IEM (3.1 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. A colorless precipitate was filtered from the reaction mixture. The pH of the filtrate was adjusted to about 7 by the addition of a few drops of a concentrated hydrochloric acid solution. $^1$H-NMR of an aliquot of the filtrate confirmed the formation of 2-[2-(2-methylprop-2-enoyloxy)ethylcarbamoylamino]ethanesulfonic acid, sodium salt. $^1$H-NMR ($D_2O$, 500 MHz) $\delta$ 1.75 (s, 3H), 2.88 (t, 2H), 3.28 (t, 2H) 3.32 (t, 2H), 4.06 (t, 2H), 5.56 (m, 1H), 5.97 (s, 1H).

Monomer Example J.

2-[[2-Methyl-2-(prop-2-enoylamino)propanoyl]amino]ethanesulfonic acid, sodium salt

(VDM-taurine, sodium salt)

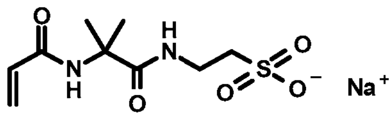

Taurine (2.50 g, 0.02 mol) was added to a 100 mL round bottom flask. An aqueous solution of sodium hydroxide solution (1.0 N, 20 mL) was added to the flask and the resulting mixture was stirred until the solids dissolved. The flask was then placed in an ice-water bath and stirred for 15 minutes. VDM (2.78 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 2-[[2-methyl-2-(prop-2-enoylamino)propanoyl]amino]ethanesulfonic acid, sodium salt. $^1$H-NMR ($D_2O$, 500 MHz) $\delta$ 1.35 (s, 6H), 2.94 (t, 2H), 3.45 (t, 2H) 5.64 (m, 1H), 6.10 (m, 2H).

Monomer Example K.

2-[2-Methyl-2-(prop-2-enoylamino)propanoyl]oxyethylphosphonic acid

(VDM-2-(hydroxyethyl)phosphonic acid)

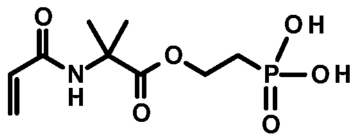

2-Hydroxyethyl dimethylphosphate (90% purity, 15.0 g, 97.3 mmol) and 1,8-diazabicyclo[5.4.0]undec-7-ene (1 mL) were added to a 250 mL flask containing a solution of VDM (13.7 g, 97.3 mmol) dissolved in 100 mL of anhydrous methylene chloride and the reaction was stirred overnight. The reaction mixture was diluted with additional methylene chloride and washed sequentially with saturated 5% $NaH_2PO_4$, $H_2O$, and brine. The organic phase was dried with $Na_2SO_4$ and then filtered. A small amount (approximately 130 mg) of BHT was added and the solution was concentrated under reduced pressure to give 11.2 g of a colorless liquid. The liquid was dissolved in 50 mL of methylene chloride and placed in ice-water bath under a nitrogen atmosphere. Bromotrimethylsilane (12.3 g, 80.2 mmol) was added and the reaction was stirred for 90 minutes at room temperature. The solution was concentrated under reduced pressure. Methanol (50 mL) was added to the resulting residue and the solution was stirred at room temperature for 1 hour. The solution was then concentrated under reduced pressure at room temperature to yield the final product as clear liquid. $^1$H-NMR of an aliquot confirmed the formation of 2-[2-methyl-2-(prop-2-enoylamino)propanoyl]oxyethylphosphonic acid. $^1$H NMR ($D_2O$, 500 MHz) $\delta$ 1.44 (s, 6H), 2.14 (dt, 2H), 4.29 (dt, 2H), 5.69 (dd, 1H), 6.12 (dd, 1H), 6.19 (broad s, 1H).

Monomer Example L.

3-[[2-Methyl-2-(prop-2-enoylamino)propanoyl]amino]propanoic acid, sodium salt

(VDM-3-aminopropanoic acid, sodium salt)

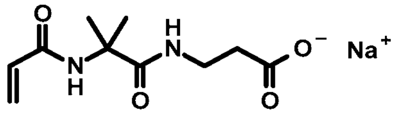

3-Aminopropanoic acid (1.78 g, 0.02 mol) was added to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. VDM (2.78 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 3-[[2-methyl-2-(prop-2-enoylamino)propanoyl]amino]propanoic acid, sodium salt. $^1$H-NMR ($D_2O$, 500 MHz) $\delta$ 1.32 (s, 6H), 2.23 (t, 2H), 3.24 (t, 2H), 5.63 (m, 1H), 6.0-6.2 (m, 2H).

Monomer Example M.

4-[[2-Methyl-2-(prop-2-enoylamino)propanoyl]amino]butanoic acid, sodium salt

(VDM-GABA, sodium salt)

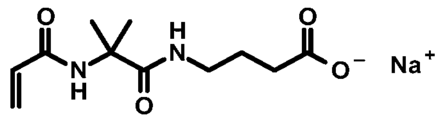

4-Aminobutanoic acid (2.06 g, 0.02 mol) was added to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. VDM (2.78 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 4-[[2-methyl-2-(prop-2-enoylamino)propanoyl]amino]butanoic acid, sodium salt. $^1$H-NMR ($D_2O$, 500 MHz) $\delta$ 1.34 (s, 6H), 1.59 (p, 2H), 2.04 (t, 2H), 3.05 (t, 2H), 5.62 (d, 1H), 6.0-6.2 (m, 2H).

Monomer Example N.

5-[[2-Methyl-2-(prop-2-enoylamino)propanoyl]amino]pentanoic acid, sodium salt

(VDM-5-aminovaleric acid, sodium salt)

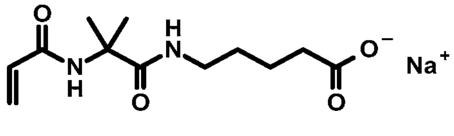

5-Aminopentanoic acid (2.34 g, 0.02 mol) was added to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. VDM (2.78 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 5-[[2-methyl-2-(prop-2-enoylamino)propanoyl]amino]pentanoic acid, sodium salt. $^1$H-NMR ($D_2O$, 500 MHz) $\delta$ 1.33 (s and m, 10H), 2.04 (t, 2H), 3.05 (t, 2H), 5.63 (d, 1H), 6.0-6.2 (m, 2H).

Monomer Example O.

6-[[2-Methyl-2-(prop-2-enoylamino)propanoyl]amino]hexanoic acid, sodium salt

(VDM-6-aminocaproic acid, sodium salt)

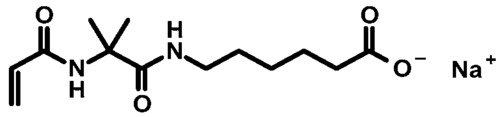

6-Aminohexanoic acid (2.62 g, 0.02 mol) was added to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. VDM (2.78 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 6-[[2-methyl-2-(prop-2-enoylamino)propanoyl]amino]hexanoic acid, sodium salt. $^1$H-NMR ($D_2O$, 500 MHz) $\delta$ 1.14 (m, 2H), 1.34 (s and m, 8H), 1.41 (m, 2H), 2.02 (t, 2H), 3.03 (t, 2H), 5.62 (d, 1H), 6.0-6.2 (m, 2H).

Monomer Example P.

2-[[2-Methyl-2-(prop-2-enoylamino)propanoyl]amino]-3-phenylpropanoic acid, sodium salt

(VDM-phenylalanine, sodium salt)

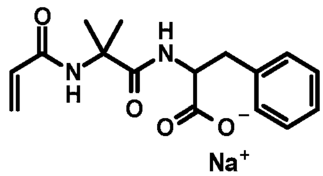

L-Phenylalanine (3.3 g, 0.02 mol) was added to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. VDM (2.78 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. The pH of the reaction was adjusted to about 7 by the addition of a few drops of a concentrated hydrochloric acid solution. $^1$H-NMR of an aliquot confirmed the formation of 2-[[2-methyl-2-(prop-2-enoylamino)propanoyl]amino]-3-phenylpropanoic acid, sodium salt. $^1$H-NMR ($D_2O$, 500 MHz) $\delta$ 1.26 (s, 6H), 2.89 (m, 1H), 2.95 (m, 1H), 4.30 (m, 1H), 5.62 (d, 1H), 6.00-6.10 (m, 2H), 7.07-7.20 (m, 5H).

Monomer Example Q.

2-[2-(2-Methylprop-2-enoyloxy)ethylcarbamoylamino]-3-phenylpropanoic acid, sodium salt

(IEM-phenylalanine, sodium salt)

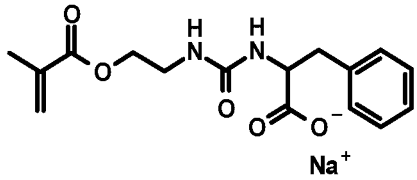

L-Phenylalanine (3.3 g, 0.02 mol) was charged to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. IEM (3.1 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. A colorless precipitate was filtered from the reaction mixture. The pH of the filtrate was adjusted to about 7 by the addition of a few drops of a concentrated hydrochloric acid solution. $^1$H-NMR of an aliquot of the filtrate confirmed the formation of 2-[2-(2-methylprop-2-enoyloxy)ethylcarbamoylamino]-3-phenylpropanoic acid, sodium salt. $^1$H-NMR ($D_2O$, 500 MHz) δ 1.74 (br. s, 3H), 2.73 (m, 1H), 2.99 (m, 1H), 3.13 (m, 1H), 3.26 (m 1H), 3.90 (m, 2H), 4.17 (m, 1H), 5.54 (m, 1H), 5.95 (m, 1H), 7.09 and 7.15 (m, 5H).

Monomer Example R.

2-[2-(2-Methylprop-2-enoyloxy)ethylcarbamoylamino]ethanoic acid, sodium salt

(IEM-glycine, sodium salt)

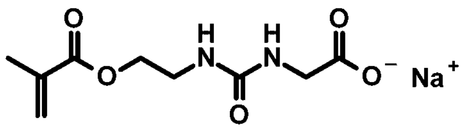

Glycine (1.5 g, 0.02 mol) was charged to a 100 mL round bottom flask. An aqueous solution of sodium hydroxide (1.0 N, 20 mL) was added to the flask and the resulting mixture was stirred until the solids dissolved. The flask was then placed in an ice-water bath and stirred for 15 minutes. IEM (3.1 g, 0.02 mol) was added by syringe and the reaction was stirred for 30 minutes with the flask continuously maintained in the ice-water bath. The cooling bath was then removed and the reaction was allowed to warm to room temperature over a period of 30 minutes. A colorless precipitate was filtered from the reaction mixture. The pH of the filtrate was adjusted to about 7 by the addition of a few drops of a concentrated hydrochloric acid solution. $^1$H-NMR of an aliquot of the filtrate confirmed the formation of 2-[2-(2-methylprop-2-enoyloxy)ethylcarbamoylamino]ethanoic acid, sodium salt. $^1$H-NMR ($D_2O$, 500 MHz) δ 1.79 (s, 3H), 3.33 (m, 2H), 3.54 (s, 2H), 4.09 (m, 2 H), 5.59 (s, 1H), 5.99 (s, 1H).

Example 1.

A coating solution was prepared by mixing a solution of 4-[[[2-methyl-2-(prop-2-enoylamino)propanoyl]amino]methyl]cyclohexanecarboxylic acid, sodium salt (Monomer Example A) (3.34 grams of a 23.85% w/w solution in deionized water, 2.5 mmol) with the initiator 4-(3-sulfopropyloxy)benzophenone, sodium salt (S-BP) (500 μL of a 0.05 g/mL solution in deionized water). This mixture was diluted to a total of 5 grams with deionized water to produce a final monomer concentration of 0.5M and initiator concentration of 0.5%. A nylon membrane substrate (9 cm x 12 cm; nylon 66 membrane, single reinforced layer nylon three-zone membrane, nominal pore size 1.8 μm, #080ZN, obtained from 3M Purification, Inc., Meridan, CT) was placed on a sheet of clear polyester film (about 0.25 mm thick), and the coating solution was pipetted onto the top surface of the substrate. The coating solution was allowed to soak into the substrate for about 1 minute, and then a second sheet of clear polyester film (about 0.25 mm thick) was placed over the top surface of the substrate. A 2.28 kg cylindrical weight was rolled over the top of the resulting three-layer sandwich (polyester film-membrane substrate-polyester film) to squeeze out excess coating solution. Ultraviolet (UV)-initiated grafting was conducted by irradiating the sandwich using a UV stand (Classic Manufacturing, Inc., Oakdale, MN) equipped with 18 bulbs (Sylvania RG2 40W F40/350BL/ECO, 10 above and 8 below the substrate, 1.17 meters (46 inches) long, spaced 5.1 cm (2 inches) on center), with an irradiation time of 15 minutes. The polyester sheets were removed, and the remaining

functionalized substrate was placed in a 250 mL polyethylene bottle. The bottle was filled with 0.9 percent (wt %) saline, sealed, and shaken for 30 minutes to wash off any residual monomer or ungrafted polymer. The saline was decanted, and the functionalized substrate was washed for another 30 minutes with fresh saline solution and then washed for 30 minutes with deionized water and allowed to dry. The finished filter element had a graft density of 0.20 mmol/gram. Graft density was determined by the measuring the mass gain after conversion of the substrate to the finished filter element.

Example 2.

A coating solution was prepared by mixing a solution of 2-hydroxy-4-[[2-methyl-2-(prop-2-enoylamino)propanoyl]amino]butanoic acid, sodium salt (Monomer Example B) (2.98 grams of a 23.55% w/w solution in deionized water, 2.5 mmol) with S-BP (500 μL of a 0.05 g/mL solution in deionized water). This mixture was diluted to a total of 5 grams with deionized water to produce a final monomer concentration of 0.5M and initiator concentration of 0.5%. The procedure described in Example 1 was followed to provide a finished filter element with a graft density of 0.07 mmol/gram.

Example 3.

A coating solution was prepared by mixing a solution of 3-hydroxy-2-[[2-methyl-2-(prop-2-enoylamino)propanoyl]amino]propanoic acid, sodium salt (Monomer Example C) (2.79 grams of a 23.85% w/w solution in deionized water, 2.5 mmol) with S-BP (500 μL of a 0.05 g/mL solution in deionized water). This mixture was diluted to a total of 5 grams with deionized water to produce a final monomer concentration of 0.5M and initiator concentration of 0.5%. The procedure described in Example 1 was followed to provide a finished filter element with a graft density of 0.23 mmol/gram.

Example 4.

A coating solution was prepared by mixing a solution of 3-(1H-imidazol-5-yl)-2-[[2-methyl-2-(prop-2-enoylamino)propanoyl]amino]propanoic acid, sodium salt (Monomer Example G) (2.87 grams of a 27.55% w/w solution in deionized water, 2.5 mmol) with S-BP (500 μL of a 0.05 g/mL solution in deionized water). This mixture was diluted to a total of 5 grams with deionized water to produce a final monomer concentration of 0.5M and initiator concentration of 0.5%. The procedure described in Example 1 was followed to provide a finished filter element with a graft density of 0.19 mmol/gram.

Example 5.

A coating solution was prepared by mixing a solution of 4-[[[2-methyl-2-(prop-2-enoylamino)propanoyl]amino]methyl]cyclohexanecarboxylic acid, sodium salt (Monomer Example A) (1.67 grams of a 23.85% w/w solution in deionized water, 1.25 mmol) with S-BP (500 μL of a 0.05 g/mL solution in deionized water). This mixture was diluted to a total of 5 grams with deionized water to produce a final monomer concentration of 0.25M and initiator concentration of 0.5%. The procedure described in Example 1 was followed to provide a finished filter element with a graft density of 0.12 mmol/gram.

Example 6.

A coating solution was prepared by mixing a solution of 2-hydroxy-4-[[2-methyl-2-(prop-2-enoylamino)propanoyl]amino]butanoic acid, sodium salt (Monomer Example B) (1.48 grams of a 23.55% w/w solution in deionized water, 1.25 mmol) with (S-BP (500 μL of a 0.05 g/mL solution in deionized water). This mixture was diluted to a total of 5 grams with deionized water to produce a final monomer concentration of 0.25M and initiator concentration of 0.5%. The procedure described in Example 1 was followed to provide a finished filter element with a graft density of 0.04 mmol/gram.

Example 7.

A coating solution was prepared by mixing a solution of 3-hydroxy-2-[[2-methyl-2-(prop-2-enoylamino)propanoyl]amino]propanoic acid, sodium salt (Monomer Example C) (1.39 grams of a 23.85% w/w solution in deionized water, 1.25 mmol) with S-BP (500 μL of a 0.05 g/mL solution in deionized water). This mixture was diluted to a total of 5 grams with deionized water to produce a final monomer concentration of 0.25M and initiator concentration of 0.5%. The procedure described in Example 1 was followed to provide a finished filter element with a graft density of 0.11 mmol/gram.

Example 8.

A coating solution was prepared by mixing a solution of 3-(1H-imidazol-5-yl)-2-[[2-methyl-2-(prop-2-enoylamino)propanoyl]amino]propanoic acid, sodium salt (Monomer Example G) (1.45 grams of a 27.55% w/w solution in deionized water, 1.25 mmol) with S-BP (500 μL of a 0.05 g/mL solution in deionized water). This mixture was diluted to a total of 5 grams with deionized water to produce a final monomer concentration of 0.25M and initiator concentration of 0.5%. The procedure described in Example 1 was followed to provide a finished filter element with a graft density of 0.08 mmol/gram.

Example 9.

A coating solution was prepared by mixing a solution of 2-hydroxy-4-[[2-methyl-2-(prop-2-enoylamino)propanoyl]amino]butanoic acid, sodium salt (Monomer Example B) (2.98 grams of a 23.55% w/w solution in deionized water, 2.5 mmol) with S-BP (100 μL of a 0.05 g/mL solution in deionized water). This mixture was diluted to a total of 5 grams with deionized water to produce a final monomer concentration of 0.5M and initiator concentration of 0.1%. The procedure described in Example 1 was followed to provide a finished filter element with a graft density of 0.10 mmol/gram.

Example 10.

A coating solution was prepared by mixing a solution of 4-[[[2-methyl-2-(prop-2-enoylamino)propanoyl]amino]methyl]cyclohexanecarboxylic acid, sodium salt (Monomer Example A) (1.67 grams of a 23.85% w/w solution in deionized water, 1.25 mmol) with S-BP (100 μL of a 0.05 g/mL solution in deionized water). This mixture was diluted to a total of 5 grams with deionized water to produce a final monomer concentration of 0.25M and initiator concentration of 0.1%. The procedure described in Example 1 was followed to provide a finished filter element with a graft density of 0.07 mmol/gram.

Example 11.

A coating solution was prepared by mixing a solution of 3-hydroxy-2-[[2-methyl-2-(prop-2-enoylamino)propanoyl]amino]propanoic acid, sodium salt (Monomer Example C) (1.39 grams of a 23.85% w/w solution in deionized water, 1.25 mmol) with S-BP (100 μL of a 0.05 g/mL solution in deionized water). This mixture was diluted to a total of 5 grams with deionized water to produce a final monomer concentration of 0.25M and initiator concentration of 0.1%. The procedure described in Example 1 was followed to provide a finished filter element with a graft density of 0.07 mmol/gram.

Example 12.

A coating solution was prepared by mixing a solution of 3-(4-hydroxyphenyl)-2-[[2-methyl-2-(prop-2-enoylamino)propanoyl]amino]propanoic acid, sodium salt (Monomer Example D) (1.25 mL of a 1M solution in deionized water) with N,N-Dimethylacrylamide (1.25 mL of a 1M solution), and S-BP (500 μL of a 0.05 g/mL solution in deionized water). This mixture was diluted to a total of 5 grams with deionized water to produce a final monomer concentration of 0.25M and initiator concentration of 0.5%. The procedure described in Example 1 was followed to provide a finished filter element with a graft density of 0.21 mmol/gram.

Example 13.

A coating solution was prepared by mixing a solution of 3-(1H-indol-3-yl)-2-[[2-methyl-2-(prop-2-enoylamino)propanoyl]amino]propanoic acid, sodium salt (monomer Example E) (1.25 mL of a 1M solution in deionized water) with S-BP (500 μL of a 0.05 g/mL solution in deionized water). This mixture was diluted to a total of 5 grams with deionized water to produce a final monomer concentration of 0.25M and initiator concentration of 0.5%. The procedure described in Example 1 was followed to provide a finished filter element with a graft density of 0.16 mmol/gram.

Example 14.

A coating solution was prepared by mixing a solution of 2-[[2-methyl-2-(prop-2-enoylamino)propanoyl]amino]ethanesulfonic acid, sodium salt (Monomer Example J) (2.5 mL of a 1M solution in deionized water) with S-BP (500 μL of a 0.05 g/mL solution in deionized water). This mixture was diluted to a total of 5 grams with deionized water to produce a final monomer concentration of 0.5M and initiator concentration of 0.5%. The procedure described in Example

1 was followed to provide a finished filter element with a graft density of 0.15 mmol/gram.

Example 15.

A coating solution was prepared by mixing a solution of 2-[2-methyl-2-(prop-2-enoylamino)propanoyl]oxyethylphosphonic acid, sodium salt (Monomer Example K) (2.5 mL of a 1M solution in deionized water) with S-BP (500 μL of a 0.05 g/mL solution in deionized water). This mixture was diluted to a total of 5 grams with deionized water to produce a final monomer concentration of 0.5M and initiator concentration of 0.5%. The procedure described in Example 1 was followed to provide a finished filter element with a graft density of 0.20 mmol/gram.

Example 16.

A coating solution was prepared by mixing a solution of 3-[2-(2-methylprop-2-enoyloxy)ethylcarbamoylamino]propanoic acid, sodium salt (monomer Example H) (3.75 mL of a 1M solution in deionized water) with S-BP (500 μL of a 0.05 g/mL solution in deionized water). This mixture was diluted to a total of 5 grams with deionized water to produce a final monomer concentration of 0.75M and initiator concentration of 0.5%. The procedure described in Example 1 was followed to provide a finished filter element with a graft density of 0.50 mmol/gram.

Example 17.

A coating solution was prepared by mixing a solution of 3-[[2-methyl-2-(prop-2-enoylamino)propanoyl]amino]propanoic acid, sodium salt (Monomer Example L) (3.75 mL of a 1M solution in deionized water) with S-BP (500 μL of a 0.05 g/mL solution in deionized water). This mixture was diluted to a total of 5 grams with deionized water to produce a final monomer concentration of 0.75M and initiator concentration of 0.5%. The procedure described in Example 1 was followed to provide a finished filter element with a graft density of 0.623 mmol/gram.

Example 18.

A coating solution was prepared by mixing a solution of 4-[[2-methyl-2-(prop-2-enoylamino)propanoyl]amino]butanoic acid, sodium salt (Monomer Example M) (1.53 grams of a 21.65% w/w solution in deionized water, 1.25 mmol) with S-BP (125 μL of a 0.05 g/mL solution in deionized water). This mixture was diluted to a total of 5 grams with deionized water to produce a final monomer concentration of 0.25M and initiator concentration of 0.125%. The procedure described in Example 1 was followed to provide a finished filter element with a graft density of 0.23 mmol/gram.

Example 19.

A coating solution was prepared by mixing a solution of 5-[[2-methyl-2-(prop-2-enoylamino)propanoyl]amino]pentanoic acid, sodium salt (Monomer Example N) (1.58 grams of a 22.05% w/w solution in deionized water, 1.25 mmol) with S-BP (62.5 μL of a 0.05 g/mL solution in deionized water). This mixture was diluted to a total of 5 grams with deionized water to produce a final monomer concentration of 0.25M and initiator concentration of 0.0625%. The procedure described in Example 1 was followed to provide a finished filter element with a graft density of 0.19 mmol/gram.

Example 20.

A coating solution was prepared by mixing a solution of 6-[[2-methyl-2-(prop-2-enoylamino)propanoyl]amino]hexanoic acid, sodium salt (Monomer Example O) (1.54 grams of a 23.7% w/w solution in deionized water, 1.25 mmol) with S-BP (31.2 μL of a 0.05 g/mL solution in deionized water). This mixture was diluted to a total of 5 grams with deionized water to produce a final monomer concentration of 0.25M and initiator concentration of 0.0312%. The procedure described in Example 1 was followed to provide a finished filter element with a graft density of 0.20 mmol/gram.

Example 21.

A coating solution was prepared by mixing a solution of 7-[[2-methyl-2-(prop-2-enoylamino)propanoyl]amino]heptanoic acid, sodium salt (Monomer Example F) (1.56 grams of a 24.5% w/w solution in deionized water, 1.25 mmol) with S-BP (62.5 μL of a 0.05 g/mL solution in deionized water). This mixture was diluted to a total of 5 grams with deionized water to produce a final monomer concentration of 0.25M and initiator concentration of 0.0625%. The procedure described in Example 1 was followed to provide a finished filter element with a graft density of 0.18 mmol/gram.

Example 22.

A coating solution was prepared by mixing a solution of 7-[[2-methyl-2-(prop-2-enoylamino)propanoyl]amino]heptanoic acid, sodium salt (Monomer Example F) (1.56 grams of a 24.5% w/w solution in deionized water, 1.25 mmol) with S-BP (125 μL of a 0.05 g/mL solution in deionized water). This mixture was diluted to a total of 5 grams with deionized water to produce a final monomer concentration of 0.25M and initiator concentration of 0.125%. The procedure described in Example 1 was followed to provide a finished filter element with a graft density of 0.24 mmol/gram.

Example 23.

A coating solution was prepared by mixing a solution of 2-[[2-methyl-2-(prop-2-enoylamino)propanoyl]amino]-3-phenylpropanoic acid, sodium salt (monomer Example P) (1.65 grams of a 24.8% w/w solution in deionized water, 1.25 mmol) with S-BP (31.2 μL of a 0.05 g/mL solution in deionized water). This mixture was diluted to a total of 5 grams with deionized water to produce a final monomer concentration of 0.25M and initiator concentration of 0.0312%. The procedure described in Example 1 was followed to provide a finished filter element with a graft density of 0.18 mmol/gram.

Example 24.

A coating solution was prepared by mixing a solution of 2-[2-(2-methylprop-2-enoyloxy)ethylcarbamoylamino]-3-phenylpropanoic acid, sodium salt (Monomer Example Q) (1.71 grams of a 25% w/w solution in deionized water, 1.25 mmol) with S-BP (31.2 μL of a 0.05 g/mL solution in deionized water). This mixture was diluted to a total of 5 grams with deionized water to produce a final monomer concentration of 0.25M and initiator concentration of 0.0312%. The procedure described in Example 1 was followed to provide a finished filter element with a graft density of 0.42 mmol/gram.

Example 25.

A coating solution was prepared by mixing a solution of 4-[[2-methyl-2-(prop-2-enoylamino)propanoyl]amino]butanoic acid, sodium salt (monomer Example M) (3.75 mL of a 0.25M solution in deionized water) with N,N-dimethylacrylamide (1.25 mL of a 0.25 M solution), and S-BP (31.25 μL of a 0.05 g/mL solution in deionized water). The procedure described in Example 1 was followed to provide a finished filter element with a graft density of 0.14 mmol/gram.

Example 26.

A coating solution was prepared by mixing a solution of 4-[[2-methyl-2-(prop-2-enoylamino)propanoyl]amino]butanoic acid, sodium salt (monomer Example M) (2.5 mL of a 0.25M solution in deionized water) with N,N-dimethylacrylamide (2.5 mL of a 0.25 M solution), and S-BP (31.25 μL of a 0.05 g/mL solution in deionized water). The procedure described in Example 1 was followed to provide a finished filter element with a graft density of 0.23 mmol/gram.

Example 27.

A coating solution was prepared by mixing a solution of 4-[[2-methyl-2-(prop-2-enoylamino)propanoyl]amino]butanoic acid, sodium salt (monomer Example M) (1.25 mL of a 0.25M solution in deionized water) with N,N-dimethylacrylamide (3.75 mL of a 0.25M solution), and S-BP (31.25 μL of a 0.05 g/mL solution in deionized water). The procedure described in Example 1 was followed to provide a finished filter element with a graft density of 0.42 mmol/gram.

Example 28.

A coating solution was prepared by mixing a solution of 4-[[2-methyl-2-(prop-2-enoylamino)propanoyl]amino]butanoic acid, sodium salt (monomer Example M) (1.25 mL of a 0.5M solution in deionized water) with N,N-dimethylacrylamide (3.75 mL of a 0.5M solution), and S-BP (31.25 μL of a 0.05 g/mL solution in deionized water). The procedure described in Example 1 was followed to provide a finished filter element with a graft density of 0.43 mmol/gram.

Example 29.

A coating solution was prepared by mixing a solution of 2-[2-(2-methylprop-2-enoyloxy)ethylcarbamoylamino]ethanesulfonic acid, sodium salt (monomer Example I) (2.5 mL of a 0.75M solution in deionized water) with 3-phenyl-2-(prop-2-enoylamino)propanoic acid (2.5 mL of a 0.75M solution), and S-BP (500 μL of a 0.05 g/mL solution in deionized water). The procedure described in Example 1 was followed to provide a finished filter element.

Example 30.

A coating solution was prepared by mixing a solution of 2-[2-(2-methylprop-2-enoyloxy)ethylcarbamoylamino]ethanoic acid, sodium salt (monomer Example R) (1.62 grams of a 19.5% w/w solution in deionized water, 1.25 mmol) with S-BP (62.5 μL of a 0.05 g/mL solution in deionized water). This mixture was diluted to a total of 5 grams with deionized water to produce a final monomer concentration of 0.25M and initiator concentration of 0.0625%. The procedure described in Example 1 was followed to provide a finished filter element with a graft density of 0.25 mmol/gram.

Example 31. Process for Separating Aggregated IgG from Monomeric IgG

The finished filter elements of Examples 1-30 were cut into 7.5 mm diameter disks. Two disks were inserted into each well of a 96-well Empore™ Filter Plate (Model 6065, 3M Corporation, St. Paul, MN) in which the original solid phase extraction material had been previously removed. The filter element disks were held in place with a plastic O-ring. The total working filter volume of each well was about 8.6 μL. Each well was equilibrated with 1 mL of a non-IgG-containing buffer solution that had the same buffer composition as in the IgG-containing solution to be tested in the well. The equilibration solutions were removed from the wells using centrifugation (Allegra 25R centrifuge, Beckman Coulter, Brea, CA). The wells of the plate were then individually loaded (by pipet) with 1 mL of a single IgG-containing buffer solution selected from Table 2. The plate was sequentially centrifuged at 100, 200, 300, 600, 1200, and 3000 rcf (relative centrifugal force) or until the individual samples completely flowed through the filter elements. Each centrifugation step was conducted for about 5 minutes. The concentration of IgG in the IgG-containing buffer solution was either 3.32 mg/mL or 0.83 mg/mL, representing a challenge load of either 386 g/L or 96.5 g/L. Challenge load is defined as amount of IgG per given volume of filtration media (grams per liter, g/L). Separate collection plates were used for the equilibration solutions and IgG sample solutions. Filtered solutions were analyzed by size exclusion chromatography (SEC) using a Shimadzu Prominence HPLC (Shimadzu Scientific Instruments, Columbia, MD) with a TSKgel™ G3000SWxl column (Tosoh Bioscience LLC, Griesheim, Germany) (analysis conditions: injection volume of 20 μL; flow rate of 1 ml/min; mobile phase: 100 mM sodium phosphate, 300 mM NaCl, pH 6.9; detection at 280 nanometers).

In Tables 3-35 the results are presented for the process of Example 31 using filter elements from Examples 1-30 and the IgG solutions (Number 1-27) described in Table 2. Monomer yield and aggregate removal were determined by comparison of peak areas of the starting and filtered solutions and the results are reported as the average of two replicates.

The percent of monomeric IgG present in the IgG solution before performing the separation process of Example 31 (Initial IgG solution) was determined using the SEC chromatography method described above. The peak area of the monomeric component (Peak A) of the sample was compared to the sum of the sample peak areas (Total Peak Area) according to Equation 1.

The percent of aggregated IgG present in the IgG solution before performing the separation process of Example 31 (Initial IgG solution) was determined using the SEC chromatography method described above. The peak area of the aggregate component (Peak B) of the sample was compared to the sum of the sample peak areas (Total Peak Area) according to Equation 2.

The ratio of the percent of monomeric IgG to the percent of aggregated IgG in the initial IgG solution (i.e. prior to performing the separation process of Example 31) was calculated according to Equation 3 and is reported as the "Initial Composition Ratio".

The percent removal of aggregated IgG was determined using the SEC chromatography method described above and measuring the peak area for the aggregated component before performing the separation process (Peak B) and after performing the separation process of Example 31 (Peak C). The "% Aggregated IgG Removed" was calculated according to Equation 4.

The percent yield of monomeric IgG was determined using the SEC chromatography method described above and measuring the peak area for the monomeric component before performing the separation process (Peak A) and after performing the separation process of Example 31 (Peak D). The "% Monomeric IgG Yield" was calculated according to Equation 5.

The calculation for Monomeric IgG Recovered following the separation process of Example 31 was determined according to Equation 6 and is reported as "Final Monomeric IgG Recovered (%)".

The calculation for Aggregated IgG Recovered following the separation process of Example 31 was determined according to Equation 7 and is reported as "Final Aggregated IgG Recovered (%)".

The ratio of the percent of monomeric IgG to the percent of aggregated IgG in the final IgG solution (i.e. after performing the separation process of Example 31) was calculated according to Equation 8 and is reported as the "Final Composition Ratio".

The "Ratio of the Final Composition Ratio to the Initial Composition Ratio" was calculated according to Equation 9.

The calculated values for Equations 1-9 are reported in Tables 3-35.

Equation 1:

$$\text{Initial Monomeric IgG (\%)} = \left(\frac{\text{Peak Area A}}{\text{Total Peak Area}}\right) \text{x } 100$$

Equation 2:

$$\text{Initial Aggregated IgG (\%)} = \left(\frac{\text{Peak Area B}}{\text{Total Peak Area}}\right) \text{x } 100$$

Equation 3:

$$\text{Initial Composition Ratio} = \left(\frac{\text{Initial Monomeric IgG (\%)}}{\text{Initial Aggregated IgG (\%)}}\right)$$

Equation 4:

$$\text{Aggregated IgG Removed (\%)} = 100 - \left(\left(\frac{\text{Peak Area C}}{\text{Peak Area B}}\right) \text{x } 100\right)$$

Equation 5:

$$\text{Monomeric IgG Yield (\%)} = \left(\frac{\text{Peak Area D}}{\text{Peak Area A}}\right) \text{x } 100$$

Equation 6:

$$\text{Final Monomeric IgG Recovered (\%)} = \left(\frac{\text{Initial Monomeric IgG (\%) x Monomeric IgG Yield (\%)}}{100}\right)$$

Equation 7:

$$\text{Final Aggregated IgG Recovered (\%)} = \text{Initial Aggregated IgG (\%) x } \left(\frac{100 - \text{Aggregated IgG Removed(\%)}}{100}\right)$$

Equation 8:

$$\text{Final Composition Ratio} = \left(\frac{\text{Final Monomeric IgG Recovered(\%)}}{\text{Final Aggregated IgG Recovered (\%)}}\right)$$

Equation 9:

$$\text{Ratio of Final Composition Ratio to Initial Composition Ratio} = \left(\frac{\text{Final Composition Ratio}}{\text{Initial Composition Ratio}}\right)$$

TABLE 3. Filter Element of Example 1

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.6 | 3.4 | 28.4 | 41.7 | 91.7 | 88.6 | 2.0 | 44.3 | 1.56 |
| 2 | 96.6 | 3.4 | 28.4 | 44.0 | 92.3 | 89.2 | 1.9 | 46.9 | 1.65 |
| 3 | 96.5 | 3.5 | 27.6 | 33.7 | 93.6 | 90.3 | 2.3 | 39.3 | 1.42 |
| 4 | 96.6 | 3.4 | 28.4 | 35.0 | 94.5 | 91.3 | 2.2 | 41.5 | 1.46 |
| 6 | 96.5 | 3.5 | 27.6 | 67.9 | 80.0 | 77.2 | 1.1 | 70.2 | 2.54 |
| 7 | 96.5 | 3.5 | 27.6 | 85.7 | 81.3 | 78.5 | 0.5 | 157.0 | 5.69 |
| 8 | 96.5 | 3.5 | 27.6 | 59.4 | 93.7 | 90.4 | 1.4 | 64.6 | 2.34 |
| 9 | 96.5 | 3.5 | 27.6 | 20.6 | 98.1 | 94.7 | 2.8 | 33.8 | 1.23 |
| 11 | 96.5 | 3.5 | 27.6 | 63.8 | 86.6 | 83.6 | 1.3 | 64.3 | 2.33 |
| 12 | 96.5 | 3.5 | 27.6 | 87.1 | 82.4 | 79.5 | 0.5 | 159.0 | 5.76 |
| 13 | 96.5 | 3.5 | 27.6 | 60.8 | 94.4 | 91.1 | 1.4 | 65.1 | 2.36 |
| 14 | 96.5 | 3.5 | 27.6 | 15.2 | 98.2 | 94.8 | 3.0 | 31.6 | 1.14 |
| 18 | 96.4 | 3.6 | 26.8 | 88.5 | 78.8 | 76.0 | 0.4 | 190.0 | 7.09 |
| 19 | 96.4 | 3.6 | 26.8 | 26.5 | 97.4 | 93.9 | 2.6 | 36.1 | 1.35 |
| 20 | 96.4 | 3.6 | 26.8 | 9.7 | 98.5 | 95.0 | 3.3 | 28.8 | 1.07 |
| 21 | 96.4 | 3.6 | 26.8 | 8.1 | 98.2 | 94.7 | 3.3 | 28.7 | 1.07 |
| 23 | 96.4 | 3.6 | 26.8 | 78.3 | 86.5 | 83.4 | 0.8 | 104.2 | 3.89 |
| 24 | 96.4 | 3.6 | 26.8 | 14.7 | 97.6 | 94.1 | 3.1 | 30.3 | 1.13 |
| 25 | 96.4 | 3.6 | 26.8 | 8.4 | 100.0 | 96.4 | 3.3 | 29.2 | 1.09 |

TABLE 4. Filter Element of Example 2

| IgG Solution Number | Initial Monomeric IgG (%) | Initial Aggregated IgG (%) | Initial Composition Ratio | 386 g/L Challenge Load | | | | | | Ratio of Final Composition Ratio to Initial Composition Ratio |
|---|---|---|---|---|---|---|---|---|---|---|
| | | | | Filtration Performance | | Final Monomeric IgG Recovered (%) | Final Aggregated IgG Recovered (%) | Final Composition Ratio | | |
| | | | | Aggregated IgG Removed (%) | Monomeric IgG Yield (%) | | | | | |
| 1 | 96.6 | 3.4 | 28.4 | 89.6 | 87.6 | 84.6 | 0.4 | 211.5 | | 7.45 |
| 2 | 96.5 | 3.5 | 27.6 | 73.0 | 95.5 | 92.2 | 0.9 | 102.4 | | 3.71 |
| 3 | 96.5 | 3.5 | 27.6 | 54.4 | 97.3 | 93.9 | 1.6 | 58.7 | | 2.13 |
| 4 | 96.6 | 3.4 | 28.4 | 9.8 | 98.1 | 94.8 | 3.1 | 30.6 | | 1.08 |
| 6 | 96.5 | 3.5 | 27.6 | 78.7 | 89.5 | 86.4 | 0.7 | 123.4 | | 4.47 |
| 7 | 96.5 | 3.5 | 27.6 | 44.7 | 97.6 | 94.2 | 1.9 | 49.6 | | 1.80 |
| 8 | 96.5 | 3.5 | 27.6 | 16.9 | 98.3 | 94.9 | 2.9 | 32.7 | | 1.18 |
| 9 | 96.5 | 3.5 | 27.6 | 7.9 | 98.4 | 95.0 | 3.2 | 29.7 | | 1.08 |
| 11 | 96.6 | 3.4 | 28.4 | 68.8 | 87.4 | 84.4 | 1.1 | 76.7 | | 2.70 |
| 12 | 96.5 | 3.5 | 27.6 | 49.6 | 97.3 | 93.9 | 1.8 | 52.2 | | 1.89 |
| 13 | 96.5 | 3.5 | 27.6 | 35.8 | 98.0 | 94.6 | 2.2 | 43.0 | | 1.56 |
| 14 | 96.5 | 3.5 | 27.6 | 9.7 | 98.3 | 94.9 | 3.2 | 29.6 | | 1.07 |
| 18 | 96.5 | 3.5 | 27.6 | 80.4 | 95.6 | 92.3 | 0.7 | 131.8 | | 4.77 |
| 19 | 96.5 | 3.5 | 27.6 | 17.9 | 98.3 | 94.9 | 2.9 | 32.7 | | 1.18 |
| 20 | 96.5 | 3.5 | 27.6 | 5.2 | 98.8 | 95.3 | 3.3 | 28.9 | | 1.05 |
| 21 | 96.5 | 3.5 | 27.6 | 4.1 | 98.6 | 95.1 | 3.4 | 28.0 | | 1.01 |
| 23 | 96.5 | 3.5 | 27.6 | 54.7 | 93.8 | 90.5 | 1.6 | 56.6 | | 2.05 |
| 24 | 96.4 | 3.6 | 26.8 | 5.3 | 98.7 | 95.1 | 3.4 | 28.0 | | 1.04 |
| 25 | 96.5 | 3.5 | 27.6 | 4.0 | 98.9 | 95.4 | 3.4 | 28.0 | | 1.01 |
| 26 | 96.5 | 3.5 | 27.6 | 6.2 | 99.0 | 95.5 | 3.3 | 28.9 | | 1.05 |

TABLE 5. Filter Element of Example 3

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 2 | 96.9 | 3.1 | 31.3 | 83.5 | 83.4 | 80.8 | 0.5 | 161.6 | 5.16 |
| 3 | 97.0 | 3.0 | 32.3 | 37.6 | 97.2 | 94.3 | 1.9 | 49.6 | 1.53 |
| 4 | 97.0 | 3.0 | 32.3 | 9.6 | 99.0 | 96.0 | 2.7 | 35.5 | 1.10 |
| 7 | 96.8 | 3.2 | 30.3 | 42.6 | 95.6 | 92.5 | 1.8 | 51.4 | 1.70 |
| 8 | 96.8 | 3.2 | 30.3 | 15.8 | 99.6 | 96.4 | 2.7 | 35.7 | 1.18 |
| 9 | 96.8 | 3.2 | 30.3 | 6.0 | 99.9 | 96.7 | 3.0 | 32.2 | 1.06 |
| 12 | 96.8 | 3.2 | 30.3 | 40.0 | 96.5 | 93.4 | 1.9 | 49.1 | 1.62 |
| 13 | 96.8 | 3.2 | 30.3 | 18.3 | 100.6 | 97.4 | 2.6 | 37.5 | 1.24 |
| 14 | 96.9 | 3.1 | 31.3 | 4.6 | 100.6 | 97.5 | 3.0 | 32.5 | 1.04 |
| 19 | 96.7 | 3.3 | 29.3 | 12.9 | 100.3 | 97.0 | 2.9 | 33.4 | 1.14 |
| 20 | 96.7 | 3.3 | 29.3 | 3.9 | 101.2 | 97.9 | 3.2 | 30.6 | 1.04 |
| 21 | 96.8 | 3.2 | 30.3 | 2.1 | 100.6 | 97.4 | 3.1 | 31.4 | 1.04 |

TABLE 6. Filter Element of Example 4

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.3 | 2.7 | 36.0 | 11.5 | 98.5 | 95.8 | 2.4 | 39.9 | 1.11 |
| 2 | 96.6 | 3.4 | 28.4 | 46.7 | 93.2 | 90.0 | 1.8 | 50.0 | 1.76 |
| 3 | 96.7 | 3.3 | 29.3 | 7.5 | 98.1 | 94.9 | 3.1 | 30.6 | 1.04 |
| 4 | 96.7 | 3.3 | 29.3 | 5.5 | 98.2 | 95.0 | 3.1 | 30.6 | 1.04 |
| 6 | 96.6 | 3.4 | 28.4 | 23.9 | 97.4 | 94.1 | 2.6 | 36.2 | 1.28 |
| 7 | 96.5 | 3.5 | 27.6 | 7.5 | 98.7 | 95.2 | 3.2 | 29.7 | 1.08 |
| 8 | 96.5 | 3.5 | 27.6 | 5.7 | 98.4 | 95.0 | 3.3 | 28.8 | 1.04 |
| 9 | 96.5 | 3.5 | 27.6 | 3.9 | 98.6 | 95.1 | 3.4 | 28.0 | 1.01 |
| 11 | 96.4 | 3.6 | 26.8 | 57.4 | 96.1 | 92.6 | 1.5 | 61.7 | 2.30 |
| 12 | 96.5 | 3.5 | 27.6 | 15.9 | 97.9 | 94.5 | 2.9 | 32.6 | 1.18 |
| 13 | 96.5 | 3.5 | 27.6 | 7.7 | 98.6 | 95.1 | 3.2 | 29.7 | 1.08 |
| 14 | 96.5 | 3.5 | 27.6 | 5.9 | 98.1 | 94.7 | 3.3 | 28.7 | 1.04 |
| 18 | 96.5 | 3.5 | 27.6 | 36.1 | 96.8 | 93.4 | 2.2 | 42.4 | 1.51 |
| 19 | 96.3 | 3.7 | 26.0 | 6.3 | 98.2 | 94.6 | 3.5 | 27.0 | 1.04 |
| 20 | 96.3 | 3.7 | 26.0 | 3.3 | 98.9 | 95.2 | 3.6 | 26.4 | 1.02 |
| 21 | 96.3 | 3.7 | 26.0 | 3.1 | 99.0 | 95.3 | 3.6 | 26.5 | 1.02 |
| 23 | 96.4 | 3.6 | 26.8 | 60.2 | 90.6 | 87.3 | 1.4 | 62.3 | 2.32 |
| 24 | 96.3 | 3.7 | 26.0 | 4.5 | 98.9 | 95.2 | 3.5 | 27.2 | 1.05 |
| 25 | 96.3 | 3.7 | 26.0 | 2.9 | 99.0 | 95.3 | 3.6 | 26.5 | 1.02 |
| 26 | 96.3 | 3.7 | 26.0 | 2.5 | 99.0 | 95.3 | 3.6 | 26.5 | 1.02 |

TABLE 7. Filter Element of Example 4

| IgG Solution Number | Initial Monomeric IgG (%) | Initial Aggregated IgG (%) | Initial Composition Ratio | 96.5 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 90.9 | 87.9 | 85.4 | 0.3 | 284.7 | 8.20 |
| 3 | 96.7 | 3.3 | 29.3 | 25.4 | 97.6 | 94.4 | 2.5 | 37.8 | 1.29 |
| 7 | 96.6 | 3.4 | 28.4 | 20.0 | 97.8 | 94.5 | 2.7 | 35.0 | 1.23 |
| 19 | 96.4 | 3.6 | 26.8 | 16.1 | 98.2 | 94.7 | 3.0 | 31.6 | 1.18 |
| 23 | 96.4 | 3.6 | 26.8 | 74.2 | 88.5 | 85.3 | 0.9 | 94.8 | 3.54 |
| 25 | 96.3 | 3.7 | 26.0 | 5.5 | 98.4 | 94.8 | 3.5 | 27.1 | 1.04 |

TABLE 8. Filter Element of Example 5

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.0 | 3.0 | 32.3 | 9.4 | 97.1 | 94.2 | 2.7 | 34.9 | 1.08 |
| 2 | 96.7 | 3.3 | 29.3 | 14.1 | 96.7 | 93.5 | 2.8 | 33.4 | 1.14 |
| 3 | 96.7 | 3.3 | 29.3 | 15.9 | 96.2 | 93.0 | 2.8 | 33.2 | 1.13 |
| 6 | 96.6 | 3.4 | 28.4 | 65.9 | 83.6 | 80.8 | 1.2 | 67.3 | 2.37 |
| 7 | 96.6 | 3.4 | 28.4 | 80.1 | 85.8 | 82.9 | 0.7 | 118.4 | 4.16 |
| 8 | 96.5 | 3.5 | 27.6 | 43.7 | 95.5 | 92.2 | 2.0 | 46.1 | 1.67 |
| 11 | 96.5 | 3.5 | 27.6 | 59.0 | 87.3 | 84.2 | 1.4 | 60.1 | 2.17 |
| 12 | 96.5 | 3.5 | 27.6 | 82.8 | 85.1 | 82.1 | 0.6 | 136.8 | 4.96 |
| 13 | 96.5 | 3.5 | 27.6 | 44.7 | 96.0 | 92.6 | 1.9 | 48.7 | 1.76 |
| 18 | 96.5 | 3.5 | 27.6 | 89.8 | 82.7 | 79.8 | 0.4 | 199.5 | 7.23 |
| 19 | 96.4 | 3.6 | 26.8 | 21.0 | 98.3 | 94.8 | 2.8 | 33.8 | 1.26 |
| 20 | 96.4 | 3.6 | 26.8 | 9.4 | 98.7 | 95.1 | 3.3 | 28.8 | 1.07 |
| 23 | 96.3 | 3.7 | 26.0 | 75.7 | 81.8 | 78.8 | 0.9 | 87.6 | 3.37 |
| 24 | 96.3 | 3.7 | 26.0 | 11.4 | 98.5 | 94.9 | 3.3 | 28.8 | 1.11 |
| 25 | 96.3 | 3.7 | 26.0 | 7.7 | 99.0 | 95.3 | 3.4 | 28.0 | 1.08 |

TABLE 9. Filter Element of Example 6

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.7 | 3.3 | 29.3 | 57.8 | 96.5 | 93.3 | 1.4 | 66.6 | 2.27 |
| 2 | 96.7 | 3.3 | 29.3 | 54.5 | 96.9 | 93.7 | 1.5 | 62.5 | 2.13 |
| 3 | 96.6 | 3.4 | 28.4 | 31.4 | 97.2 | 93.9 | 2.3 | 40.8 | 1.44 |
| 6 | 96.6 | 3.4 | 28.4 | 63.1 | 95.4 | 92.2 | 1.3 | 70.9 | 2.50 |
| 7 | 96.6 | 3.4 | 28.4 | 36.2 | 97.1 | 93.8 | 2.2 | 42.6 | 1.50 |
| 8 | 96.5 | 3.5 | 27.6 | 19.2 | 97.4 | 94.0 | 2.8 | 33.6 | 1.22 |
| 11 | 96.5 | 3.5 | 27.6 | 67.2 | 93.9 | 90.6 | 1.1 | 82.4 | 2.99 |
| 12 | 96.5 | 3.5 | 27.6 | 41.6 | 97.3 | 93.9 | 2.0 | 46.9 | 1.70 |
| 13 | 96.5 | 3.5 | 27.6 | 19.9 | 97.9 | 94.5 | 2.8 | 33.7 | 1.22 |
| 18 | 96.5 | 3.5 | 27.6 | 49.2 | 96.9 | 93.5 | 1.8 | 51.9 | 1.88 |
| 19 | 96.4 | 3.6 | 26.8 | 19.8 | 98.0 | 94.5 | 2.9 | 32.6 | 1.22 |
| 20 | 96.4 | 3.6 | 26.8 | 11.4 | 98.0 | 94.5 | 3.2 | 29.5 | 1.10 |

TABLE 10. Filter Element of Example 7

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.5 | 3.5 | 27.6 | 76.8 | 84.8 | 81.8 | 0.8 | 102.2 | 3.70 |
| 2 | 96.7 | 3.3 | 29.3 | 91.4 | 88.4 | 85.5 | 0.3 | 285.0 | 9.72 |
| 3 | 96.7 | 3.3 | 29.3 | 47.2 | 97.2 | 94.0 | 1.7 | 55.3 | 1.89 |
| 6 | 96.7 | 3.3 | 29.3 | 86.0 | 76.3 | 73.8 | 0.5 | 147.6 | 5.04 |
| 7 | 96.6 | 3.4 | 28.4 | 51.8 | 96.5 | 93.2 | 1.6 | 58.2 | 2.05 |
| 8 | 96.6 | 3.4 | 28.4 | 19.6 | 98.5 | 95.2 | 2.7 | 35.2 | 1.24 |
| 11 | 96.5 | 3.5 | 27.6 | 74.6 | 77.2 | 74.5 | 0.9 | 82.8 | 3.00 |
| 12 | 96.5 | 3.5 | 27.6 | 63.9 | 95.4 | 92.1 | 1.3 | 70.8 | 2.56 |
| 13 | 96.6 | 3.4 | 28.4 | 17.4 | 98.9 | 95.5 | 2.8 | 34.1 | 1.20 |
| 18 | 96.5 | 3.5 | 27.6 | 89.2 | 89.3 | 86.2 | 0.4 | 215.5 | 7.81 |
| 19 | 96.4 | 3.6 | 26.8 | 17.7 | 98.8 | 95.2 | 3.0 | 31.7 | 1.18 |
| 20 | 96.4 | 3.6 | 26.8 | 7.2 | 99.2 | 95.6 | 3.3 | 29.0 | 1.08 |

TABLE 11. Filter Element of Example 8

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.7 | 3.3 | 29.3 | 18.7 | 98.9 | 95.6 | 2.7 | 35.4 | 1.21 |
| 2 | 96.7 | 3.3 | 29.3 | 18.9 | 98.7 | 95.4 | 2.7 | 35.3 | 1.20 |
| 3 | 96.7 | 3.3 | 29.3 | 17.3 | 98.5 | 95.2 | 2.7 | 35.2 | 1.20 |
| 6 | 96.6 | 3.4 | 28.4 | 23.6 | 98.2 | 94.9 | 2.6 | 36.5 | 1.29 |
| 7 | 96.6 | 3.4 | 28.4 | 15.1 | 98.4 | 95.1 | 2.9 | 32.8 | 1.16 |
| 8 | 96.6 | 3.4 | 28.4 | 12.3 | 98.4 | 95.1 | 3.0 | 31.7 | 1.12 |
| 11 | 96.5 | 3.5 | 27.6 | 40.8 | 98.2 | 94.8 | 2.1 | 45.1 | 1.63 |
| 12 | 96.5 | 3.5 | 27.6 | 15.8 | 98.6 | 95.1 | 2.9 | 32.8 | 1.19 |
| 13 | 96.6 | 3.4 | 28.4 | 13.4 | 98.7 | 95.3 | 2.9 | 32.9 | 1.16 |
| 18 | 96.5 | 3.5 | 27.6 | 27.8 | 96.6 | 93.2 | 2.5 | 37.3 | 1.35 |
| 19 | 96.4 | 3.6 | 26.8 | 12.4 | 97.0 | 93.5 | 3.2 | 29.2 | 1.09 |
| 20 | 96.4 | 3.6 | 26.8 | 10.2 | 97.2 | 93.7 | 3.2 | 29.3 | 1.09 |
| 23 | 96.4 | 3.6 | 26.8 | 38.2 | 96.2 | 92.7 | 2.2 | 42.1 | 1.57 |
| 24 | 96.3 | 3.7 | 26.0 | 6.2 | 99.0 | 95.3 | 3.5 | 27.2 | 1.05 |
| 25 | 96.3 | 3.7 | 26.0 | 5.4 | 98.9 | 95.2 | 3.5 | 27.2 | 1.05 |

TABLE 12. Filter Element of Example 9

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.7 | 3.3 | 29.3 | 67.1 | 95.5 | 92.3 | 1.1 | 83.9 | 2.86 |
| 2 | 96.6 | 3.4 | 28.4 | 65.0 | 96.3 | 93.0 | 1.2 | 77.5 | 2.73 |
| 3 | 96.6 | 3.4 | 28.4 | 35.3 | 97.6 | 94.3 | 2.2 | 42.9 | 1.51 |
| 6 | 96.6 | 3.4 | 28.4 | 73.3 | 91.5 | 88.4 | 0.9 | 98.2 | 3.46 |
| 7 | 96.6 | 3.4 | 28.4 | 40.9 | 97.4 | 94.1 | 2.0 | 47.0 | 1.65 |
| 8 | 96.5 | 3.5 | 27.6 | 17.0 | 98.2 | 94.8 | 2.9 | 32.7 | 1.18 |
| 11 | 96.5 | 3.5 | 27.6 | 70.0 | 88.7 | 85.6 | 1.1 | 77.8 | 2.82 |
| 12 | 96.5 | 3.5 | 27.6 | 49.9 | 97.2 | 93.8 | 1.8 | 52.1 | 1.89 |
| 13 | 96.5 | 3.5 | 27.6 | 18.3 | 98.7 | 95.2 | 2.9 | 32.8 | 1.19 |
| 15 | 96.6 | 3.4 | 28.4 | 82.6 | 88.1 | 85.1 | 0.6 | 142.7 | 5.02 |
| 16 | 96.4 | 3.6 | 26.8 | 31.1 | 98.3 | 94.8 | 2.5 | 37.9 | 1.41 |
| 17 | 96.4 | 3.6 | 26.8 | 13.8 | 98.8 | 95.2 | 3.1 | 30.7 | 1.15 |
| 18 | 96.4 | 3.6 | 26.8 | 65.4 | 96.2 | 92.7 | 1.2 | 77.2 | 2.88 |
| 19 | 96.3 | 3.7 | 26.0 | 19.4 | 98.8 | 95.1 | 3.0 | 31.7 | 1.22 |
| 20 | 96.3 | 3.7 | 26.0 | 11.4 | 98.8 | 95.1 | 3.3 | 28.8 | 1.11 |
| 23 | 96.3 | 3.7 | 26.0 | 46.2 | 95.3 | 91.8 | 2.0 | 45.9 | 1.77 |
| 24 | 96.2 | 3.8 | 25.3 | 8.4 | 98.7 | 94.9 | 3.5 | 27.1 | 1.07 |
| 25 | 96.2 | 3.8 | 25.3 | 5.3 | 98.9 | 95.1 | 3.6 | 26.4 | 1.04 |

TABLE 13. Filter Element of Example 10

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.5 | 3.5 | 27.6 | 26.5 | 96.4 | 93.0 | 2.6 | 35.8 | 1.30 |
| 2 | 96.7 | 3.3 | 29.3 | 21.6 | 96.8 | 93.6 | 2.6 | 36.0 | 1.23 |
| 3 | 96.7 | 3.3 | 29.3 | 22.6 | 96.9 | 93.7 | 2.6 | 36.0 | 1.23 |
| 6 | 96.5 | 3.5 | 27.6 | 45.6 | 93.4 | 90.1 | 1.9 | 47.4 | 1.72 |
| 7 | 96.6 | 3.4 | 28.4 | 58.9 | 94.3 | 91.1 | 1.4 | 65.1 | 2.29 |
| 8 | 96.6 | 3.4 | 28.4 | 33.4 | 97.3 | 94.0 | 2.3 | 40.9 | 1.44 |
| 11 | 96.5 | 3.5 | 27.6 | 40.3 | 94.5 | 91.2 | 2.1 | 43.4 | 1.57 |
| 12 | 96.5 | 3.5 | 27.6 | 50.2 | 95.8 | 92.4 | 1.7 | 54.3 | 1.97 |
| 13 | 96.6 | 3.4 | 28.4 | 35.0 | 97.4 | 94.1 | 2.2 | 42.8 | 1.51 |
| 15 | 96.6 | 3.4 | 28.4 | 44.1 | 94.3 | 91.1 | 1.9 | 47.9 | 1.69 |
| 16 | 96.4 | 3.6 | 26.8 | 46.6 | 97.1 | 93.6 | 1.9 | 49.3 | 1.84 |
| 17 | 96.3 | 3.7 | 26.0 | 24.9 | 98.3 | 94.7 | 2.8 | 33.8 | 1.30 |
| 18 | 96.5 | 3.5 | 27.6 | 81.3 | 92.6 | 89.4 | 0.7 | 127.7 | 4.63 |
| 19 | 96.4 | 3.6 | 26.8 | 19.9 | 98.9 | 95.3 | 2.9 | 32.9 | 1.23 |
| 20 | 96.4 | 3.6 | 26.8 | 11.5 | 99.0 | 95.4 | 3.2 | 29.8 | 1.11 |
| 23 | 96.4 | 3.6 | 26.8 | 53.4 | 93.7 | 90.3 | 1.7 | 53.1 | 1.98 |
| 24 | 96.3 | 3.7 | 26.0 | 14.1 | 98.6 | 95.0 | 3.2 | 29.7 | 1.14 |
| 25 | 96.3 | 3.7 | 26.0 | 11.3 | 98.8 | 95.1 | 3.3 | 28.8 | 1.11 |

TABLE 14. Filter Element of Example 11

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.7 | 3.3 | 29.3 | 61.1 | 90.9 | 87.9 | 1.3 | 67.6 | 2.31 |
| 2 | 96.7 | 3.3 | 29.3 | 77.4 | 93.2 | 90.1 | 0.7 | 128.7 | 4.39 |
| 3 | 96.7 | 3.3 | 29.3 | 35.7 | 97.7 | 94.5 | 2.1 | 45.0 | 1.54 |
| 6 | 96.7 | 3.3 | 29.3 | 80.7 | 85.9 | 83.1 | 0.6 | 138.5 | 4.72 |
| 7 | 96.6 | 3.4 | 28.4 | 45.5 | 97.0 | 93.7 | 1.9 | 49.3 | 1.74 |
| 8 | 96.6 | 3.4 | 28.4 | 36.5 | 97.8 | 94.5 | 2.2 | 43.0 | 1.51 |
| 11 | 96.6 | 3.4 | 28.4 | 65.4 | 89.7 | 86.7 | 1.2 | 72.2 | 2.54 |
| 12 | 96.6 | 3.4 | 28.4 | 44.3 | 97.4 | 94.1 | 1.9 | 49.5 | 1.74 |
| 13 | 96.6 | 3.4 | 28.4 | 18.3 | 98.5 | 95.2 | 2.8 | 34.0 | 1.20 |
| 15 | 96.8 | 3.2 | 30.3 | 66.6 | 88.3 | 85.5 | 1.1 | 77.7 | 2.56 |
| 16 | 96.5 | 3.5 | 27.6 | 30.7 | 98.2 | 94.8 | 2.4 | 39.5 | 1.43 |
| 17 | 96.5 | 3.5 | 27.6 | 14.1 | 98.9 | 95.4 | 3.0 | 31.8 | 1.15 |
| 18 | 96.5 | 3.5 | 27.6 | 78.4 | 93.9 | 90.6 | 0.8 | 113.2 | 4.10 |
| 19 | 96.4 | 3.6 | 26.8 | 17.9 | 98.9 | 95.3 | 3.0 | 31.8 | 1.19 |
| 20 | 96.4 | 3.6 | 26.8 | 9.6 | 98.9 | 95.3 | 3.3 | 28.9 | 1.08 |

TABLE 15. Filter Element of Example 12

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.3 | 45.4 | 96.6 | 93.5 | 1.7 | 55.0 | 1.82 |
| 2 | 96.7 | 3.3 | 29.3 | 30.5 | 98.4 | 95.2 | 2.3 | 41.4 | 1.41 |
| 3 | 96.7 | 3.3 | 29.3 | 13.3 | 99.4 | 96.1 | 2.9 | 33.1 | 1.13 |
| 4 | 96.7 | 3.3 | 29.3 | 2.4 | 100.3 | 97.0 | 3.2 | 30.3 | 1.03 |
| 6 | 96.7 | 3.3 | 29.3 | 55.3 | 96.6 | 93.4 | 1.5 | 62.2 | 2.12 |
| 7 | 96.7 | 3.3 | 29.3 | 14.6 | 100.1 | 96.8 | 2.8 | 34.6 | 1.18 |
| 8 | 96.6 | 3.4 | 28.4 | 2.8 | 101.0 | 97.6 | 3.3 | 29.6 | 1.04 |
| 9 | 96.6 | 3.4 | 28.4 | 0.3 | 100.3 | 96.9 | 3.4 | 28.5 | 1.00 |
| 18 | 96.4 | 3.6 | 26.8 | 29.5 | 98.2 | 94.7 | 2.5 | 37.9 | 1.41 |
| 19 | 96.3 | 3.7 | 26.0 | 2.7 | 99.5 | 95.8 | 3.6 | 26.6 | 0.99 |
| 20 | 96.3 | 3.7 | 26.0 | 1.2 | 100.8 | 97.1 | 3.7 | 26.2 | 1.01 |
| 21 | 96.3 | 3.7 | 26.0 | 0.7 | 100.8 | 97.1 | 3.7 | 26.2 | 1.01 |
| 23 | 96.3 | 3.7 | 26.0 | 10.9 | 99.3 | 95.6 | 3.3 | 29.0 | 1.11 |
| 24 | 96.2 | 3.8 | 25.3 | 0.5 | 99.5 | 95.7 | 3.8 | 25.2 | 1.00 |
| 25 | 96.2 | 3.8 | 25.3 | 0.3 | 100.1 | 96.3 | 3.8 | 25.3 | 1.00 |
| 26 | 96.2 | 3.8 | 25.3 | 0.0 | 100.2 | 96.4 | 3.8 | 25.4 | 1.00 |

TABLE 16. Filter Element of Example 12

| IgG Solution Number | Initial Monomeric IgG (%) | Initial Aggregated IgG (%) | Initial Composition Ratio | 96.5 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 83.8 | 87.6 | 85.1 | 0.5 | 170.2 | 4.90 |
| 3 | 96.7 | 3.3 | 29.3 | 25.5 | 97.9 | 94.7 | 2.5 | 37.9 | 1.29 |
| 7 | 96.6 | 3.4 | 28.4 | 15.4 | 98.3 | 95.0 | 2.9 | 32.8 | 1.15 |
| 19 | 96.4 | 3.6 | 26.8 | 5.4 | 98.8 | 95.2 | 3.4 | 28.0 | 1.04 |
| 23 | 96.4 | 3.6 | 26.8 | 20.7 | 97.0 | 93.5 | 2.9 | 32.2 | 1.20 |
| 25 | 96.3 | 3.7 | 26.0 | 2.9 | 99.2 | 95.5 | 3.6 | 26.5 | 1.02 |

TABLE 17. Filter Element of Example 13

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.3 | 28.5 | 98.2 | 95.1 | 2.3 | 41.3 | 1.36 |
| 2 | 96.7 | 3.3 | 29.3 | 18.5 | 98.6 | 95.3 | 2.7 | 35.3 | 1.20 |
| 3 | 96.7 | 3.3 | 29.3 | 11.9 | 99.1 | 95.8 | 2.9 | 33.0 | 1.13 |
| 4 | 96.7 | 3.3 | 29.3 | 8.3 | 99.6 | 96.3 | 3.0 | 32.1 | 1.09 |
| 6 | 96.7 | 3.3 | 29.3 | 32.3 | 98.4 | 95.2 | 2.2 | 43.3 | 1.48 |
| 7 | 96.7 | 3.3 | 29.3 | 12.1 | 99.9 | 96.6 | 2.9 | 33.3 | 1.14 |
| 8 | 96.6 | 3.4 | 28.4 | 7.0 | 100.6 | 97.2 | 3.2 | 30.4 | 1.07 |
| 9 | 96.6 | 3.4 | 28.4 | 5.3 | 99.9 | 96.5 | 3.2 | 30.2 | 1.06 |
| 18 | 96.4 | 3.6 | 26.8 | 22.2 | 98.3 | 94.8 | 2.8 | 33.8 | 1.26 |
| 19 | 96.3 | 3.7 | 26.0 | 5.9 | 99.1 | 95.4 | 3.5 | 27.3 | 1.05 |
| 20 | 96.3 | 3.7 | 26.0 | 4.6 | 100.4 | 96.7 | 3.5 | 27.6 | 1.06 |
| 21 | 96.3 | 3.7 | 26.0 | 4.4 | 100.4 | 96.7 | 3.5 | 27.6 | 1.06 |
| 23 | 96.3 | 3.7 | 26.0 | 11.7 | 99.0 | 95.3 | 3.3 | 28.9 | 1.16 |
| 24 | 96.2 | 3.8 | 25.3 | 3.4 | 99.5 | 95.7 | 3.7 | 25.9 | 1.02 |
| 25 | 96.2 | 3.8 | 25.3 | 2.7 | 100.0 | 96.2 | 3.7 | 26.0 | 1.03 |
| 26 | 96.2 | 3.8 | 25.3 | 2.4 | 100.1 | 96.3 | 3.7 | 26.0 | 1.03 |

TABLE 18. Filter Element of Example 13

| IgG Solution Number | Initial Monomeric IgG (%) | Initial Aggregated IgG (%) | Initial Composition Ratio | 96.5 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 54.4 | 94.3 | 91.7 | 1.3 | 70.5 | 2.03 |
| 3 | 96.7 | 3.3 | 29.3 | 41.1 | 96.8 | 93.6 | 1.9 | 49.3 | 1.68 |
| 7 | 96.6 | 3.4 | 28.4 | 34.4 | 97.0 | 93.7 | 2.2 | 42.6 | 1.50 |
| 19 | 96.4 | 3.6 | 26.8 | 18.6 | 97.9 | 94.4 | 2.9 | 32.5 | 1.21 |
| 23 | 96.4 | 3.6 | 26.8 | 32.4 | 95.5 | 92.1 | 2.4 | 38.4 | 1.43 |
| 25 | 96.3 | 3.7 | 26.0 | 11.3 | 98.2 | 94.6 | 3.3 | 28.7 | 1.10 |

TABLE 19. Filter Element of Example 14

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 61.4 | 73.0 | 71.0 | 1.1 | 64.5 | 1.86 |
| 2 | 96.5 | 3.5 | 27.6 | 41.0 | 96.7 | 93.3 | 2.1 | 44.4 | 1.61 |
| 3 | 96.8 | 3.2 | 30.3 | 5.9 | 99.0 | 95.8 | 3.0 | 31.9 | 1.05 |
| 4 | 96.8 | 3.2 | 30.3 | 5.9 | 99.0 | 95.8 | 3.0 | 31.9 | 1.05 |
| 5 | 96.5 | 3.5 | 27.6 | 1.2 | 98.9 | 95.4 | 3.5 | 27.2 | 0.99 |
| 6 | 96.6 | 3.4 | 28.4 | 81.3 | 71.2 | 68.8 | 0.6 | 114.7 | 4.04 |
| 7 | 96.5 | 3.5 | 27.6 | 53.4 | 95.6 | 92.3 | 1.6 | 57.7 | 2.09 |
| 8 | 96.5 | 3.5 | 27.6 | 7.8 | 98.5 | 95.1 | 3.2 | 29.7 | 1.08 |
| 9 | 96.7 | 3.3 | 29.3 | 0.3 | 99.9 | 96.6 | 3.3 | 29.3 | 1.00 |
| 10 | 96.4 | 3.6 | 26.8 | 0.0 | 99.7 | 96.1 | 3.6 | 26.7 | 1.00 |
| 18 | 96.4 | 3.6 | 26.8 | 80.4 | 81.4 | 78.5 | 0.7 | 112.1 | 4.18 |
| 19 | 96.2 | 3.8 | 25.3 | 9.0 | 98.9 | 95.1 | 3.5 | 27.2 | 1.08 |
| 20 | 96.3 | 3.7 | 26.0 | 2.2 | 99.1 | 95.4 | 3.6 | 26.5 | 1.02 |
| 21 | 96.3 | 3.7 | 26.0 | 1.3 | 99.0 | 95.3 | 3.7 | 25.8 | 0.99 |
| 22 | 96.3 | 3.7 | 26.0 | 0.5 | 99.8 | 96.1 | 3.7 | 26.0 | 1.00 |
| 23 | 96.4 | 3.6 | 26.8 | 67.0 | 85.3 | 82.2 | 1.2 | 68.5 | 2.56 |
| 24 | 96.6 | 3.4 | 28.4 | 3.3 | 98.4 | 95.1 | 3.3 | 28.8 | 1.01 |
| 25 | 96.3 | 3.7 | 26.0 | 3.9 | 98.6 | 95.0 | 3.6 | 26.4 | 1.01 |
| 26 | 96.2 | 3.8 | 25.3 | 3.3 | 98.7 | 94.9 | 3.7 | 25.6 | 1.01 |
| 27 | 96.2 | 3.8 | 25.3 | 1.6 | 98.6 | 94.9 | 3.7 | 25.6 | 1.01 |

TABLE 20. Filter Element of Example 15

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 73.8 | 68.0 | 66.1 | 0.7 | 94.41 | 2.72 |
| 2 | 96.5 | 3.5 | 27.6 | 58.7 | 97.5 | 94.1 | 1.4 | 67.2 | 2.43 |
| 3 | 96.8 | 3.2 | 30.3 | 2.6 | 99.1 | 95.9 | 3.1 | 30.9 | 1.02 |
| 4 | 96.8 | 3.2 | 30.3 | 2.6 | 99.1 | 95.9 | 3.1 | 30.9 | 1.02 |
| 5 | 96.5 | 3.5 | 27.6 | 0.6 | 98.8 | 95.3 | 3.5 | 27.2 | 0.99 |
| 6 | 96.6 | 3.4 | 28.4 | 52.8 | 75.9 | 73.3 | 1.6 | 45.8 | 1.61 |
| 7 | 96.5 | 3.5 | 27.6 | 33.4 | 97.1 | 93.7 | 2.3 | 40.7 | 1.46 |
| 8 | 96.5 | 3.5 | 27.6 | 6.8 | 99.0 | 95.5 | 3.3 | 28.9 | 1.05 |
| 9 | 96.7 | 3.3 | 29.3 | -0.7 | 99.9 | 96.6 | 3.3 | 29.3 | 1.00 |
| 10 | 96.4 | 3.6 | 26.8 | 0.6 | 99.8 | 96.2 | 3.6 | 26.7 | 1.00 |
| 18 | 96.4 | 3.6 | 26.8 | 89.9 | 83.9 | 80.9 | 0.4 | 202.2 | 7.54 |
| 19 | 96.2 | 3.8 | 25.3 | 3.3 | 99.9 | 96.1 | 3.7 | 26.0 | 1.03 |
| 20 | 96.3 | 3.7 | 26.0 | 0.9 | 99.9 | 96.2 | 3.7 | 26.0 | 1.00 |
| 21 | 96.3 | 3.7 | 26.0 | 1.1 | 99.3 | 95.6 | 3.7 | 25.8 | 0.99 |
| 22 | 96.3 | 3.7 | 26.0 | 0.2 | 100.2 | 96.5 | 3.7 | 26.1 | 1.00 |
| 23 | 96.4 | 3.6 | 26.8 | 56.8 | 86.1 | 83.0 | 1.6 | 51.9 | 1.94 |
| 24 | 96.6 | 3.4 | 28.4 | 7.5 | 98.7 | 95.3 | 3.1 | 30.7 | 1.08 |
| 25 | 96.3 | 3.7 | 26.0 | 1.5 | 99.3 | 95.6 | 3.6 | 26.5 | 1.02 |
| 26 | 96.2 | 3.8 | 25.3 | 1.8 | 99.1 | 95.3 | 3.7 | 25.8 | 1.02 |
| 27 | 96.2 | 3.8 | 25.3 | 2.1 | 99.0 | 95.2 | 3.7 | 25.7 | 1.02 |

TABLE 21. Filter Element of Example 16

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 72.0 | 87.1 | 84.7 | 0.8 | 105.9 | 3.05 |
| 2 | 96.5 | 3.5 | 27.6 | 70.2 | 91.9 | 88.7 | 1.0 | 88.7 | 3.21 |
| 3 | 96.8 | 3.2 | 30.3 | 28.6 | 98.7 | 95.5 | 2.3 | 41.5 | 1.37 |
| 4 | 96.8 | 3.2 | 30.3 | 28.6 | 98.7 | 95.5 | 2.3 | 41.5 | 1.37 |
| 5 | 96.5 | 3.5 | 27.6 | 1.1 | 99.3 | 95.8 | 3.5 | 27.4 | 0.99 |
| 6 | 96.6 | 3.4 | 28.4 | 77.4 | 79.2 | 76.5 | 0.8 | 95.6 | 3.37 |
| 7 | 96.5 | 3.5 | 27.6 | 68.7 | 93.6 | 90.3 | 1.1 | 82.1 | 2.97 |
| 8 | 96.5 | 3.5 | 27.6 | 24.5 | 99.2 | 95.7 | 2.6 | 36.8 | 1.33 |
| 9 | 96.7 | 3.3 | 29.3 | 12.1 | 100.1 | 96.8 | 2.9 | 33.4 | 1.14 |
| 18 | 96.4 | 3.6 | 26.8 | 86.1 | 88.7 | 85.5 | 0.5 | 171.0 | 6.38 |
| 19 | 96.2 | 3.8 | 25.3 | 4.9 | 100.5 | 96.7 | 3.6 | 26.9 | 1.06 |
| 21 | 96.3 | 3.7 | 26.0 | 0.3 | 99.8 | 96.1 | 3.7 | 26.0 | 1.00 |
| 23 | 96.4 | 3.6 | 26.8 | 55.7 | 91.2 | 87.9 | 1.6 | 54.9 | 2.05 |
| 24 | 96.6 | 3.4 | 28.4 | 2.2 | 99.4 | 96.0 | 3.3 | 29.1 | 1.02 |
| 25 | 96.3 | 3.7 | 26.0 | 0.7 | 99.2 | 95.5 | 3.7 | 25.8 | 0.99 |
| 26 | 96.2 | 3.8 | 25.3 | 1.7 | 99.0 | 95.2 | 3.7 | 257 | 1.01 |
| 27 | 96.2 | 3.8 | 25.3 | 1.0 | 99.7 | 95.9 | 3.8 | 25.2 | 1.00 |

TABLE 22. Filter Element of Example 17

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.2 | 2.8 | 34.7 | 97.5 | 63.6 | 61.8 | 0.1 | 618.0 | 17.80 |
| 2 | 96.5 | 3.5 | 27.6 | 33.5 | 97.4 | 94.0 | 2.3 | 40.9 | 1.48 |
| 3 | 96.8 | 3.2 | 30.3 | 1.3 | 99.9 | 96.7 | 3.2 | 30.2 | 1.00 |
| 4 | 96.8 | 3.2 | 30.3 | 1.3 | 99.9 | 96.7 | 3.2 | 30.2 | 1.00 |
| 5 | 96.5 | 3.5 | 27.6 | 0.2 | 99.7 | 96.2 | 3.5 | 27.5 | 1.00 |
| 6 | 96.6 | 3.4 | 28.4 | 95.3 | 66.8 | 64.5 | 0.2 | 322.5 | 11.4 |
| 7 | 96.5 | 3.5 | 27.6 | 37.9 | 97.7 | 94.3 | 2.2 | 42.9 | 1.55 |
| 8 | 96.5 | 3.5 | 27.6 | 15.9 | 88.6 | 85.5 | 2.9 | 29.5 | 1.07 |
| 18 | 96.4 | 3.6 | 26.8 | 64.8 | 82.4 | 79.4 | 1.3 | 61.1 | 2.28 |
| 19 | 96.2 | 3.8 | 25.3 | 0.9 | 100.8 | 97.0 | 3.8 | 25.5 | 1.01 |
| 21 | 96.3 | 3.7 | 26.0 | 0.0 | 100.1 | 96.4 | 3.7 | 26.0 | 1.00 |
| 24 | 96.6 | 3.4 | 28.4 | 1.3 | 99.5 | 96.1 | 3.4 | 28.3 | 1.00 |
| 25 | 96.3 | 3.7 | 26.0 | 0.5 | 99.9 | 96.2 | 3.7 | 26.0 | 1.00 |
| 26 | 96.2 | 3.8 | 25.3 | 1.1 | 99.4 | 95.6 | 3.8 | 25.2 | 1.00 |
| 27 | 96.2 | 3.8 | 25.3 | 97.5 | 63.6 | 61.2 | 0.1 | 612.0 | 24.19 |

TABLE 23. Filter Element of Example 18

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.3 | 95.0 | 83.3 | 80.6 | 0.2 | 403.0 | 13.30 |
| 2 | 96.7 | 3.3 | 29.3 | 92.5 | 87.7 | 84.8 | 0.2 | 424.0 | 14.47 |
| 3 | 96.7 | 3.3 | 29.3 | 42.3 | 97.3 | 94.1 | 1.9 | 49.5 | 1.69 |
| 4 | 96.6 | 3.4 | 28.4 | 3.0 | 99.9 | 96.5 | 3.3 | 29.2 | 1.03 |
| 6 | 96.6 | 3.4 | 28.4 | 94.8 | 75.0 | 72.5 | 0.2 | 362.5 | 12.76 |
| 7 | 96.7 | 3.3 | 29.3 | 48.3 | 96.7 | 93.5 | 1.7 | 55.0 | 1.87 |
| 8 | 96.6 | 3.4 | 28.4 | 2.2 | 99.9 | 96.5 | 3.3 | 29.2 | 1.02 |
| 9 | 96.5 | 3.5 | 27.6 | 0.2 | 101.1 | 97.6 | 3.5 | 27.9 | 1.01 |
| 18 | 96.3 | 3.7 | 26.0 | 92.5 | 86.7 | 83.5 | 0.3 | 278.3 | 10.70 |
| 19 | 96.3 | 3.7 | 26.0 | 2.2 | 100.6 | 96.9 | 3.6 | 26.9 | 1.03 |
| 20 | 96.3 | 3.7 | 26.0 | 0.7 | 100.3 | 96.6 | 3.7 | 26.1 | 1.00 |
| 21 | 96.3 | 3.7 | 26.0 | 0.6 | 100.4 | 96.7 | 3.7 | 26.1 | 1.00 |
| 23 | 96.2 | 3.8 | 25.3 | 63.8 | 90.5 | 87.1 | 1.4 | 62.2 | 2.46 |

TABLE 24. Filter Element of Example 19

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.3 | 90.7 | 86.1 | 83.3 | 0.3 | 277.7 | 9.17 |
| 2 | 96.7 | 3.3 | 29.3 | 91.6 | 87.7 | 84.8 | 0.3 | 282.7 | 9.65 |
| 3 | 96.7 | 3.3 | 29.3 | 66.6 | 94.5 | 91.4 | 1.1 | 83.1 | 2.83 |
| 4 | 96.6 | 3.4 | 28.4 | 15.0 | 99.0 | 95.6 | 2.9 | 33.0 | 1.16 |
| 6 | 96.6 | 3.4 | 28.4 | 93.5 | 75.2 | 72.6 | 0.2 | 363.0 | 12.78 |
| 7 | 96.7 | 3.3 | 29.3 | 54.8 | 95.5 | 92.3 | 1.5 | 61.5 | 2.10 |
| 8 | 96.6 | 3.4 | 28.4 | 7.8 | 99.2 | 95.8 | 3.1 | 30.9 | 1.09 |
| 9 | 96.5 | 3.5 | 27.6 | 1.8 | 100.6 | 97.1 | 3.4 | 28.5 | 1.03 |
| 18 | 96.3 | 3.7 | 26.0 | 94.7 | 83.6 | 80.5 | 0.2 | 402.5 | 15.48 |
| 19 | 96.3 | 3.7 | 26.0 | 5.8 | 100.3 | 96.6 | 3.5 | 27.6 | 1.06 |
| 20 | 96.3 | 3.7 | 26.0 | 1.9 | 100.4 | 96.7 | 3.6 | 26.9 | 1.03 |
| 21 | 96.3 | 3.7 | 26.0 | 1.0 | 100.3 | 96.6 | 3.7 | 26.1 | 1.00 |
| 23 | 96.2 | 3.8 | 25.3 | 70.1 | 86.9 | 83.6 | 1.1 | 76.0 | 3.00 |
| 25 | 96.2 | 3.8 | 25.3 | 0.3 | 101.1 | 97.3 | 3.8 | 25.6 | 1.01 |

TABLE 25. Filter Element of Example 20

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.3 | 57.6 | 93.3 | 90.3 | 1.4 | 64.5 | 2.13 |
| 2 | 96.7 | 3.3 | 29.3 | 76.9 | 89.8 | 86.8 | 0.8 | 108.5 | 3.70 |
| 3 | 96.7 | 3.3 | 29.3 | 75.9 | 90.7 | 87.7 | 0.8 | 109.6 | 3.74 |
| 4 | 96.6 | 3.4 | 28.4 | 54.5 | 95.8 | 92.5 | 1.5 | 61.7 | 2.17 |
| 6 | 96.6 | 3.4 | 28.4 | 79.9 | 79.6 | 76.9 | 0.7 | 109.9 | 3.87 |
| 7 | 96.7 | 3.3 | 29.3 | 81.1 | 89.2 | 86.3 | 0.6 | 143.8 | 4.91 |
| 8 | 96.6 | 3.4 | 28.4 | 12.1 | 98.7 | 95.3 | 3.0 | 31.8 | 1.12 |
| 9 | 96.5 | 3.5 | 27.6 | 5.8 | 99.5 | 96.0 | 3.3 | 29.1 | 1.06 |
| 18 | 96.3 | 3.7 | 26.0 | 76.9 | 84.0 | 80.9 | 0.9 | 89.9 | 3.46 |
| 19 | 96.3 | 3.7 | 26.0 | 8.5 | 99.1 | 95.4 | 3.4 | 28.1 | 1.08 |
| 20 | 96.3 | 3.7 | 26.0 | 4.6 | 99.2 | 95.5 | 3.5 | 27.3 | 1.05 |
| 21 | 96.3 | 3.7 | 26.0 | 3.8 | 99.5 | 95.8 | 3.6 | 26.6 | 1.02 |
| 23 | 96.2 | 3.8 | 25.3 | 50.7 | 89.8 | 86.4 | 1.9 | 45.5 | 1.80 |
| 24 | 96.1 | 3.9 | 24.6 | 5.0 | 99.2 | 95.3 | 3.7 | 25.7 | 1.04 |
| 25 | 96.2 | 3.8 | 25.3 | 3.1 | 99.4 | 95.6 | 3.7 | 25.8 | 1.02 |
| 26 | 96.2 | 3.8 | 25.3 | 4.1 | 99.4 | 95.6 | 3.6 | 26.5 | 1.05 |

TABLE 26. Filter Element of Example 21

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.1 | 3.4 | 97.1 | 94.0 | 3.1 | 30.2 | 1.0 |
| 2 | 96.7 | 3.3 | 29.4 | 6.4 | 96.6 | 93.4 | 3.1 | 30.3 | 1.0 |
| 3 | 96.7 | 3.3 | 29.2 | 5.6 | 96.8 | 93.6 | 3.1 | 29.9 | 1.0 |
| 4 | 96.6 | 3.4 | 28.8 | 6.9 | 96.8 | 93.6 | 3.1 | 29.9 | 1.0 |
| 6 | 96.6 | 3.4 | 28.2 | 68.1 | 71.2 | 68.8 | 1.1 | 63.0 | 2.2 |
| 7 | 96.7 | 3.3 | 28.9 | 87.7 | 76.7 | 74.2 | 0.4 | 180.4 | 6.2 |
| 8 | 96.6 | 3.4 | 28.0 | 40.7 | 96.6 | 93.2 | 2.0 | 45.6 | 1.6 |
| 9 | 96.5 | 3.5 | 27.9 | 8.4 | 99.8 | 96.4 | 3.2 | 30.4 | 1.1 |
| 18 | 96.3 | 3.7 | 26.3 | 88.1 | 69.8 | 67.2 | 0.4 | 154.7 | 5.9 |
| 19 | 96.3 | 3.7 | 25.9 | 7.7 | 100.2 | 96.5 | 3.4 | 28.1 | 1.1 |
| 20 | 96.3 | 3.7 | 26.2 | 0.4 | 100.2 | 96.6 | 3.7 | 26.4 | 1.0 |
| 21 | 96.3 | 3.7 | 26.3 | 1.0 | 100.3 | 96.6 | 3.6 | 26.6 | 1.0 |
| 23 | 96.2 | 3.8 | 25.3 | 68.4 | 82.4 | 79.3 | 1.2 | 66.0 | 2.6 |
| 24 | 96.1 | 3.9 | 24.9 | 1.9 | 100.1 | 96.2 | 3.8 | 25.4 | 1.0 |
| 25 | 96.2 | 3.8 | 25.1 | 1.7 | 99.9 | 96.0 | 3.8 | 25.5 | 1.0 |
| 26 | 96.2 | 3.8 | 25.2 | -0.3 | 100.6 | 96.8 | 3.8 | 25.3 | 1.0 |

TABLE 27. Filter Element of Example 22

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.8 | 3.2 | 30.3 | 2.9 | 96.5 | 93.4 | 3.1 | 30.1 | 0.99 |
| 2 | 96.7 | 3.3 | 29.3 | 6.1 | 96.0 | 92.8 | 3.1 | 29.9 | 1.02 |
| 3 | 96.7 | 3.3 | 29.3 | 11.1 | 96.2 | 93.0 | 2.9 | 32.1 | 1.09 |
| 4 | 96.6 | 3.4 | 28.4 | 6.6 | 96.7 | 93.4 | 3.2 | 29.2 | 1.03 |
| 6 | 96.6 | 3.4 | 28.4 | 73.3 | 73.3 | 70.8 | 0.9 | 78.7 | 2.77 |
| 7 | 96.7 | 3.3 | 29.3 | 82.4 | 76.8 | 74.3 | 0.6 | 123.8 | 4.23 |
| 8 | 96.6 | 3.4 | 28.4 | 33.3 | 97.9 | 94.6 | 2.3 | 41.1 | 1.45 |
| 9 | 96.5 | 3.5 | 27.6 | 8.7 | 99.6 | 96.1 | 3.2 | 30.0 | 1.09 |
| 18 | 96.3 | 3.7 | 26.0 | 79.6 | 80.3 | 77.3 | 0.8 | 96.6 | 3.71 |
| 19 | 96.3 | 3.7 | 26.0 | 5.1 | 99.8 | 96.1 | 3.5 | 27.4 | 1.05 |
| 20 | 96.3 | 3.7 | 26.0 | 0.4 | 100.4 | 96.7 | 3.7 | 26.2 | 1.01 |
| 21 | 96.3 | 3.7 | 26.0 | 0.8 | 100.3 | 96.6 | 3.7 | 26.1 | 1.00 |
| 23 | 96.2 | 3.8 | 25.3 | 51.4 | 86.6 | 83.3 | 1.8 | 46.3 | 1.83 |
| 24 | 96.1 | 3.9 | 24.6 | 1.0 | 100.2 | 96.3 | 3.9 | 24.7 | 1.00 |
| 25 | 96.2 | 3.8 | 25.3 | 1.7 | 99.6 | 95.8 | 3.7 | 25.9 | 1.02 |
| 26 | 96.2 | 3.8 | 25.3 | 0.2 | 100.5 | 96.7 | 3.8 | 25.4 | 1.00 |

TABLE 28. Filter Element of Example 23

| IgG Solution Number | Initial Monomeric IgG (%) | Initial Aggregated IgG (%) | Initial Composition Ratio | 386 g/L Challenge Load | | | | | | Ratio of Final Composition Ratio to Initial Composition Ratio |
|---|---|---|---|---|---|---|---|---|---|---|
| | | | | Filtration Performance | | Final Monomeric IgG Recovered (%) | Final Aggregated IgG Recovered (%) | Final Composition Ratio | | |
| | | | | Aggregated IgG Removed (%) | Monomeric IgG Yield (%) | | | | | |
| 1 | 97.5 | 2.5 | 39.0 | 10.8 | 91.9 | 89.6 | 2.2 | 40.7 | | 1.04 |
| 2 | 97.6 | 2.4 | 40.7 | 5.9 | 93.3 | 91.1 | 2.3 | 39.6 | | 0.97 |
| 3 | 97.4 | 2.6 | 37.5 | 21.1 | 94.2 | 91.8 | 2.1 | 43.7 | | 1.17 |
| 4 | 97.3 | 2.7 | 36.0 | 33.3 | 95.1 | 92.5 | 1.8 | 51.4 | | 1.43 |
| 5 | 97.4 | 2.6 | 37.5 | 29.9 | 97.2 | 94.7 | 1.8 | 52.6 | | 1.40 |
| 6 | 97.2 | 2.8 | 34.7 | 17.2 | 92.2 | 89.6 | 2.3 | 38.9 | | 1.12 |
| 7 | 97.0 | 3.0 | 32.3 | 38.3 | 91.0 | 88.3 | 1.9 | 46.5 | | 1.44 |
| 8 | 97.0 | 3.0 | 32.3 | 46.8 | 93.9 | 91.1 | 1.6 | 56.9 | | 1.76 |
| 9 | 97.1 | 2.9 | 33.5 | 43.0 | 96.4 | 93.6 | 1.7 | 55.0 | | 1.64 |
| 10 | 97.4 | 2.6 | 37.5 | 21.2 | 98.4 | 95.8 | 2.0 | 47.9 | | 1.28 |
| 18 | 96.8 | 3.2 | 30.3 | 57.4 | 87.2 | 84.4 | 1.4 | 60.3 | | 1.99 |
| 19 | 96.6 | 3.4 | 28.4 | 69.0 | 93.3 | 90.1 | 1.1 | 81.9 | | 2.88 |
| 20 | 96.8 | 3.2 | 30.3 | 41.5 | 97.9 | 94.8 | 1.9 | 49.9 | | 1.65 |
| 21 | 96.9 | 3.1 | 31.3 | 21.9 | 98.5 | 95.4 | 2.4 | 39.8 | | 1.27 |
| 22 | 97.3 | 2.7 | 36.0 | 10.3 | 98.9 | 96.2 | 2.4 | 40.1 | | 1.11 |
| 23 | 96.7 | 3.3 | 29.3 | 86.4 | 81.4 | 78.7 | 0.4 | 196.8 | | 6.72 |
| 24 | 96.7 | 3.3 | 29.3 | 43.3 | 97.4 | 94.2 | 1.9 | 49.6 | | 1.69 |
| 25 | 96.7 | 3.3 | 29.3 | 21.0 | 98.8 | 95.5 | 2.6 | 36.7 | | 1.25 |
| 26 | 96.7 | 3.3 | 29.3 | 10.0 | 99.2 | 95.9 | 3.0 | 32.0 | | 1.10 |
| 27 | 97.2 | 2.8 | 34.7 | 5.5 | 98.9 | 96.1 | 2.6 | 37.0 | | 1.07 |

TABLE 29. Filter Element of Example 24

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.4 | 3.6 | 26.8 | 10.7 | 92.7 | 89.4 | 3.2 | 27.9 | 1.04 |
| 2 | 97.1 | 2.9 | 33.5 | 8.0 | 92.5 | 89.8 | 2.7 | 33.2 | 0.99 |
| 3 | 96.7 | 3.3 | 29.3 | 14.5 | 93.8 | 90.7 | 2.8 | 32.4 | 1.10 |
| 4 | 96.9 | 3.1 | 31.3 | 19.2 | 93.3 | 90.4 | 2.5 | 36.1 | 1.15 |
| 6 | 96.8 | 3.2 | 30.3 | 22.1 | 93.1 | 90.1 | 2.5 | 36.0 | 1.19 |
| 7 | 96.5 | 3.5 | 27.6 | 48.5 | 83.9 | 81.0 | 1.8 | 45.0 | 1.63 |
| 8 | 95.3 | 4.7 | 20.3 | 66.7 | 76.6 | 73.0 | 1.6 | 45.6 | 2.25 |
| 9 | 97.3 | 2.7 | 36.0 | 71.4 | 80.1 | 77.9 | 0.8 | 97.4 | 2.71 |
| 18 | 95.6 | 4.4 | 21.7 | 71.9 | 63.0 | 60.2 | 1.2 | 50.2 | 2.31 |
| 19 | 94.9 | 5.1 | 18.6 | 75.0 | 86.3 | 81.9 | 1.3 | 63.0 | 3.39 |
| 20 | 97.1 | 2.9 | 33.5 | 36.3 | 95.4 | 92.6 | 1.8 | 51.4 | 1.53 |
| 21 | 96.5 | 3.5 | 27.6 | 17.7 | 98.7 | 95.2 | 2.9 | 32.8 | 1.19 |
| 23 | 94.7 | 5.3 | 17.9 | 81.8 | 68.5 | 64.9 | 1.0 | 64.9 | 3.63 |
| 24 | 94.6 | 5.4 | 17.5 | 22.6 | 97.1 | 91.9 | 4.2 | 21.9 | 1.25 |
| 25 | 94.7 | 5.3 | 17.9 | 7.6 | 99.5 | 94.2 | 4.9 | 19.2 | 1.08 |
| 26 | 96.1 | 3.9 | 24.6 | 6.5 | 99.9 | 96.0 | 3.6 | 26.7 | 1.08 |

TABLE 30. Filter Element of Example 25

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.1 | 2.9 | 33.5 | 94.8 | 86.5 | 84.0 | 0.2 | 420.0 | 12.54 |
| 2 | 97.1 | 2.9 | 33.5 | 83.0 | 91.3 | 88.7 | 0.5 | 177.4 | 5.30 |
| 3 | 96.6 | 3.4 | 28.4 | 26.1 | 97.4 | 94.1 | 2.5 | 37.6 | 1.32 |
| 6 | 96.8 | 3.2 | 30.3 | 93.1 | 79.1 | 76.6 | 0.2 | 383.0 | 12.64 |
| 7 | 96.5 | 3.5 | 27.6 | 32.6 | 97.1 | 93.7 | 2.4 | 39.0 | 1.41 |
| 8 | 98.4 | 1.6 | 61.5 | 6.4 | 98.1 | 96.5 | 1.5 | 64.3 | 1.05 |
| 18 | 96.0 | 4.0 | 24.0 | 79.8 | 87.4 | 83.9 | 0.8 | 104.9 | 4.37 |
| 19 | 96.2 | 3.8 | 25.3 | 8.9 | 98.5 | 94.8 | 3.5 | 27.1 | 1.07 |
| 20 | 96.2 | 3.8 | 25.3 | 5.2 | 99.6 | 95.8 | 3.6 | 26.6 | 1.05 |
| 23 | 96.2 | 3.8 | 25.3 | 50.8 | 88.6 | 85.2 | 1.9 | 44.8 | 1.77 |
| 24 | 96.0 | 4.0 | 24.0 | 6.3 | 98.8 | 94.8 | 3.7 | 25.6 | 1.07 |
| 25 | 96.0 | 4.0 | 24.0 | 6.1 | 98.7 | 94.8 | 3.8 | 24.9 | 1.04 |

TABLE 31. Filter Element of Example 26

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.1 | 2.9 | 33.5 | 90.8 | 89.6 | 87.0 | 0.3 | 290.0 | 8.66 |
| 2 | 97.1 | 2.9 | 33.5 | 43.1 | 96.1 | 93.3 | 1.7 | 54.9 | 1.64 |
| 3 | 96.6 | 3.4 | 28.4 | 15.9 | 98.1 | 94.8 | 2.9 | 32.7 | 1.15 |
| 6 | 96.8 | 3.2 | 30.3 | 95.1 | 81.8 | 79.2 | 0.2 | 396.0 | 13.07 |
| 7 | 96.5 | 3.5 | 27.6 | 21.3 | 98.0 | 94.6 | 2.8 | 33.8 | 1.22 |
| 8 | 98.4 | 1.6 | 61.5 | 2.6 | 98.4 | 96.8 | 1.6 | 60.5 | 0.98 |
| 18 | 96.0 | 4.0 | 24.0 | 59.1 | 91.8 | 88.1 | 1.6 | 55.1 | 2.30 |
| 19 | 96.2 | 3.8 | 25.3 | 8.3 | 99.1 | 95.3 | 3.5 | 27.2 | 1.08 |
| 20 | 96.2 | 3.8 | 25.3 | 4.4 | 99.3 | 95.5 | 3.6 | 26.5 | 1.05 |
| 23 | 96.2 | 3.8 | 25.3 | 34.7 | 92.5 | 89.0 | 2.5 | 35.6 | 1.41 |
| 24 | 96.0 | 4.0 | 24.0 | 4.7 | 98.8 | 94.8 | 3.8 | 24.9 | 1.04 |
| 25 | 96.0 | 4.0 | 24.0 | 2.4 | 99.3 | 95.3 | 3.9 | 24.4 | 1.02 |

TABLE 32. Filter Element of Example 27

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.1 | 2.9 | 33.5 | 51.6 | 96.0 | 93.2 | 1.4 | 66.6 | 1.98 |
| 2 | 97.1 | 2.9 | 33.5 | 14.4 | 97.9 | 95.1 | 2.5 | 38.0 | 1.14 |
| 3 | 96.6 | 3.4 | 28.4 | 11.9 | 98.2 | 94.9 | 3.0 | 31.6 | 1.11 |
| 6 | 96.8 | 3.2 | 30.3 | 71.0 | 93.4 | 90.4 | 0.9 | 100.4 | 3.31 |
| 7 | 96.5 | 3.5 | 27.6 | 18.9 | 98.2 | 94.8 | 2.8 | 33.8 | 1.21 |
| 8 | 98.4 | 1.6 | 61.5 | 4.7 | 98.8 | 97.2 | 1.5 | 64.8 | 1.05 |
| 18 | 96.0 | 4.0 | 24.0 | 30.0 | 96.7 | 92.8 | 2.8 | 33.1 | 1.38 |
| 19 | 96.2 | 3.8 | 25.3 | 6.4 | 98.5 | 94.8 | 3.6 | 26.3 | 1.04 |
| 20 | 96.2 | 3.8 | 25.3 | 5.7 | 98.8 | 95.0 | 3.6 | 26.4 | 1.04 |
| 23 | 96.2 | 3.8 | 25.3 | 19.6 | 96.1 | 92.4 | 3.1 | 29.8 | 1.18 |
| 24 | 96.0 | 4.0 | 24.0 | 5.2 | 98.6 | 94.7 | 3.8 | 24.9 | 1.04 |
| 25 | 96.0 | 4.0 | 24.0 | 4.5 | 99.0 | 95.0 | 3.8 | 25.0 | 1.04 |

TABLE 33. Filter Element of Example 28

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 97.1 | 2.9 | 33.5 | 73.7 | 92.1 | 89.4 | 0.8 | 111.7 | 3.33 |
| 2 | 97.1 | 2.9 | 33.5 | 16.0 | 98.1 | 95.3 | 2.4 | 39.7 | 1.19 |
| 3 | 96.6 | 3.4 | 28.4 | 10.0 | 98.5 | 95.2 | 3.1 | 30.7 | 1.08 |
| 6 | 96.8 | 3.2 | 30.3 | 83.0 | 84.7 | 82.0 | 0.5 | 164.0 | 5.41 |
| 7 | 96.5 | 3.5 | 27.6 | 16.1 | 98.7 | 95.2 | 2.9 | 32.8 | 1.19 |
| 8 | 98.4 | 1.6 | 61.5 | 1.1 | 99.2 | 97.6 | 1.6 | 61.0 | 0.99 |
| 18 | 96.0 | 4.0 | 24.0 | 35.4 | 94.0 | 90.2 | 2.6 | 34.7 | 1.45 |
| 19 | 96.2 | 3.8 | 25.3 | 3.3 | 99.3 | 95.5 | 3.7 | 25.8 | 1.02 |
| 20 | 96.2 | 3.8 | 25.3 | 2.2 | 99.9 | 96.1 | 3.7 | 26.0 | 1.03 |
| 23 | 96.2 | 3.8 | 25.3 | 27.5 | 92.8 | 89.3 | 2.8 | 31.9 | 1.26 |
| 24 | 96.0 | 4.0 | 24.0 | 1.6 | 99.7 | 95.7 | 3.9 | 24.5 | 1.02 |
| 25 | 96.0 | 4.0 | 24.0 | 1.7 | 100.0 | 96.0 | 3.9 | 24.6 | 1.02 |

TABLE 34. Filter Element of Example 29

| IgG Solution Number | Initial Monomeric IgG (%) | Initial Aggregated IgG (%) | Initial Composition Ratio | 387 g/L Challenge Load | | | | | Ratio of Final Composition Ratio to Initial Composition Ratio |
|---|---|---|---|---|---|---|---|---|---|
| | | | | Filtration Performance | | | | | |
| | | | | Aggregated IgG Removed (%) | Monomeric IgG Yield (%) | Final Monomeric IgG Recovered (%) | Final Aggregated IgG Recovered (%) | Final Composition Ratio | |
| 1 | 97.6 | 2.4 | 40.7 | 80.4 | 69.9 | 68.2 | 0.5 | 136.4 | 3.35 |
| 3 | 97.6 | 2.4 | 40.7 | 93.3 | 84.2 | 82.2 | 0.2 | 411.0 | 10.10 |
| 4 | 97.6 | 2.4 | 40.7 | 47.8 | 101.1 | 98.7 | 1.3 | 75.9 | 1.86 |
| 6 | 97.2 | 2.8 | 34.7 | 73.8 | 59.9 | 58.2 | 0.7 | 83.1 | 2.39 |
| 7 | 97.1 | 2.9 | 33.5 | 93.4 | 80.8 | 78.5 | 0.2 | 329.5 | 9.84 |
| 8 | 97.0 | 3.0 | 32.3 | 49.2 | 99.6 | 96.6 | 1.5 | 64.4 | 1.99 |
| 9 | 96.9 | 3.1 | 31.3 | 24.4 | 100.1 | 97.0 | 2.3 | 42.2 | 1.34 |
| 18 | 95.8 | 4.2 | 22.8 | 91.4 | 74.4 | 71.3 | 0.4 | 178.2 | 7.82 |
| 19 | 96.6 | 3.4 | 28.4 | 34.8 | 100.0 | 96.6 | 2.2 | 43.9 | 1.54 |
| 21 | 96.6 | 3.4 | 28.4 | 11.7 | 100.1 | 96.7 | 3.0 | 32.2 | 1.13 |
| 23 | 96.7 | 3.3 | 29.3 | 76.2 | 85.7 | 82.9 | 0.8 | 103.6 | 3.53 |
| 24 | 96.8 | 3.2 | 30.3 | 11.6 | 100.7 | 97.5 | 2.8 | 34.8 | 1.15 |
| 25 | 96.8 | 3.2 | 30.3 | 6.2 | 100.6 | 97.4 | 3.0 | 32.5 | 1.07 |

TABLE 35. Filter Element of Example 30

| IgG Solution Number | Initial Monomeric IgG (%) | Initial Aggregated IgG (%) | Initial Composition Ratio | 386 g/L Challenge Load | | | | | Ratio of Final Composition Ratio to Initial Composition Ratio |
|---|---|---|---|---|---|---|---|---|---|
| | | | | Filtration Performance | | Final Monomeric IgG Recovered (%) | Final Aggregated IgG Recovered (%) | Final Composition Ratio | |
| | | | | Aggregated IgG Removed (%) | Monomeric IgG Yield (%) | | | | |
| 1 | 96.6 | 3.4 | 28.4 | 68.3 | 73.1 | 70.6 | 1.1 | 64.2 | 2.26 |
| 2 | 96.8 | 3.2 | 30.3 | 83.8 | 78.2 | 75.7 | 0.5 | 151.4 | 5.00 |
| 3 | 96.7 | 3.3 | 29.3 | 80.7 | 90.0 | 87.0 | 0.6 | 145.0 | 4.95 |
| 4 | 96.8 | 3.2 | 30.3 | 11.9 | 98.3 | 95.2 | 2.8 | 34.0 | 1.12 |
| 6 | 96.8 | 3.2 | 30.3 | 73.3 | 70.9 | 68.6 | 0.9 | 76.2 | 2.51 |
| 7 | 96.4 | 3.6 | 26.8 | 76.2 | 91.5 | 88.2 | 0.9 | 98.0 | 3.65 |
| 8 | 95.1 | 4.9 | 19.4 | 6.3 | 97.2 | 92.4 | 4.6 | 20.1 | 1.04 |
| 9 | 97.1 | 2.9 | 33.5 | 3.2 | 99.1 | 96.2 | 2.8 | 34.3 | 1.02 |
| 11 | 95.8 | 4.2 | 22.8 | 92.5 | 80.6 | 77.2 | 0.3 | 257.3 | 11.29 |
| 12 | 95.1 | 4.9 | 19.4 | 5.7 | 97.4 | 92.6 | 4.6 | 20.1 | 1.04 |
| 13 | 96.4 | 3.6 | 26.8 | 2.2 | 99.2 | 95.6 | 3.5 | 27.3 | 1.02 |
| 14 | 96.2 | 3.8 | 25.3 | 0.9 | 99.4 | 95.6 | 3.8 | 25.1 | 0.99 |
| 16 | 95.2 | 4.8 | 19.8 | 74.8 | 87.8 | 83.6 | 1.2 | 69.7 | 3.52 |
| 17 | 95.1 | 4.9 | 19.4 | 1.9 | 99.4 | 94.5 | 4.8 | 19.7 | 1.01 |
| 18 | 95.1 | 4.9 | 19.4 | 1.3 | 99.3 | 94.4 | 4.8 | 19.7 | 1.01 |
| 19 | 96.0 | 4.0 | 24.0 | 1.8 | 99.7 | 95.7 | 3.9 | 24.5 | 1.02 |

## Claims

1. A process for separating aggregated proteins from monomeric proteins in a biological solution, the process comprising:

    providing at least one filter element having a contacting surface, wherein the filter element comprises filter media comprising:

        a porous substrate; and
        disposed on the porous substrate, a polymer comprising a hydrocarbon backbone and a plurality of pendant groups attached to the hydrocarbon backbone, wherein each of a first plurality of pendant groups comprises:

            (1) at least one acidic group or salt thereof; and
            (2) a spacer group that directly links the at least one acidic group or salt thereof to the hydrocarbon backbone by a chain of at least 6 catenated atoms; and

    allowing an initial biological solution to contact the contacting surface of the filter element under conditions effective to separate the aggregated proteins from the monomeric proteins such that a final biological solution includes purified monomeric proteins.

2. The process of claim 1 wherein the polymer comprises interpolymerized units of at least one monomer comprising:

    (1) at least one ethylenically unsaturated group;
    (2) at least one acidic group or salt thereof; and
    (3) a spacer group that directly links the at least one ethylenically unsaturated group and the at least one acidic group or salt thereof by a chain of at least 6 catenated atoms.

3. The process of claim 1 or 2 further comprising recovering the monomeric proteins without eluting.

4. The process of any one of claims 1 through 3 wherein the conditions are effective to recover, in the final solution, at least 70% of the monomeric proteins present in the initial biological solution.

5. The process of any one of claims 1 through 4 wherein the conditions are effective to remove at least 10% of the aggregated proteins from the initial biological solution.

6. The process of any one of claims 1 through 5 wherein the proteins comprise antibodies, enzymes, or hormones.

7. The process of claim 6 wherein the proteins comprise antibodies.

8. The process of any one of claims 1 through 7 wherein the conditions comprise a pH of the biological solution of below 9.

9. The process of any one of claims 1 through 8 wherein the conditions comprise a conductivity of the biological solution of at least 1 mS/cm.

10. The process of any one of claims 1 through 9 wherein the at least one acidic group or salt thereof of the polymer disposed on the porous substrate is present at a density of at least 0.02 mmole/gram of filter media.

11. The process of any one of claims 2 through 10, as dependent on claim 2, wherein the at least one ethylenically unsaturated group is selected from an ethenyl group, a 1-alkylethenyl group, and a combination thereof.

12. The process of any one of claims 1 through 11 wherein the chain of the spacer group has at least 8 catenated atoms.

13. The process of any one of claims 1 through 12 wherein the at least one acidic group or salt thereof is selected from a carboxy group, a phosphono group, a phosphate group, a sulfono group, a sulfato group, a boronato group, and a combination thereof.

14. The process of any one of claims 1 through 13 wherein the spacer group is a catenated heteroatom-containing hydrocarbon group.

**15.** The process of any one of claims 1 through 14 wherein the spacer group comprises at least one hydrogen bonding moiety.

## Patentansprüche

**1.** Ein Verfahren zum Trennen aggregierter Proteine von monomeren Proteinen in einer biologischen Lösung, das Verfahren umfassend:
Bereitstellen mindestens eines Filterelements, das eine Kontaktoberfläche aufweist, wobei das Filterelement Filtermedien umfasst, umfassend:

ein poröses Substrat; und
angeordnet auf dem porösen Substrat, ein Polymer, umfassend ein Kohlenwasserstoffrückgrat und eine Mehrzahl von Seitengruppen, die an das Kohlenwasserstoffrückgrat gebunden sind, wobei jede einer ersten Mehrzahl von Seitengruppen umfasst:

(1) mindestens eine saure Gruppe oder ein Salz davon; und
(2) eine Abstandsgruppe, die mit der mindestens einen sauren Gruppe oder dem Salz davon mit dem Kohlenwasserstoffrückgrat durch eine Kette von mindestens 6 Kettenatomen direkt verbunden ist; und

Ermöglichen, dass eine biologische Ausgangslösung die Kontaktoberfläche des Filterelements unter Bedingungen kontaktiert, die wirksam sind, um die aggregierten Proteine von den monomeren Proteinen derart zu trennen, dass eine biologische Endlösung gereinigte monomere Proteine einschließt.

**2.** Das Verfahren nach Anspruch 1, wobei das Polymer interpolymerisierte Einheiten mit mindestens einem Monomer umfasst, umfassend:

(1) mindestens eine ethylenisch ungesättigte Gruppe;
(2) mindestens eine saure Gruppe oder ein Salz davon; und
(3) eine Abstandsgruppe, die mit der mindestens einen ethylenisch ungesättigte Gruppe und der mindestens einen sauren Gruppe oder dem Salz davon durch eine Kette von mindestens 6 Kettenatomen direkt verbunden ist.

**3.** Das Verfahren nach Anspruch 1 oder 2, ferner umfassend ein Gewinnen der monomeren Proteine ohne Eluieren.

**4.** Das Verfahren nach einem der Ansprüche 1 bis 3, wobei die Bedingungen wirksam sind, um in der Endlösung mindestens 70% der monomeren Proteine, die in der biologischen Ausgangslösung vorhanden sind, zu gewinnen.

**5.** Das Verfahren nach einem der Ansprüche 1 bis 4, wobei die Bedingungen wirksam sind, um mindestens 10% der aggregierten Proteine aus der biologischen Ausgangslösung zu entfernen.

**6.** Das Verfahren nach einem der Ansprüche 1 bis 5, wobei die Proteine Antikörper, Enzyme oder Hormone umfassen.

**7.** Das Verfahren nach Anspruch 6, wobei die Proteine Antikörper umfassen:

**8.** Das Verfahren nach einem der Ansprüche 1 bis 7, wobei die Bedingungen einen pH-Wert der biologischen Lösung von unter 9 umfassen.

**9.** Das Verfahren nach einem der Ansprüche 1 bis 8, wobei die Bedingungen eine Leitfähigkeit der biologischen Lösung von mindestens 1 mS/cm umfassen.

**10.** Das Verfahren nach einem der Ansprüche 1 bis 9, wobei die mindestens eine saure Gruppe oder das Salz davon des Polymers, das auf dem porösen Substrat angeordnet ist, in einer Dichte von mindestens 0.02 mmol/Gramm der Filtermedien vorliegt.

**11.** Das Verfahren nach einem der Ansprüche 2 bis 10, in Abhängigkeit von Anspruch 2, wobei die mindestens eine ethylenisch ungesättigte Gruppe ausgewählt ist aus einer Ethenylgruppe, einer 1-Alkylphenyl-Gruppe und einer Kombination davon.

**12.** Das Verfahren nach einem der Ansprüche 1 bis 11, wobei die Kette der Abstandsgruppe mindestens 8 Kettenatome aufweist.

**13.** Das Verfahren nach einem der Ansprüche 1 bis 12, wobei die mindestens eine saure Gruppe oder das Salz davon ausgewählt ist aus einer Carboxygruppe, einer Phosphonogruppe, einer Phosphatgruppe, einer Sulfonogruppe, einer Sulfatgruppe, einer Boronogruppe und einer Kombination davon.

**14.** Das Verfahren nach einem der Ansprüche 1 bis 13, wobei die Abstandsgruppe eine verkettete heteroatomhaltige Kohlenwasserstoffgruppe ist.

**15.** Das Verfahren nach einem der Ansprüche 1 bis 14, wobei die Abstandsgruppe mindestens eine Wasserstoffbindungseinheit umfasst.

## Revendications

**1.** Procédé de séparation de protéines agrégées par rapport à des protéines monomères dans une solution biologique, le procédé comprenant:
la fourniture d'au moins un élément filtrant ayant une surface de contact, dans lequel l'élément filtrant comprend un milieu filtrant comprenant:

> un substrat poreux; et
> disposé sur le substrat poreux, un polymère comprenant un squelette hydrocarboné et une pluralité de groupes greffés fixés au squelette hydrocarboné, dans lequel chacun parmi une première pluralité de groupes greffés comprend:
>
> > (1) au moins un groupe acide ou sel de celui-ci; et
> > (2) un groupe espaceur qui lie directement l'au moins un groupe acide ou sel de celui-ci au squelette hydrocarboné par une chaîne d'au moins 6 atomes caténaires; et
>
> le fait de permettre à une solution biologique initiale de venir en contact avec la surface de contact de l'élément filtrant dans des conditions efficaces pour séparer les protéines agrégées par rapport aux protéines monomères de telle sorte qu'une solution biologique finale inclut des protéines monomères purifiées.

**2.** Procédé selon la revendication 1 dans lequel le polymère comprend des motifs interpolymérisés d'au moins un monomère comprenant:

> (1) au moins un groupe à insaturation éthylénique;
> (2) au moins un groupe acide ou sel de celui-ci; et
> (3) un groupe espaceur qui lie directement l'au moins un groupe à insaturation éthylénique et l'au moins un groupe acide ou sel de celui-ci par une chaîne d'au moins 6 atomes caténaires.

**3.** Procédé selon la revendication 1 ou 2 comprenant en outre la récupération des protéines monomères sans élution.

**4.** Procédé selon l'une quelconque des revendications 1 à 3 dans lequel les conditions sont efficaces pour récupérer, dans la solution finale, au moins 70% des protéines monomères présentes dans la solution biologique initiale.

**5.** Procédé selon l'une quelconque des revendications 1 à 4 dans lequel les conditions sont efficaces pour éliminer au moins 10% des protéines agrégées par rapport à la solution biologique initiale.

**6.** Procédé selon l'une quelconque des revendications 1 à 5 dans lequel les protéines comprennent des anticorps, des enzymes, ou des hormones.

**7.** Procédé selon la revendication 6 dans lequel les protéines comprennent des anticorps.

**8.** Procédé selon l'une quelconque des revendications 1 à 7 dans lequel les conditions comprennent un pH de la solution biologique inférieur à 9.

**9.** Procédé selon l'une quelconque des revendications 1 à 8 dans lequel les conditions comprennent une conductivité de la solution biologique d'au moins 1 mS/cm.

**10.** Procédé selon l'une quelconque des revendications 1 à 9 dans lequel l'au moins un groupe acide ou sel de celui-ci du polymère disposé sur le substrat poreux est présent à une densité d'au moins 0.02 mmole/gramme de milieu filtrant.

**11.** Procédé selon l'une quelconque des revendications 2 à 10, prise en dépendance de la revendication 2, dans lequel l'au moins un groupe à insaturation éthylénique est choisi parmi un groupe éthényle, un groupe 1-alkyléthényle, et une combinaison de ceux-ci.

**12.** Procédé selon l'une quelconque des revendications 1 à 11 dans lequel la chaîne du groupe espaceur a au moins 8 atomes caténaires.

**13.** Procédé selon l'une quelconque des revendications 1 à 12 dans lequel l'au moins un groupe acide ou sel de celui-ci est choisi parmi un groupe carboxy, un groupe phosphono, un groupe phosphate, un groupe sulfono, un groupe sulfato, un groupe boronato, et une combinaison de ceux-ci.

**14.** Procédé selon l'une quelconque des revendications 1 à 13 dans lequel le groupe espaceur est un groupe hydrocarboné contenant un hétéroatome caténaire.

**15.** Procédé selon l'une quelconque des revendications 1 à 14 dans lequel le groupe espaceur comprend au moins un fragment à liaison hydrogène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62385385
- WO 2015088677 A1
- WO 2010098867 A1
- WO 2015050767 A1
- US 5506279 A, Babu
- US 7098253 B, Rasmussen
- US 7674835 B, Rasmussen
- US 7647836 B, Rasmussen
- US 7683100 B, Rasmussen
- US 7125603 B, David
- US 4539256 A, Shipman
- US 4726989 A, Mrozinski
- US 4867881 A, Kinzer
- US 5120594 A, Mrozinski
- US 5260360 A, Mrozinski
- US 5962544 A, Waller, Jr.
- US 7338692 B, Smith
- US 6056529 A, Meyering
- US 6267916 B, Meyering
- US 6413070 B, Meyering
- US 6776940 B, Meyering
- US 3876738 A, Marinaccio
- US 3928517 A, Knight
- US 4707265 A, Barnes, Jr.
- US 5458782 A, Hou
- US 20150136698 A, 3M Innovative Properties Co.
- WO 2014052215 A, Rasmussen
- US 20120252091 A1, Rasmussen
- US 20110100916 A1, Shannon
- JP 47040913 B, Teijin Ltd.


### Non-patent literature cited in the description


- **WENTE.** Superfine Thermoplastic Fibers. *Indus. Eng. Chem.,* 1956, vol. 48, 1342
- **WENTE et al.** Manufacture Of Superfine Organic Fibers. *Naval Research Laboratories Report,* 1954, (4364